# EUROPEAN PATENT APPLICATION

(11) **EP 4 450 073 A1**
(43) Date of publication of application: **23.10.2024**
(21) Application number: 22906989.3
(22) Date of filing: 12.10.2022
(51) Int. Cl.: A61K 31/716, A61P 1/00, A61P 35/00, A61P 43/00

(54) **INTESTINAL TUMOR SUPPRESSOR, PGE2 PRODUCTION INHIBITOR, AND IL-22BP PRODUCTION PROMOTER**

(30) Priority: 16.12.2021 JP 2021204572
(71) Applicant: Louis Pasteur Center for Medical Research, Kyoto 606-8225 (JP)
(72) Inventor: IWAKURA, Yoichiro, Kyoto-shi, Kyoto 606-8225 (JP); TANG, Ce, Kyoto-shi, Kyoto 606-8225 (JP)
(74) Representative: Cabinet Laurent & Charras
(86) International application number: PCT/JP2022/038130
(87) International publication number: WO 2023/112454

(57) **Abstract**

An intestinal tumor suppressor that contains a Dectin-1 inhibitor or a β-glucan having a Dectin-1 inhibitory activity and that suppresses an intestinal tumor through Dectin-1 inhibition; a PGE₂ production inhibitor that contains a β-glucan having a Dectin-1 inhibitory activity; and an IL-22BP production promoter containing a β-glucan having a Dectin-1 inhibitory activity, are provided.

## Description

### Technical Field

The present disclosure relates to an intestinal tumor suppressor, a PGE₂ production inhibitor, and an IL-22BP production promoter.

### Background Art

Colorectal cancer (CRC) is the third most common cancer in the world, with over 1 million new cases and 500,000 deaths globally every year (Bray, F., et al., CA Cancer J Clin 68, 394-424 (2018)). Many cases of the CRC occur in older people and those having lifestyles of developed countries (Bosman, F. & Yan, P., Pol J Pathol 65, 257-266 (2014)), and dietary habit is suggested to be a possible risk factor for CRC. However, underlying mechanisms that regulate intestinal tumor development have not been elucidated completely.

A C-type lectin receptor (CLR) is one of pattern recognition receptors, and mainly involved in host defense against pathogens by recognizing glycan structures specific to the pathogens. Dectin-1 (also called Dectin-1 or DECTIN-1, gene symbol: Clec7a or CLEC7A), which is a member of the bone marrow type II type C lectin family, is one of the CLRs preferentially expressed in myeloid-derived cells, and a receptor for β-1,3-linked glucans (β-glucan) or glucans with a β-1,6-linked branched chain on a β-1,3-linked main chain (β-glucan) (Non-Patent Literature 1: Taylor, P. R., et al., J Immunol 169, 3876-3882 (2002)). Since glucans are major cell wall components of most fungi, Dectin-1 stands on the first line of the host defense against fungal infection (Taylor, P. R., et al., Nat Immunol 8, 31-38 (2007), Saijo, S., et al., Nat Immunol 8, 39-46 (2007)). Recent studies, however, have revealed that this receptor is also involved in the development of allergic, autoimmune, and cancerous diseases (Tang, C., Makusheva, Y, Sun, H., Han, W. & Iwakura, Y, J Leukoc Biol 106, 903-917 (2019), Brown, G. D., Willment, J. A. & Whitehead, L., Nat Rev Immunol 18, 374-389 (2018)).

Administration of a Dectin-1 agonistic ligand Curdlan evokes arthritis, spondyloarthritis and ileitis in SKG mice, an autoimmune-prone mouse with a spontaneous mutation in ZAP-70 (Ruutu, M., et al., Arthritis Rheum 64, 2211-2222 (2012), Yoshitomi, H., et al., J Exp Med 201, 949-960 (2005)). Dectin-1-induced IL-22 aggravates airway hypersensitivity by inducing proallergic chemokines and mucus (Lilly, L. M., et al., J Immunol 189, 3653-3660 (2012)). It has recently been reported that Dectin-1-deficiency causes overexpansion of a commensal bacteria *Lactobacillus murinus,* which can induce regulatory T (Treg) cell differentiation, in the mouse colon, resulting in the suppression of dextran sodium sulfate (DSS)-induced colitis (Tang, C., et al., Cell Host Microbe 18, 183-197 (2015)). Dectin-1 signaling also induces IL-17F in the downstream and suppresses the growth of a group of commensal bacteria including *Clostridium* cluster XIVa, which promotes Treg differentiation, by inducing specific antimicrobial proteins (Kamiya, T., et al., Mucosal Immunol 11, 763-773 (2018), Tang, C., et al., Nat Immunol 19, 755-765 (2018)). Therefore, Dectin-1 basically activates the immune system to eradicate pathogens, while this activity, on the other hand, can also trigger or enhance inflammation-related diseases.

Contradictory results were reported about the roles of Dectin-1 in the development of tumors (hereinafter, also referred to as polyps) (Tang, C., Makusheva, Y, Sun, H., Han, W. & Iwakura, Y, J Leukoc Biol 106, 903-917 (2019)). Dectin-1 signaling enhances tumor-killing activity of NK cells through induction of INAM on DCs by binding N-glycans on B16 melanoma cells (Chiba, S., et al., Elife 3, e04177 (2014)). By activating Raf1 and NF-xB, Dectin-1 upregulates TNFSF15 and OX40L expression on DCs to promote anti-tumorigenic Th9 cell differentiation (Zhao, Y, et al., Nat Commun 7, 12368 (2016)). Dectin-1 is also reported to suppress chemical carcinogen-induced hepatocellular carcinogenesis by inducing M-CSF to suppress Toll-like receptor (TLR) 4 and CD14 expression (Seifert, L., et al., Cell Rep 13, 1909-1921 (2015)). In contrast, Dectin-1 recognizes non-canonical ligand galectin-9 expressed on pancreatic ductal adenocarcinoma to suppresses anti-tumorigenic M1 macrophage differentiation, indicating a promotive role of Dectin-1 signaling in pancreatic tumorigenesis (Non-Patent Literature 2: Daley, D., et al., Nat Med 23, 556-567 (2017)).

### Prior Art Literature

### Non-Patent Literature

Non-Patent Literature 1: Taylor, P.R., et al. The beta-glucan receptor, Dectin-1, is predominantly expressed on the surface of cells of the monocyte/macrophage and neutrophil lineages. J Immunol 169, 3876-3882 (2002)
Non-Patent Literature 2: Daley, D., et al. Dectin 1 activation on macrophages by galectin 9 promotes pancreatic carcinoma and peritumoral immune tolerance. Nat Med 23, 556-567 (2017)

### SUMMARY OF INVENTION

### Technical Problem

However, there have been limited findings on agents and the like for intestinal tumors in previous reports.

The present disclosure has been made in view of the foregoing, and an object of the present disclosure is to provide an intestinal tumor suppressor capable of suppressing intestinal tumors, a PGE₂ production inhibitor capable of suppressing PGE₂ production, and an IL-22BP production promoter capable of promoting IL-22BP production.

### Solution to Problem

Means to solve the foregoing problem include the following aspects.
(1) An intestinal tumor suppressor containing a β-glucan having a Dectin-1 inhibitory activity, wherein the intestinal tumor suppressor suppresses an intestinal tumor through Dectin-1 inhibition.
(2) The intestinal tumor suppressor according to (1), wherein the β-glucan is a β-glucan including β-1,3 linkages, or a β-glucan including a β-1,6-linked branched chain on a β-1,3-linked main chain.
(3) The intestinal tumor suppressor according to (1) or (2), wherein a molecular weight of the β-glucan is from 0.2 K to 100 K.
(4) The intestinal tumor suppressor according to any one of (1) to (3), wherein the β-glucan is laminarin.
(5) A PGE₂ production inhibitor containing a β-glucan having a Dectin-1 inhibitory activity.
(6) The PGE₂ production inhibitor according to (5), wherein the β-glucan is a β-glucan including β-1,3 linkages, or a β-glucan including a β-1,6-linked branched chain on a β-1,3-linked main chain.
(7) The PGE₂ production inhibitor according to (5) or (6), wherein a molecular weight of the β-glucan is from 0.2 K to 100 K.
(8) The PGE₂ production inhibitor according to any one of (5) to (7), wherein the β-glucan is laminarin.
(9) An IL-22BP production promoter containing a β-glucan having a Dectin-1 inhibitory activity.
(10) The IL-22BP production promoter according to (9), wherein the β-glucan is a β-glucan including β-1,3 linkages, or a β-glucan including a β-1,6-linked branched chain on a β-1,3-linked main chain.
(11) The IL-22BP production promoter according to (9) or (10), wherein a molecular weight of the β-glucan is from 0.2 K to 100 K.
(12) The IL-22BP production promoter according to any one of (9) to (11), wherein the β-glucan is laminarin.
(13) An intestinal tumor suppressor containing a Dectin-1 inhibitor, wherein the intestinal tumor suppressor suppresses an intestinal tumor through Dectin-1 inhibition.
(14) The intestinal tumor suppressor according to (13), wherein the Dectin-1 inhibitor is a Dectin-1 antagonist.
(15) The intestinal tumor suppressor according to (13), wherein the Dectin-1 inhibitor is an anti-Dectin-1 inhibitory antibody or a Dectin-1 inhibitory small molecule compound.
(16) The intestinal tumor suppressor according to any one of (1) to (4), (13), and (14), wherein the intestinal tumor is an intestinal tumor accompanied by an increase in an expression level of PGE₂ or an intestinal tumor accompanied by a decrease in an expression level of IL-22BP.
(17) A β-glucan having a Dectin-1 inhibitory activity for use in the prevention or treatment of an intestinal tumor.
(18) The β-glucan having a Dectin-1 inhibitory activity according to (17), wherein the intestinal tumor is an intestinal tumor accompanied by an increase in an expression level of PGE₂.
(19) The β-glucan having a Dectin-1 inhibitory activity according to (17) or (18), wherein the intestinal tumor is an intestinal tumor accompanied by a decrease in an expression level of IL-22BP.
(20) A use of a β-glucan having a Dectin-1 inhibitory activity in the manufacture of a medicament for use in the prevention or treatment of an intestinal tumor
(21) The use according to (20), wherein the intestinal tumor is an intestinal tumor accompanied by an increase in an expression level of PGE₂.
(22) The use according to (20) or (21), wherein the intestinal tumor is an intestinal tumor accompanied by a decrease in an expression level of IL-22BP.
(23) A method for predicting severity of an intestinal tumor, including measuring at least one of an expression level of a Dectin-1 protein or the expression level of a CLEC7A gene in a tumor tissue.

### Advantageous Effects of Invention

According to the present disclosure, an intestinal tumor suppressor capable of suppressing intestinal tumors, a PGE₂ production inhibitor capable of suppressing PGE₂ production, and an IL-22BP production promoter capable of promoting IL-22BP production are provided.

### BRIEF DESCRIPTION OF DRAWINGS

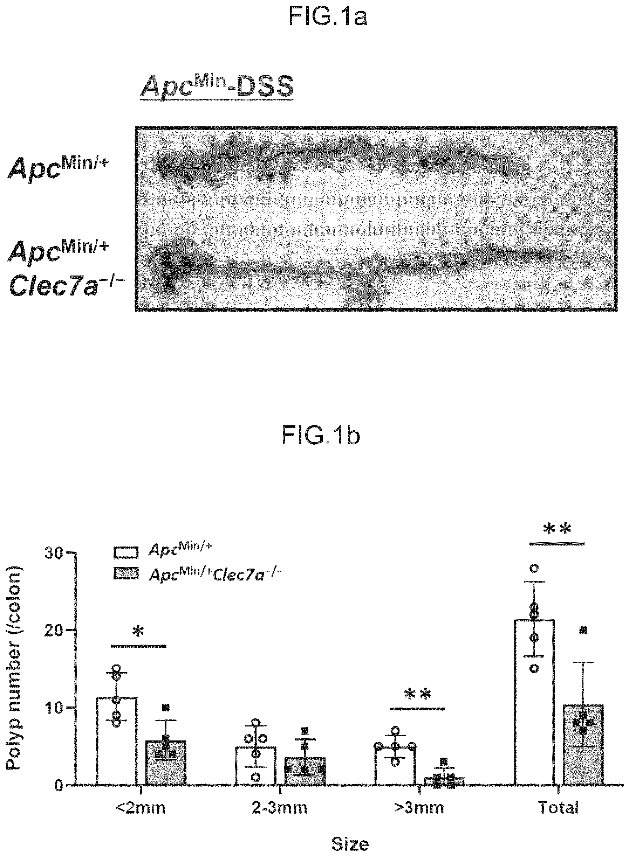

Fig. 1a: Dectin-1 deficiency suppresses colorectal tumors, and this suppression is independent of commensal microbiota. Apc^{Min/+} mice and Apc^{Min/+}Clec7a^{-/-} mice at 8 to 10 weeks old were administered with 1% by mass of DSS dissolved in drinking water for 7 days, and were sacrificed 4 weeks later. Gross observation of colorectal tumors was performed (n = 5/group).
Fig. 1b: Apc^{Min/+} mice and Apc^{Min/+}Clec7a^{-/-} mice at 8 to 10 weeks old were administered with 1% by mass of DSS dissolved in drinking water for 7 days, and were sacrificed 4 weeks later. The number of colorectal polyps with indicated sizes was counted (n = 5/group).

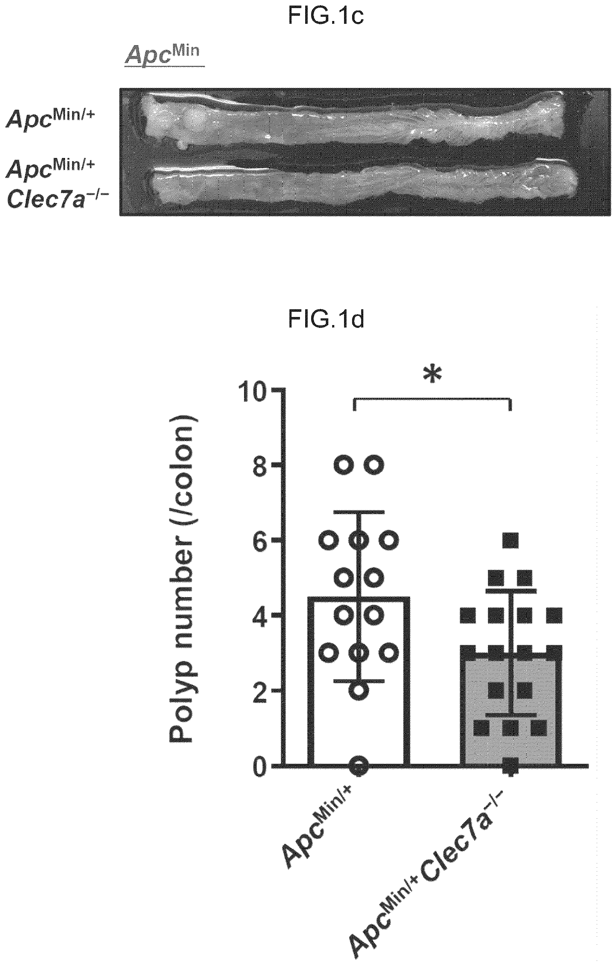

Fig. 1c: Apc^{Min/+} mice and Apc^{Min/+}Clec7a^{-/-} mice were raised under SPF conditions and were sacrificed at 20 to 23 weeks old, and gross observation of colorectal tumors was performed (n = 14/Apc^{Min/+} group, and n = 17/Apc^{Min/+}Clec7a^{-/-} group).
Fig. 1d: Apc^{Min/+} mice and the Apc^{min/+}Clec7a^{-/-} mice were raised under SPF conditions and were sacrificed at 20 to 23 weeks old. The number of colorectal polyps was counted (n = 14/Apc^{Min/+} group, and n = 17/Apc^{Min/+}Clec7a^{-/-} group).

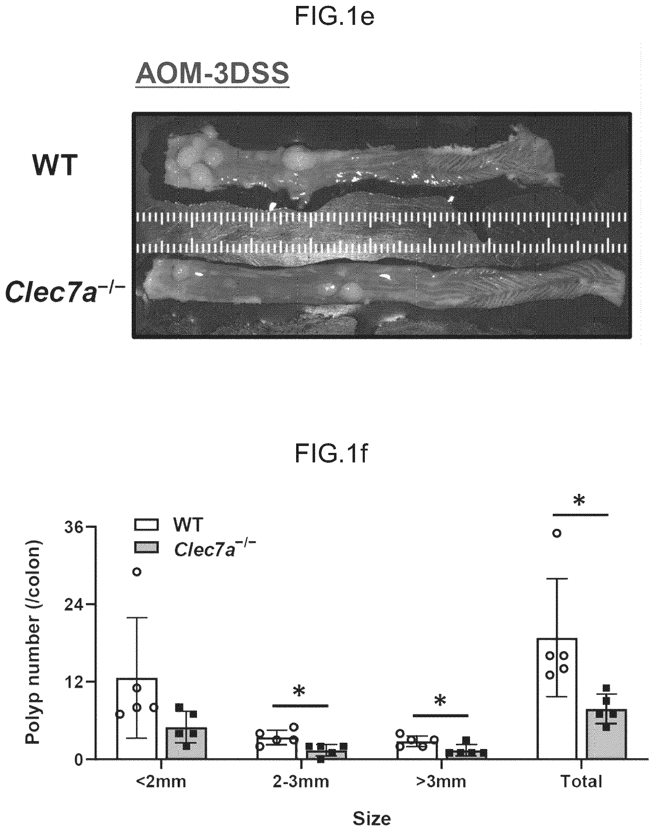

Fig. 1e: WT and Clec7a^{-/-} mice that had been separately raised were intraperitoneally administered with AOM, and were administered 7 days later with 1% by mass of DSS for three cycles as described in "Materials and Methods" to be described later. At Week 16 from the first AOM administration, the mice were sacrificed and gross observation of colorectal tumors was performed (n = 5/group).
Fig. 1f: WT and Clec7a^{-/-} mice that had been separately raised were intraperitoneally administered with AOM, and were administered 7 days later with 1% by mass of DSS for three cycles, as described in "Materials and Methods" to be described later. At Week 16 from the first AOM administration, the mice were sacrificed and the number of the colorectal polyps with indicated sizes was counted (n = 5/group).

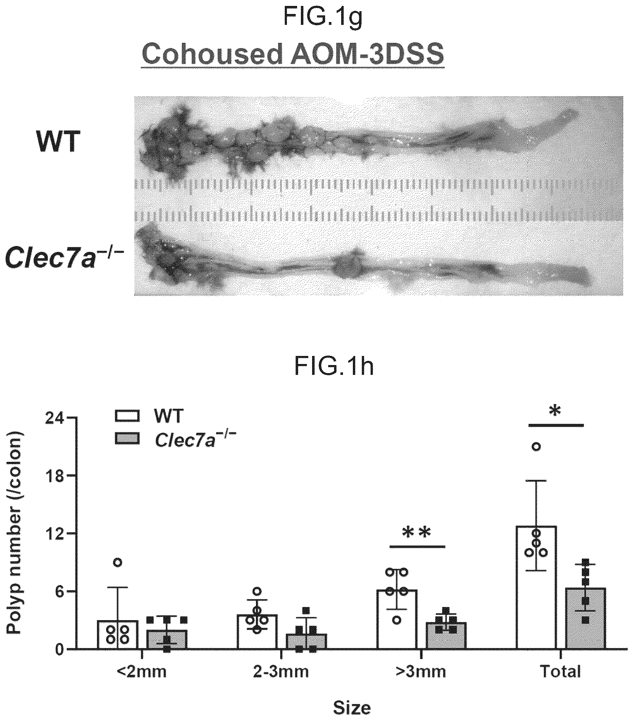

Fig. 1g: WT mice and Clec7a^{-/-} mice were co-housed after weaning at 4 weeks old, and were intraperitoneally administered 4 weeks later with AOM, followed by three cycles of 1% by mass of DSS (as described in "Materials and Methods"). At Week 16 from the first AOM administration, the mice were sacrificed and gross observation of colorectal tumors was performed (n = 5/group).
Fig. 1h: WT mice and Clec7a^{-/-} mice were co-housed after weaning at 4 weeks old, and were intraperitoneally administered 4 weeks later with AOM, followed by three cycles of 1% by mass of DSS (as described in "Materials and Methods"). At Week 16 from the first AOM administration, the mice were sacrificed and the number of colorectal polyps with indicated sizes was measured (n = 5/group).

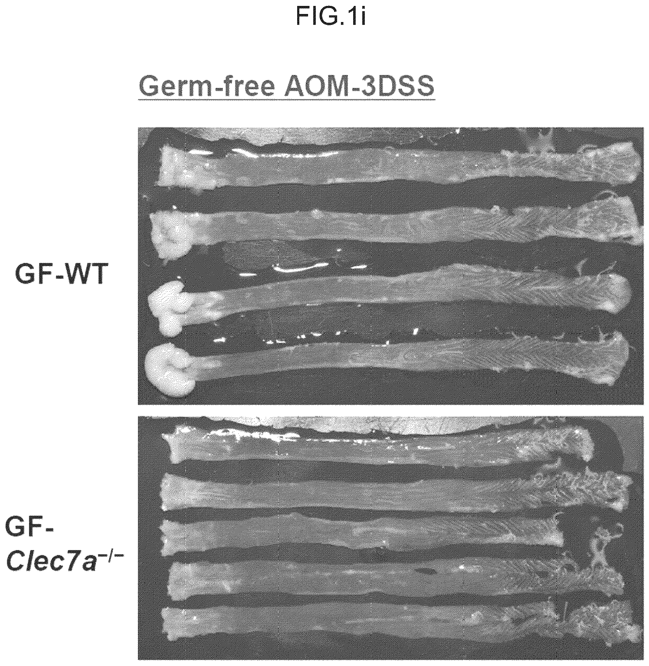

Fig. 1i: Sterile (GF) WT mice and GF Clec7a^{-/-} mice were administered with AOM, and then were administered with 1% by mass of DSS for three cycles. After 36 weeks from the AOM administration, the mice were sacrificed. The colorectal tumors were observed (n = 4/GF WT group, and n = 5/GF Clec7a^{-/-} group).

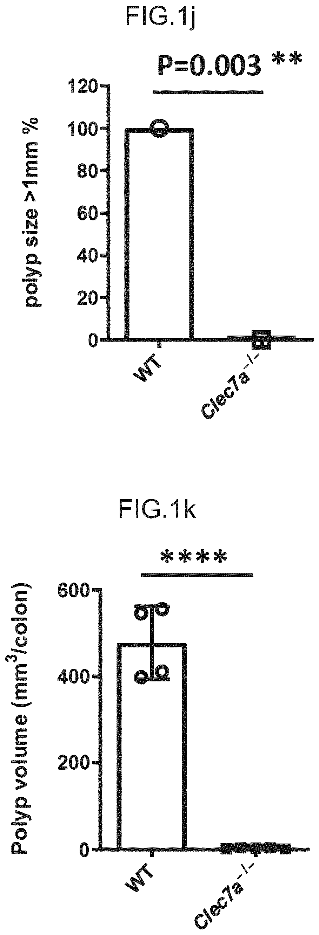

Fig. 1j: Sterile (GF) WT mice and the GF Clec7a^{-/-} mice were administered with AOM, and then were administered with 1% by mass of DSS for three cycles. After 36 weeks from the AOM administration, these mice were sacrificed. Frequency of individuals with a polyp diameter of 1 mm or larger was measured (n = 4/GF WT group, and n = 5/GF Clec7a^{-/-} group).
Fig. 1k: Sterile (GF) WT mice and GF Clec7a^{-/-} mice were administered with AOM, and then were administered with 1% by mass of DSS for three cycles. After 36 weeks from the AOM administration, the mice were sacrificed. The volumes of polyps in the colons were calculated as (diameters)³ (n = 4/GF WT group, and n = 5/GF Clec7a^{-/-} group). The Data in (Figs. 1a to 1j) are representative of at least three independent experiments. The data in (Figs. 1a, 1c, 1d, 1e, 1g, 1h, and 1j) are expressed as mean ± SD. *p < 0.05, and **p < 0.01.

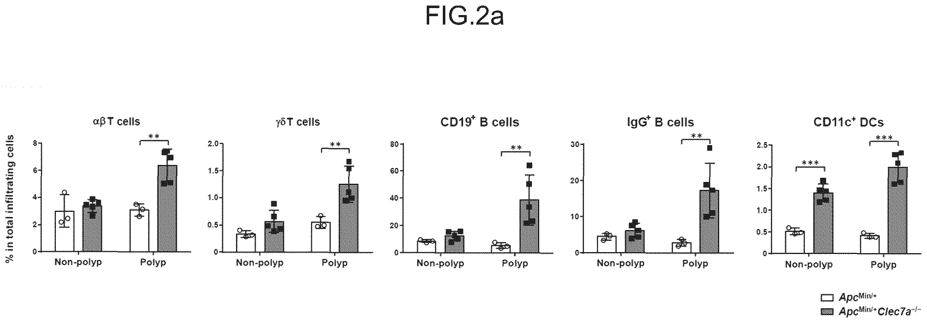

Fig. 2a: Differentiation of MDSCs is promoted by Dectin-1 signaling in colorectal tumors. Apc^{Min/+} mice and Apc^{Min/+}Clec7a^{-/-} mice were administered with 1% by mass of DSS for 7 days and were sacrificed 4 weeks later. The proportions of T cells, B cells and DCs in total tumor-infiltrating cells were examined by flow cytometry (n = 3/Apc^{Min/+} group, and n = 5/Apc^{Min/+}Clec7a^{-/-} group).

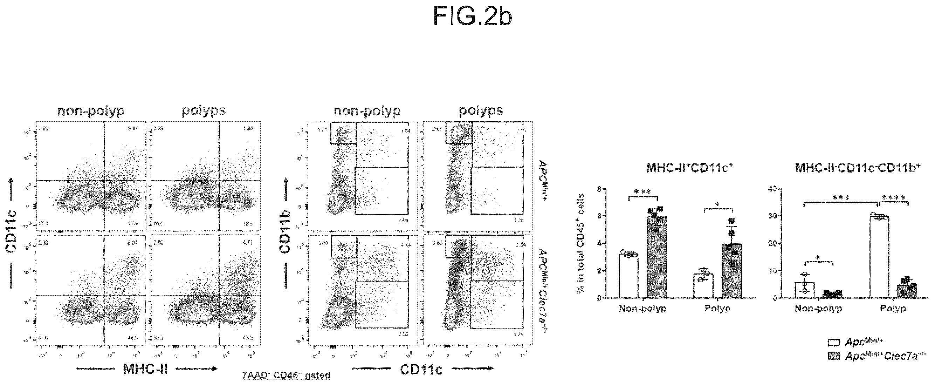

Fig. 2b: Apc^{Min/+} mice and Apc^{min/+}Clec7a^{-/-} mice were administered with 1% by mass of DSS for 7 days and were sacrificed 4 weeks later. Proportions of MHC-II⁺CD11c⁺ cells and MHC-II⁺CD11c⁻CD11b⁺ cells in CD45⁺ cells infiltrating non-polyp tissues or colon polyps were examined by flow cytometry (n = 3/Apc^{Min/+} group, and n = 5/Apc^{Min/+}Clec7a^{-/-} group).

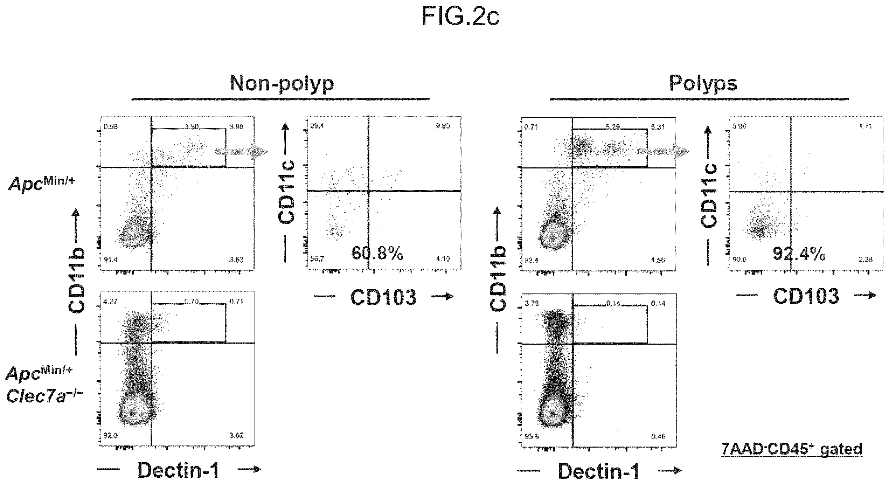

Fig. 2c: WT mice and Clec7a^{-/-} mice were administered with AOM, then were administered with 1% by mass of DSS for three cycles, and were sacrificed after 12 weeks from the AOM administration. Flow cytometry analysis was performed to identify Dectin-1-expressing cells in colorectal polyps and non-polyp tissues.

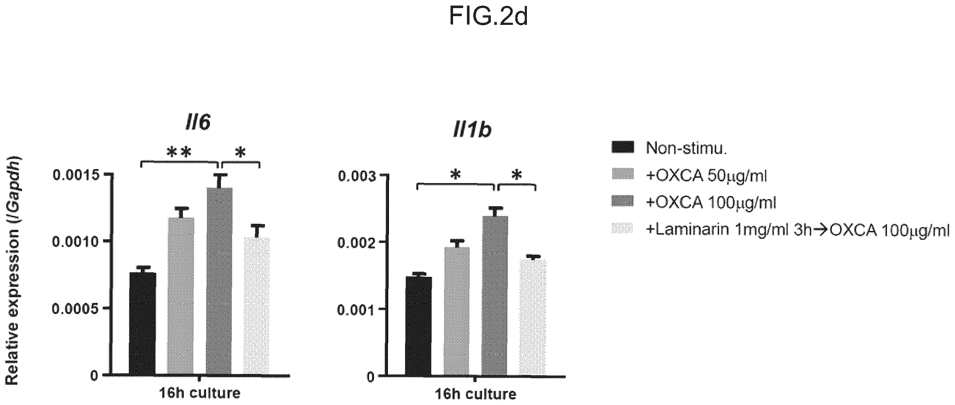

Fig 2d: WT SPF mice were administered with AOM, followed by three cycles of DSS. After 11 weeks, the mice were sacrificed, and CD11b⁺ cells and CD11c⁺ cells were purified from colorectal polyps with autoMACS. The purified cells were stimulated with an agonistic β-glucan OXCA, or were treated with an antagonistic β-glucan laminarin for 3 hours followed by the stimulation with OXCA for 16 hours, and the expression of Il6 and Il1b was measured by qPCR. The data in (Figs. 2a to 2d) are representative of two independent experiments, and the data in (Figs. 2a, 2b, and 2d) are expressed as mean ± SD. *p < 0.05, **p < 0.01, ***p < 0.001, and ****p < 0.0001.

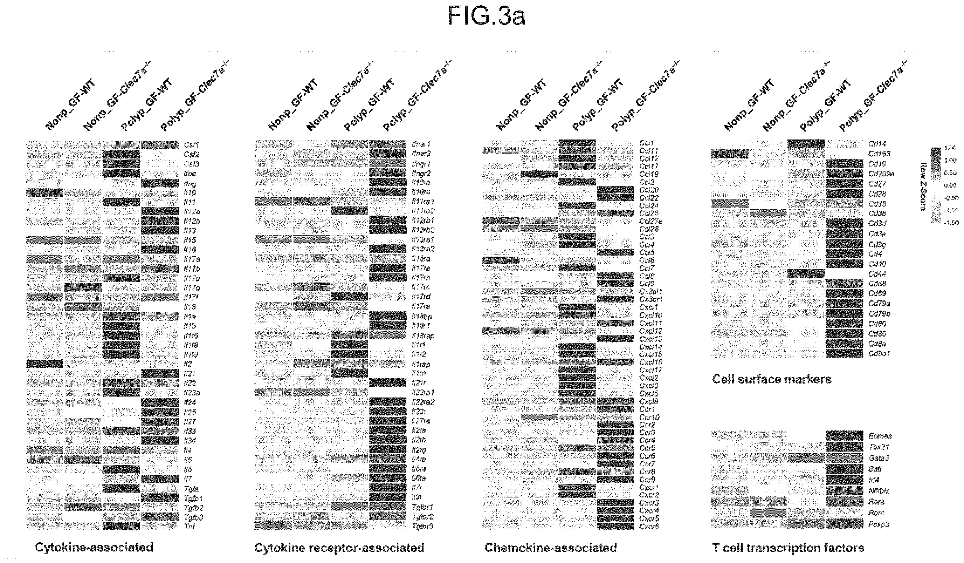

Fig. 3a: The expression of most of the genes that represent the tumor-associated immune system is dependent on the Dectin-1 signaling, and is not affected by commensal microbiota. GF WT mice and GF Clec7a^{-/-} mice were administered with AOM, followed by administration of 1% by mass of DSS for three cycles. The mice were sacrificed at 36 weeks after the AOM administration (similarly to the experiments illustrated in Figs. 1i to 1k), and RNAs were purified from polyps and non-polyp tissues in the vicinity and were analyzed with RNA-seq. A comparison between the WT mice and the Clec7a^{-/-} mice for cytokines, cytokine receptors, chemokines, cell surface markers, T cell transcription factor-related genes in the polyps and the non-polyp tissues are shown as a heatmap.

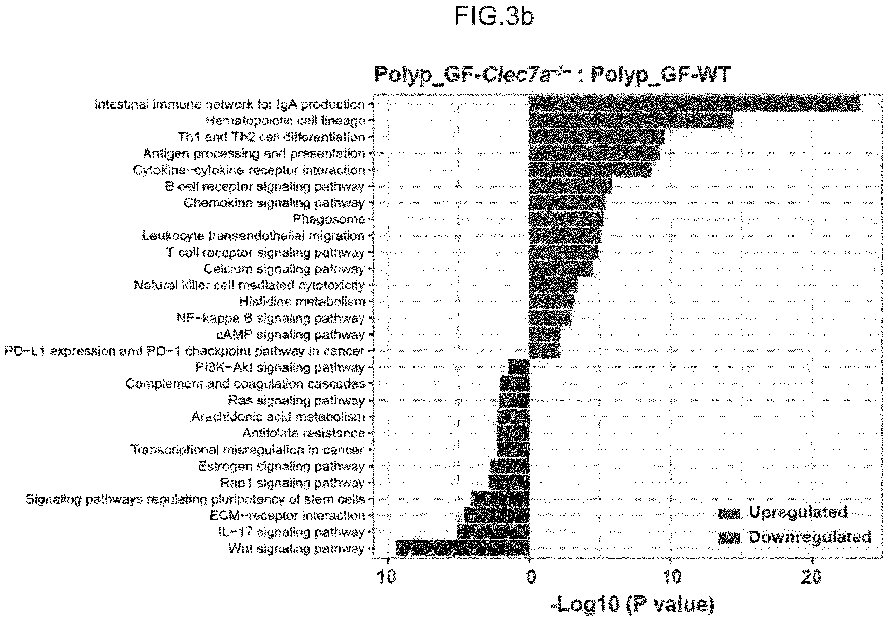

Fig. 3b: Using a KEGG database, cell populations, signaling pathways, and metabolic pathways were compared between the polyps of the GF Clec7a^{-/-} mice and the polyps of the WT mice.

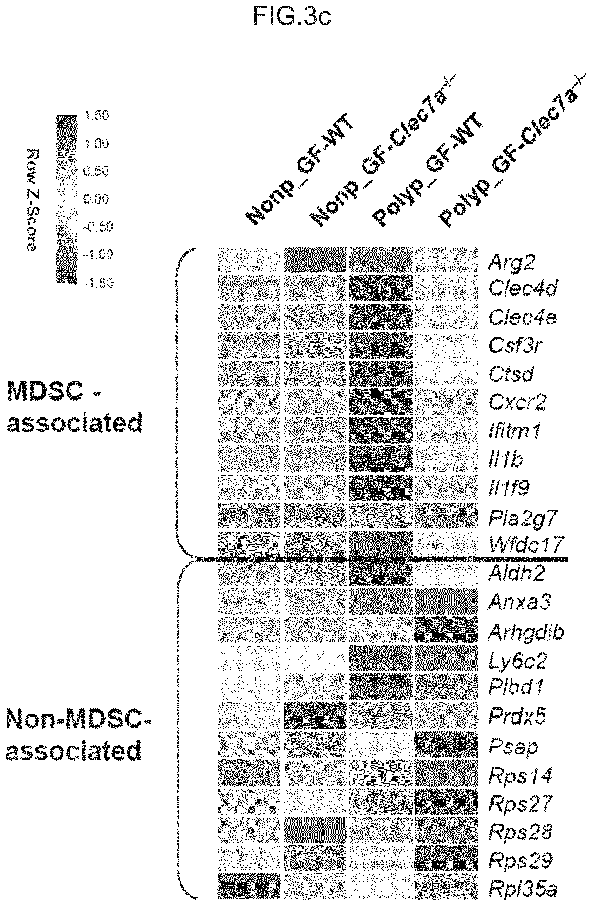

Fig. 3c: Comparison between WT mice and Clec7a^{-/-} mice for MDSC- and non-MDSC-associated genes is shown as a heatmap.

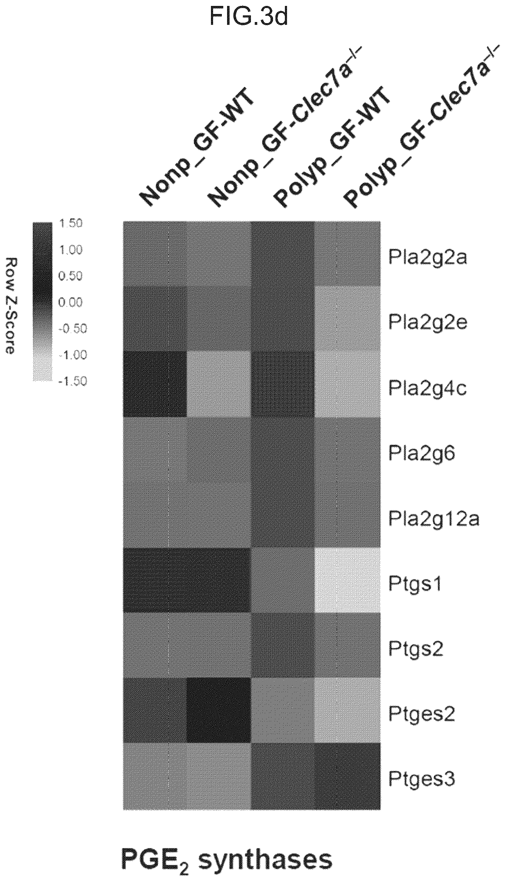

Fig. 3d: Comparison between WT mice and Clec7a^{-/-} mice for prostaglandin E₂ (PGE₂) synthase-encoding genes is shown as a heatmap.

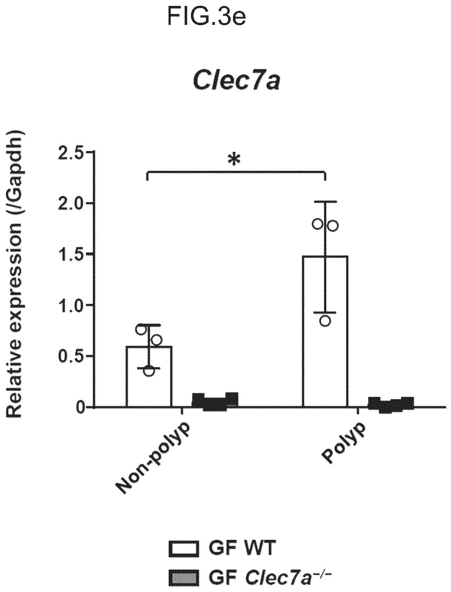

Fig. 3e: Dectin-1 mRNA (Clec7a) expression in colorectal polyps and non-polyp tissues of the GF WT mice and the Clec7a^{-/-} mice was examined by qPCR (WT n = 3, and Clec7a^{-/-} n = 4). The data in (Fig. 3e) are representative of two independent experiments, and expressed as mean ± SD. *p < 0.05.

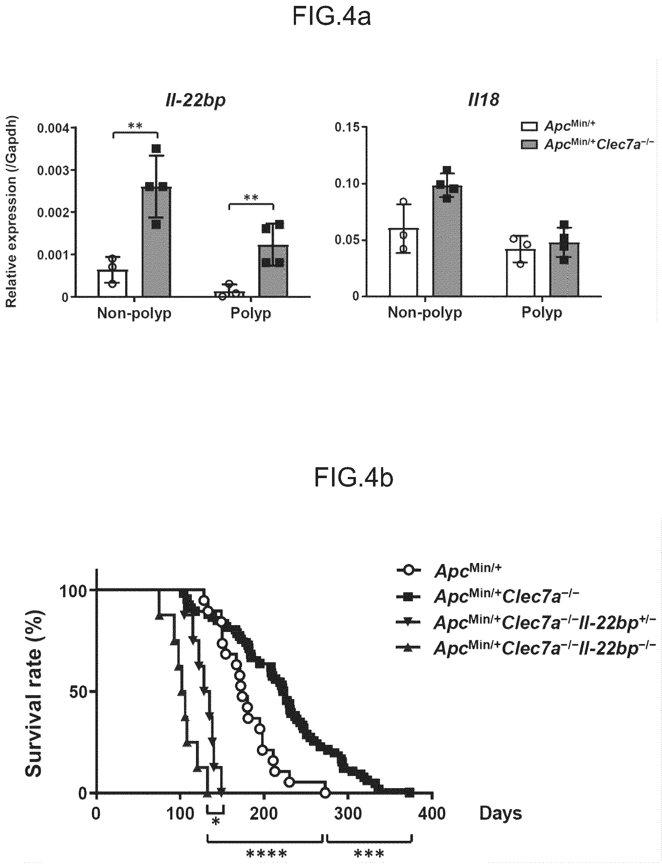

Fig. 4a: IL-22bp, the expression of which is upregulated in Clec7a^{-/-} mice, suppresses generation of intestinal tumors in Apc^{Min} mice. Apc^{Min/+} mice and Apc^{Min/+}Clec7a^{/-} mice that had been raised to the age of 20 weeks were sacrificed, and expression levels of Il-22bp and Il18 in intestinal polyps and non-polyp tissues were measured by qPCR (Apc^{Min/+} n = 3, and Apc^{Min/+}Clec7a^{-/-} n = 4).
Fig. 4b: Survival rates of indicated mice (Apc^{Min/+} n = 19, Apc^{Min/+}Clec7a^{-/-} n = 66, Apc^{Min/+}Clec7a^{-/-}Il-22bp^{+/-} n = 8, and Apc^{Min/+}Clec7a^{-/-}Il-22bp^{-/-} n = 8).

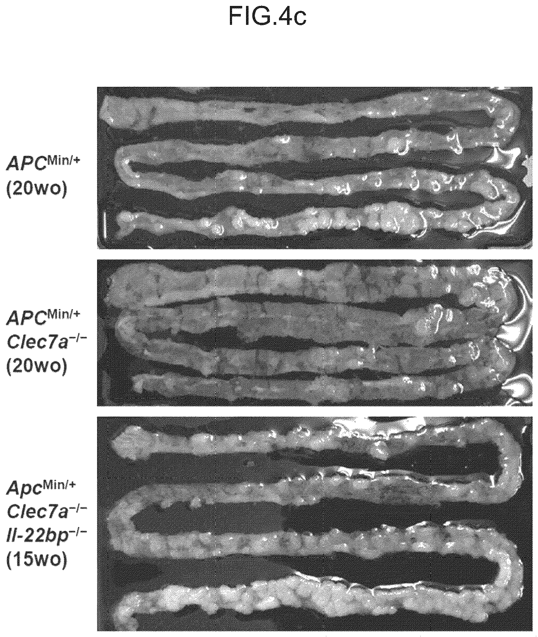

Fig. 4c: Representative photographs of small intestines of 20-week-old Apc^{Min/+} and Apc^{Min/+}Clec7a^{-/-} mice and 15-week-old Apc^{Min/+}Clec7a^{/-}Il-22bp^{-/-} mice.

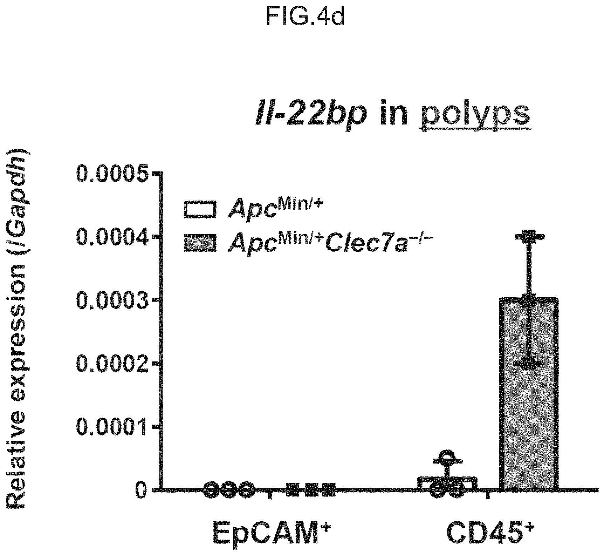

Fig. 4d: All the cells of the intestinal polyps collected from 23-week-old Apc^{Min/+} and Apc^{Min/+}Clec7a^{-/-} mice were separated into epithelial cells and leucocytes by autoMACS. Thereafter, Il-22bp expression was examined by qPCR.

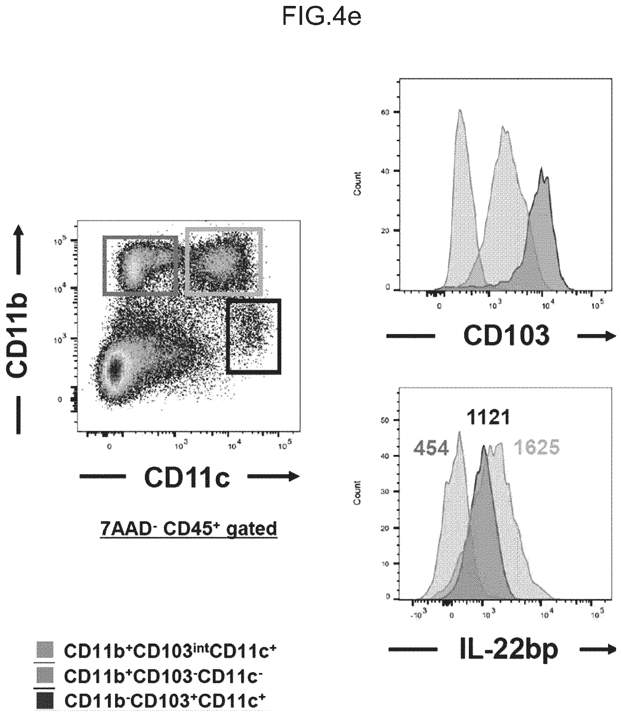

Fig 4e: Polyp-infiltrating cells from Apc^{Min/+} mice were analyzed by flow cytometry to examine intracellular expression of IL-22bp in three indicated myeloid cell populations. The numbers in the IL-22bp panel represent mean fluorescence intensities.

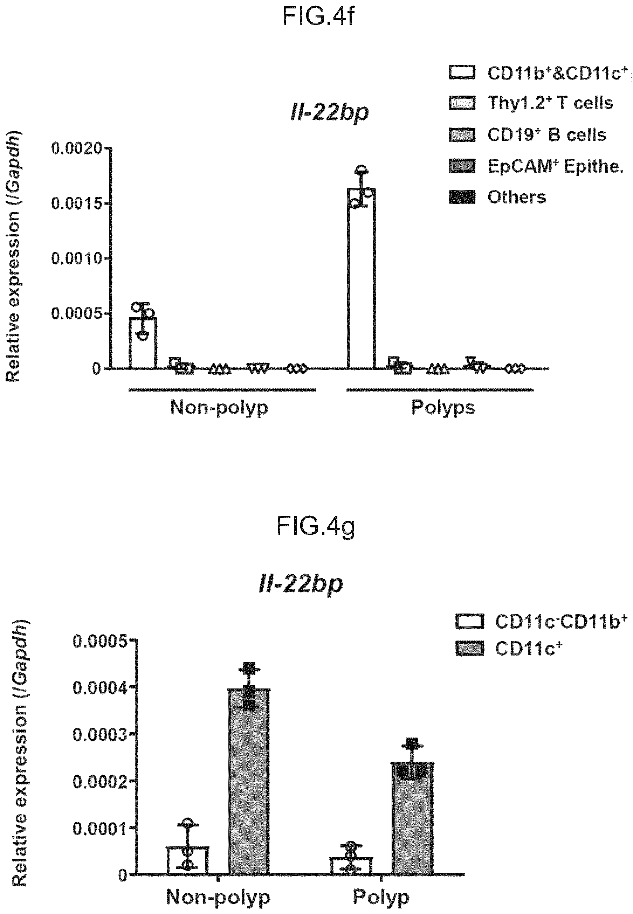

Fig. 4f: Clec7a^{-/-} mice were administered with AOM, followed by administration of 1% by mass of DSS for three cycles, and were sacrificed 12 weeks after the AOM administration. Indicated types of cells in polyps and non-polyp tissues were purified by autoMACS, and Il-22bp expression was examined by qPCR.
Fig. 4g: CD11c⁺ cells and CD11c-CD11b⁺ cells were purified by autoMACS, and expression of Il-22bp in these subsets was examined by qPCR.

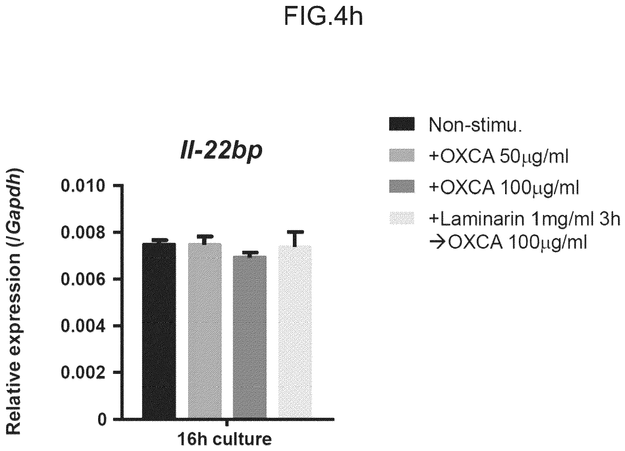

Fig. 4h: C57BL/6J mice were administered with AOM, and were administered with DSS for three cycles. After 11 weeks from the AOM administration, the mice were sacrificed and CD11b⁺CD11c⁺ cells were purified from the colorectal polyps. The cells were then stimulated with OXCA and expression of Il-22bp was measured by qPCR. In the last experimental group, laminarin treatment was performed 3 hours before OXCA treatment. The data in (Fig. 4a and Figs. 4d to 4h) are representative of two independent experiments, and the data in (Figs. 4a, 4d, and 4f to 4h) are expressed as mean ± SD. **p < 0.01, ***p < 0.001, and ****p < 0.0001.

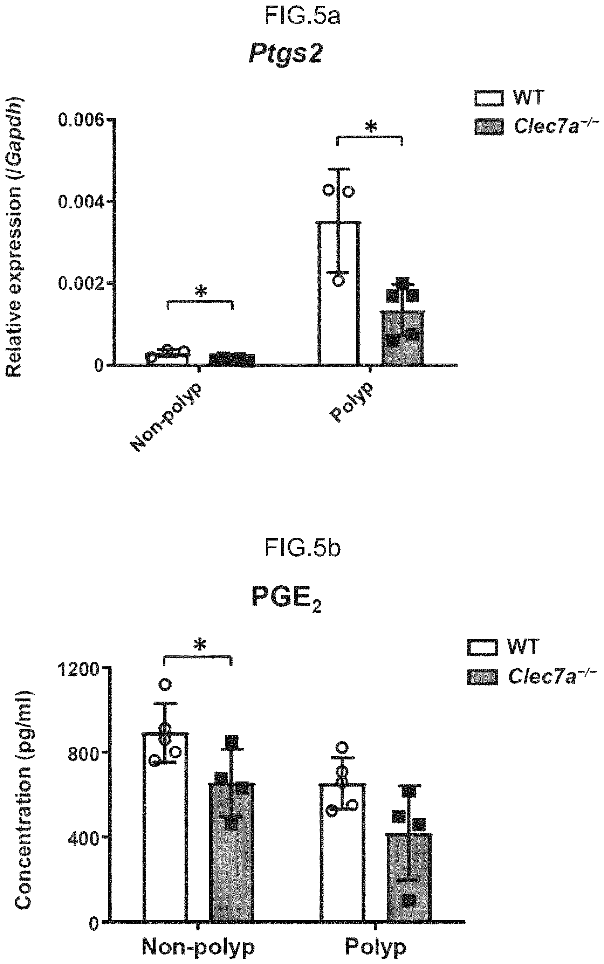

Fig. 5a: Genes involved in PGE₂ synthesis are activated by Dectin-1 signaling. WT mice and Clec7a^{-/-} mice were administered with AOM followed by 1% by mass of DSS administration for three cycles, and sacrificed after 16 weeks from the AOM administration. The expression of Ptgs2, which encodes Cox2, in colon polyps and non-polyp sites was measured by qPCR (WT n = 3, and Clec7a^{-/-} n = 4).
Fig. 5b: WT mice and Clec7a^{-/-} mice were administered with AOM, followed by administration of 1% by mass of DSS for three cycles, and were sacrificed after 16 weeks from the AOM administration. Concentrations of PGE₂ in tissue homogenates were measured by ELISA (WT n = 5, and Clec7a^{-/-} n = 4).

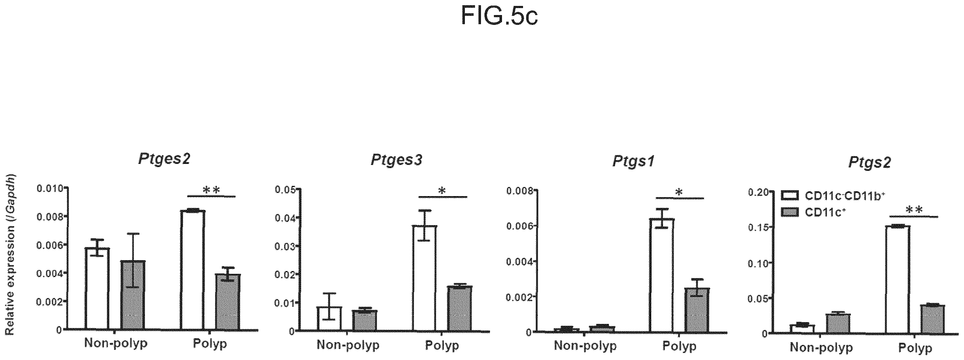

Fig. 5c: C57BL/6J mice were administered with AOM, followed by administration of 1% by mass of DSS for three cycles, and were sacrificed after 12 weeks from the AOM administration. CD11c⁺ cells and CD11c⁻CD11b⁺ cells were purified by autoMACS from colorectal polyps and non-polyp tissues, and mRNA expression of indicated genes was measured by qPCR.

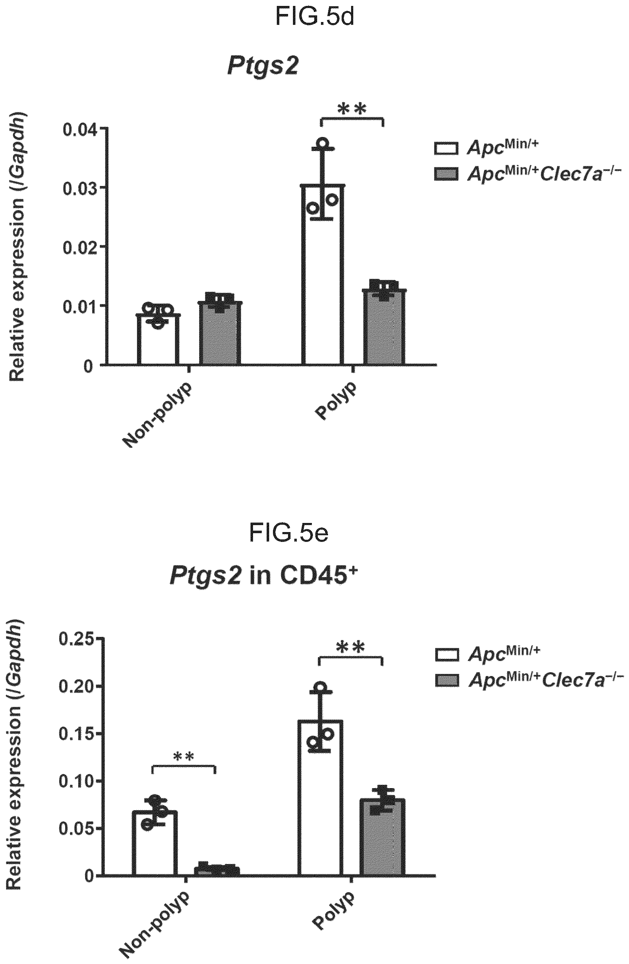

Fig. 5d: The expression of Ptgs2 in intestinal polyps and non-polyp tissues of 20-week-old Apc^{Min/+} and Apc^{Min/+}Clec7a^{-/-} mice was measured by qPCR (n = 3/group).
Fig. 5e: The expression of Ptgs2 in tissue-infiltrating CD45+ leucocytes of 20-week-old Apc^{Min/+} and Apc^{Min/+}Clec7a^{-/-} mice was measured by qPCR (n = 3/group).

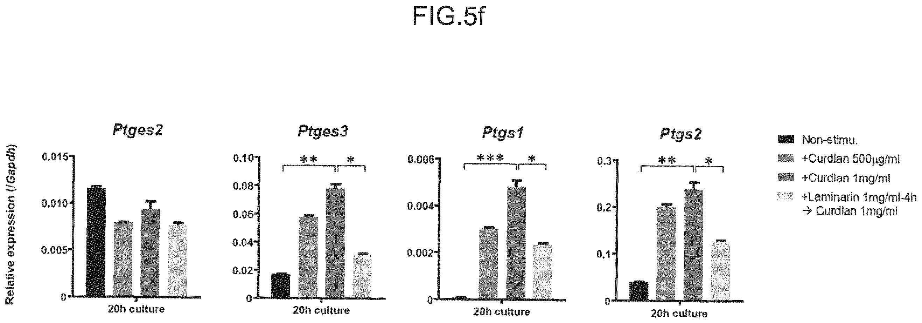

Fig. 5f: All polyp-infiltrating cells of the Apc^{Min/+} mice were collected, and were stimulated with Curdlan for 20 hours, and then the expression of the genes involved in the PGE₂ synthesis was measured by qPCR. A laminarin treatment was performed for 4 hours before the Curdlan treatment.

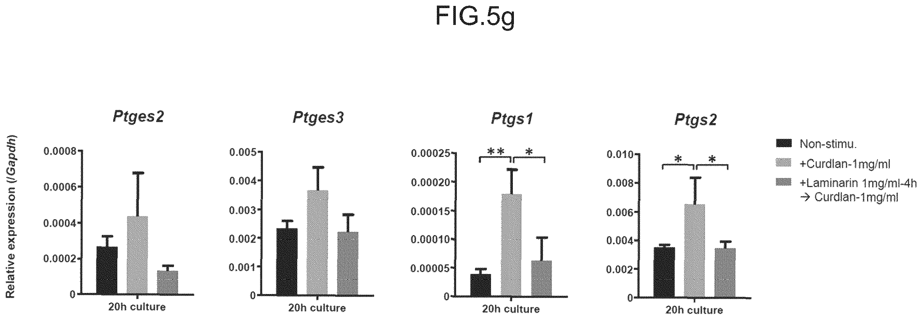

Fig. 5g: Purified CD11b⁺ polyp-infiltrating cells of the Apc^{Min/+} mice were collected, and were stimulated with Curdlan for 20 hours, and then the expression of the genes involved in the PGE₂ synthesis was measured by qPCR. A laminarin treatment was performed for 4 hours before the Curdlan treatment. The data in (Figs. 5a to 5g) are representative of two independent experiments and expressed as mean ± SD. *p < 0.05, **p < 0.01, and ***p < 0.001.

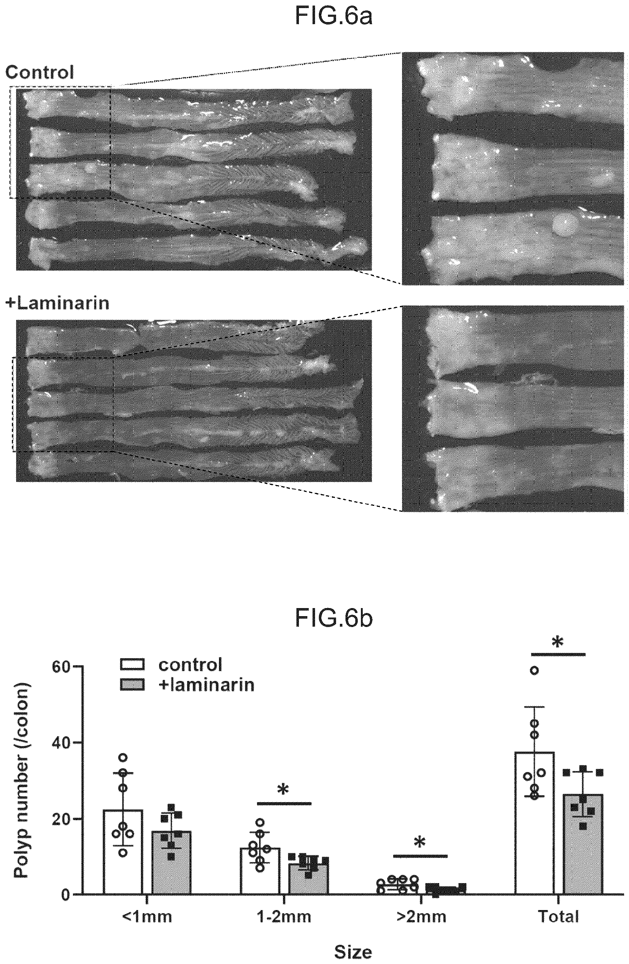

Fig. 6a: Oral administration of laminarin suppresses tumor formation in the large intestine, and PGE₂ administration promotes tumor formation. AOM was administered to C57BL/6J mice, and 5% by mass of laminarin-containing powdered food was administered (continued for 10 days/cycle) from 3 days before to the end of the one-week administration of DSS, for three cycles in total. At Week 11 from the first AOM administration, the mice were sacrificed and the generation of tumors in the large intestines was observed macroscopically (n = 7/group).
Fig. 6b: AOM was administered to C57BL/6J mice, and 5% by mass of laminarin-containing powdered food was administered (continued for 10 days/cycle) from 3 days before to the end of the one-week administration of DSS, for three cycles in total. At Week 11 from the first AOM administration, the mice were sacrificed and the number of the colorectal polyps with indicated sizes was counted (n = 7/group).

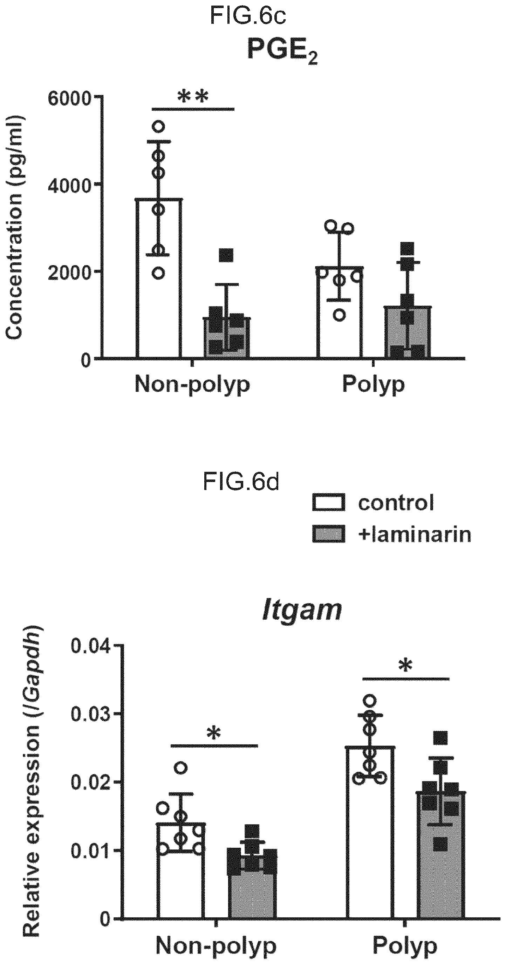

Fig. 6c: AOM was administered to C57BL/6J mice, and 5% by mass of laminarin-containing powdered food was administered (continued for 10 days/cycle) from 3 days before to the end of the one-week administration of DSS, for three cycles in total. The mice were sacrificed at Week 11 after the first AOM administration and the concentration of PGE₂ in tissue homogenates was measured by the ELISA (n = 7/group).
Fig. 6d: AOM was administered to C57BL/6J mice, and 5% by mass of laminarin-containing powdered food was administered (continued for 10 days/cycle) from 3 days before to the end of the one-week administration of DSS, for three cycles in total. The mice were sacrificed at Week 11 from the first AOM administration and an mRNA expression of CD11b (Itgam) was examined by qPCR (n = 7/group).

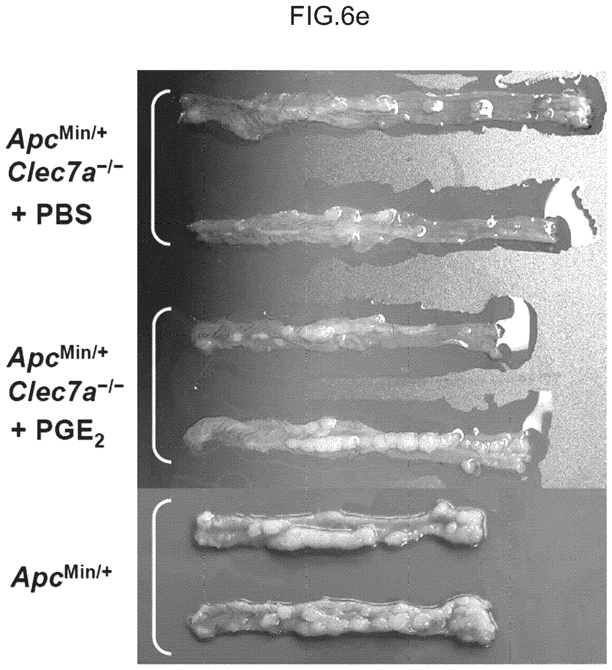

Fig. 6e: 8-week-old Apc^{Min/+}Clec7a^{-/-} mice (n = 3) were administered with 2% by mass of DSS for 1 week, and then were administered with PGE₂ (40 g/mouse) or PBS intraperitoneally every other day for 4 weeks. After euthanization, generation of colorectal polyps in the Apc^{Min/+}Clec7a^{-/-} mice with or without PGE₂ treatment was examined in comparison to that of the Apc^{Min/+} mice (n = 3/group).

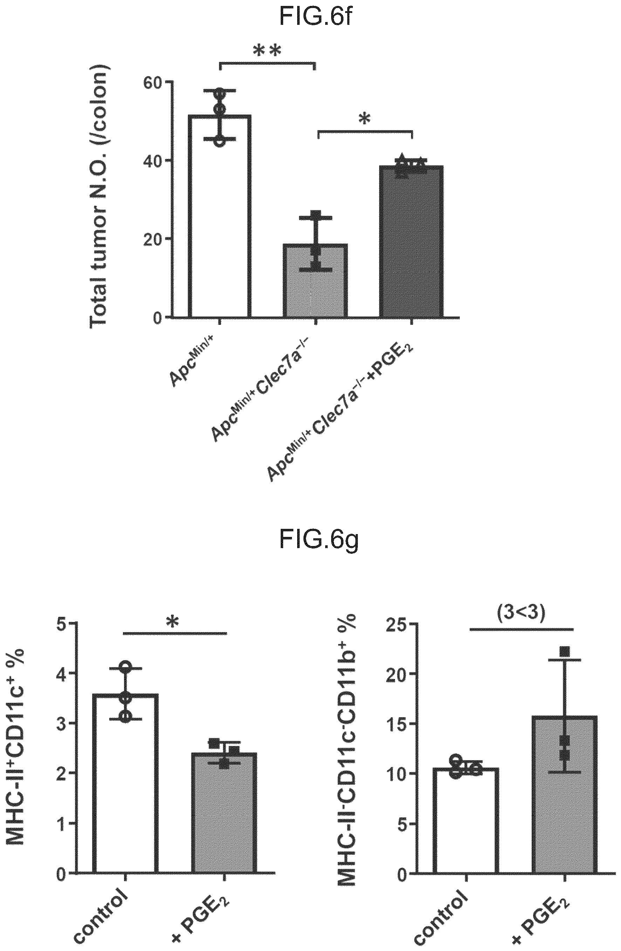

Fig. 6f: 8-week-old Apc^{Min/+}Clec7a^{-/-} mice (n = 3) were administered with 2% by mass of DSS for 1 week, and then were administered with PGE₂ (40 g/mouse) or PBS intraperitoneally every other day for 4 weeks. After euthanization, generation of colorectal polyps in the Apc^{Min/+}Clec7a^{-/-} mice with or without the PGE₂ treatment was examined in comparison to that of the Apc^{Min/+} mice (n = 3/group).
Fig. 6g: 8-week-old Apc^{Min/+}Clec7a^{-/-} mice (n = 3) were administered with 2% by mass of DSS for 1 week, and then were administered with PGE₂ (40 g/mouse) or PBS intraperitoneally every other day for 4 weeks. After euthanization, populations of MHC-II⁺CD11c⁺DCs and MHC-II⁻CD11b⁺MDSCs in the polyps in the Apc^{Min/+}Clec7a^{-/-} mice were measured by flow cytometry (n = 3/group).

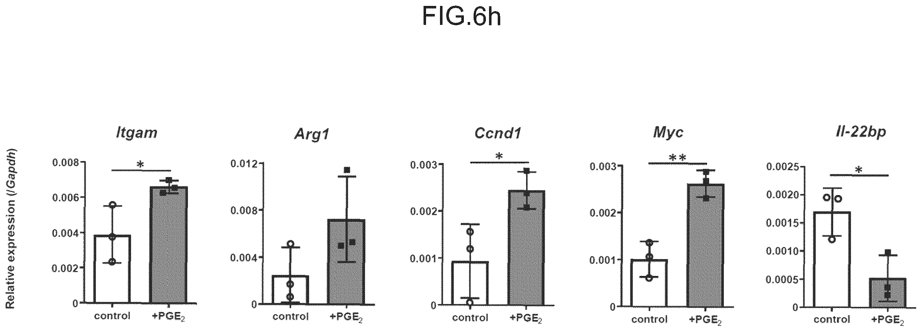

Fig. 6h: 8-week-old Apc^{Min/+}Clec7a^{-/-} mice (n = 3) were administered with 2% by mass of DSS for 1 week, and then administered with PGE₂ (40 g/mouse) or PBS intraperitoneally every other day for 4 weeks. After euthanization, expression of indicated genes in the Apc^{Min/+}Clec7a^{-/-} mice was examined by qPCR (n = 3/group).

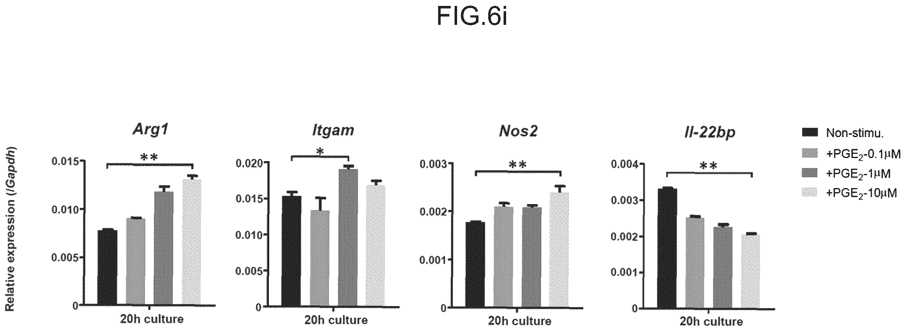

Fig. 6i: Tumor-infiltrating cells of the Apc^{Min/+} mice (n = 2, cells that were pooled during collection) were collected, stimulated with indicated doses of PGE₂ for 20 hours, and then the expression of indicated genes was measured by qPCR. The data are mean ± SD of 3 wells.

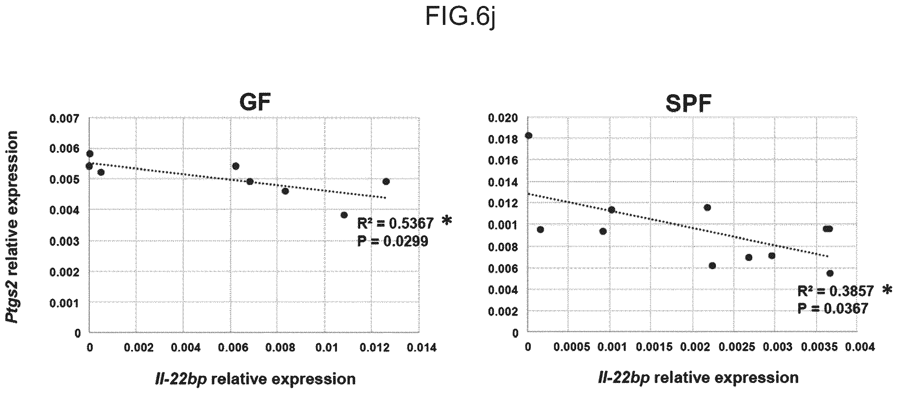

Fig. 6j: A correlation between the expression levels of Il-22bp and Ptgs2 in colorectal polyps of GF mice or SPF mice after AOM + DSS treatment (n = 8/GF group, and n = 11/SPF group) is shown. (Figs. 6a to 6h) are representative of two independent experiments, and (Fig. 6i) is representative of three independent experiments. The data in (Figs. 6b to 6d and 6f to 6i) are expressed as mean ± SD. *p < and **p < 0.01.

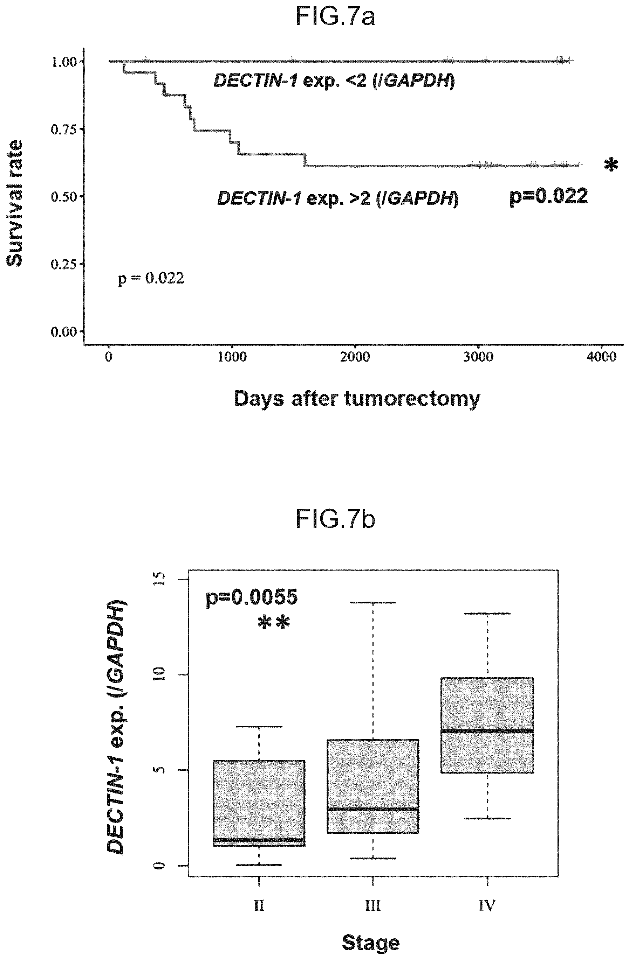

Fig. 7a: The expression of PGE₂-synthesizing enzymes and IL22RA2 is modified in CRC patients. Specimens from 36 CRC patients were collected and the post-surgery survival rates of the patients with Dectin-1 mRNA expression of less than 2 (n = 12) or from 2 to 20 (n = 24) (normalized by GAPDH) in tumor tissues are shown.
Fig. 7b: Specimens from 36 CRC patients were collected, and the correlation between Dectin-1 expression levels and tumor TNM stage of the patients with Dectin-1 mRNA expression of less than 2 (n = 12) or from 2 to 20 (n = 24) (normalized by GAPDH) in tumor tissues is shown. Tumor TNM stage was determined according to the individual clinical information.

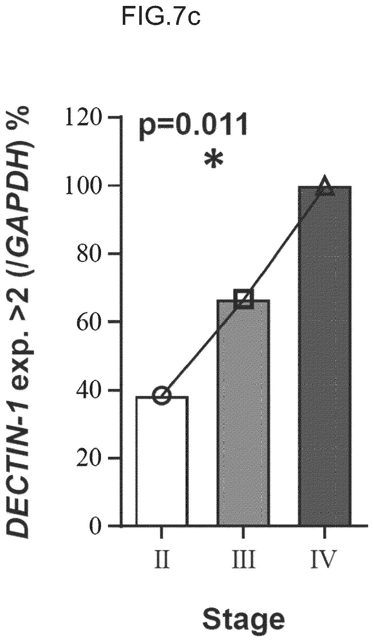

Fig. 7c: Specimens from 36 CRC patients were collected, and the correlation between each stage and the proportion of patients with Dectin-1 mRNA expression of less than 2 in patients with Dectin-1 mRNA expression of less than 2 (n = 12) and from 2 to 20 (n = 24) (normalized by GAPDH) in tumor tissues is shown. Tumor TNM stage was determined according to the individual clinical information.

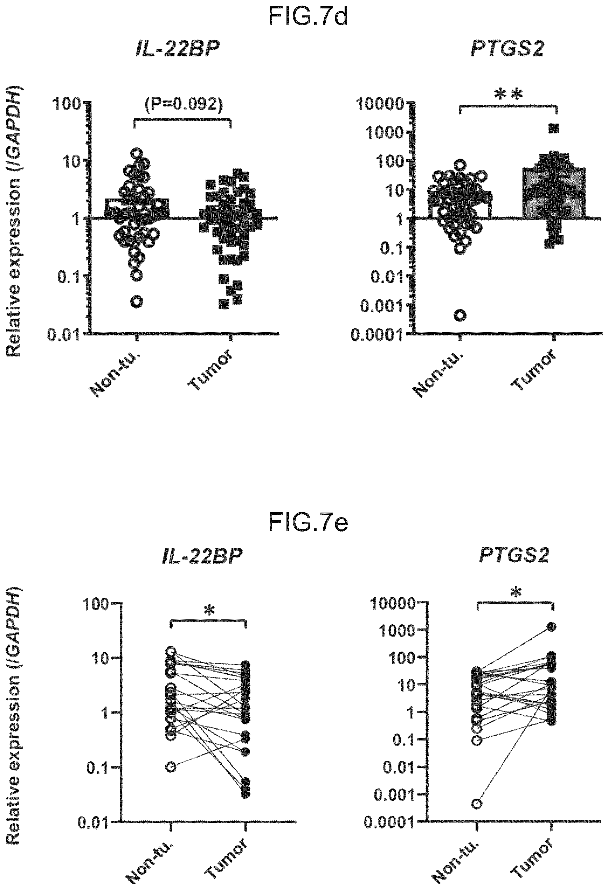

Fig. 7d: From a total of 68 CRC patients, 23 pairs of tumors and non-tumor specimens from the same individuals were collected. IL22RA2 and PTGS2 expression in tumors and non-tumor tissues was examined by qPCR (n = 47 in tumor group, n = 44 in non-tumor group).
Fig. 7e: 23 pairs of tumors and non-tumor specimens from the same individuals were collected, and IL-22BP and PTGS2 expression was examined (n = 23/group).

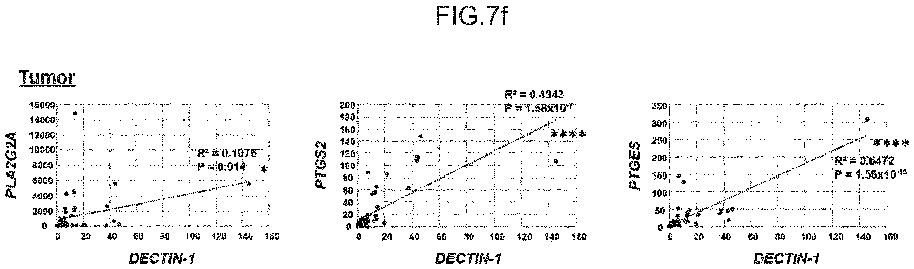

Fig. 7f: Correlations of expression levels between PGE₂ synthesizing enzymes and Dectin-1 in CRC tissues (n = 47/group).

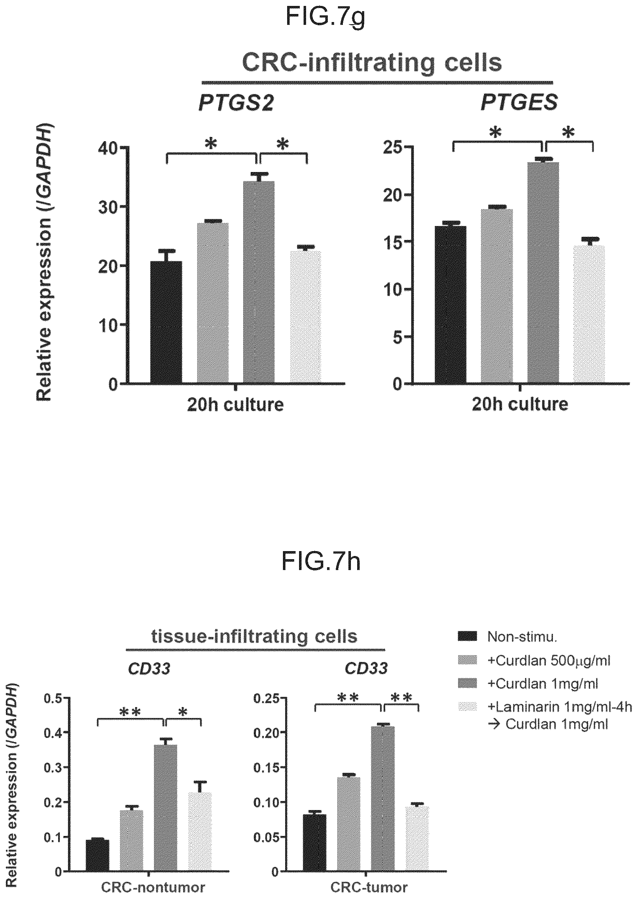

Fig. 7g: CRC samples were collected, and tumor-infiltrating cells were stimulated with Curdlan for 20 hours. In the case of the laminarin treatment, the cells were first treated with laminarin for 4 hours, and thereafter, were supplied with Curdlan and cultured. Expression of PGE₂ synthesizing enzymes was determined by qPCR. Data are mean ± SD of 3 wells.
Fig. 7h: CRC samples were collected, and tumor-infiltrating cells and normal tissue-infiltrating cells were stimulated with Curdlan for 20 hours. In the case of the laminarin treatment, the cells were first treated with laminarin for 4 hours, and thereafter, were supplied with Curdlan and cultured. Expression of CD33 was determined by qPCR. Data are mean ± SD of 3 wells.

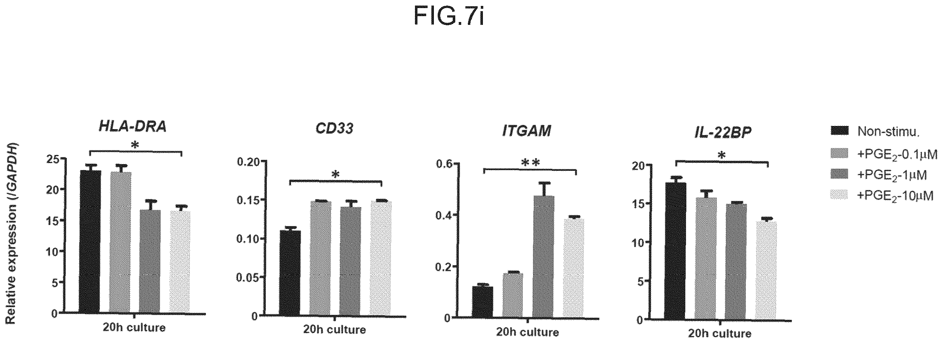

Fig. 7i: Tumor tissues of CRC patients were collected, minced, and then cultured in the presence of PGE₂ for 20 hours. Data are mean ± SD of 3 wells. The data in (Figs. 7g to 7i) are representative of two independent experiments. *p < **p < 0.01, and ****p < 0.0001.

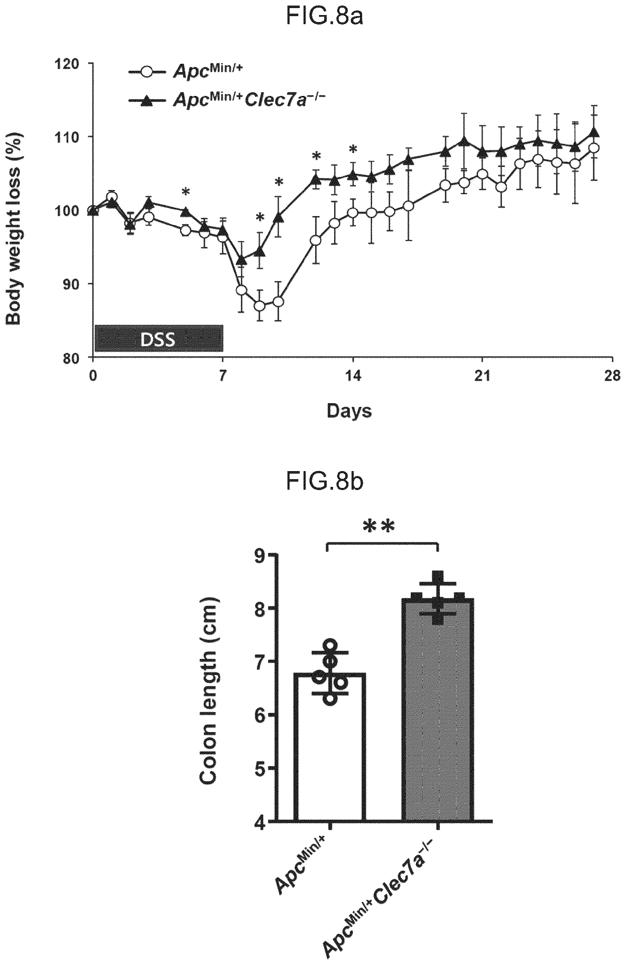

Fig. 8a: Dectin-1 deficiency suppresses colorectal tumors, and this suppression is independent of commensal microbiota. Apc^{Min/+} mice and Apc^{Min/+}Clec7a^{-/-} mice at 8 to 10 weeks old were administered with 1% by mass of DSS for 7 days and were sacrificed 4 weeks later. Changes in the body weight were measured in time series after the DSS administration (n = 5/group).
Fig. 8b: Apc^{Min/+} mice and Apc^{Min/+}Clec7a^{-/-} mice at the ages of from 8 to 10 weeks were administered with 1% by mass of DSS for 7 days and were sacrificed 4 weeks later. After dissection, the lengths of the colon were measured (n = 5/group).

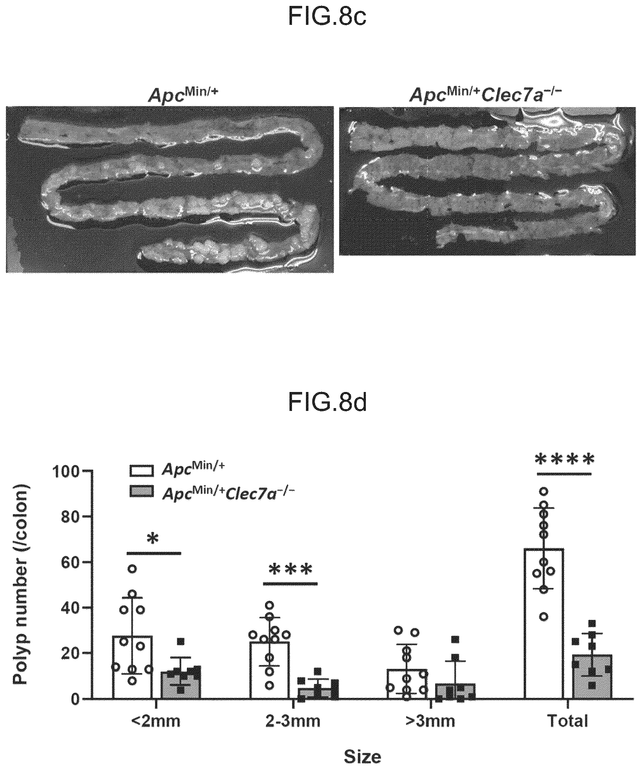

Fig. 8c: Apc^{Min/+} mice and Apc^{Min/+}Clec7a^{-/-} mice were sacrificed at the ages of from 20 to 23 weeks. Gross observation of small intestinal tumors of the mice was performed (n = 10/Apc^{Min/+} group, and n = 8/Apc^{Min/+}Clec7a^{-/-} group).
Fig. 8d: Apc^{Min/+} mice and Apc^{Min/+}Clec7a^{-/-} mice were sacrificed at the ages of from 20 to 23 weeks. The number of polyps in the mice was counted (n = 10/Apc^{Min/+} group, and n = 8/Apc^{Min/+}Clec7a^{-/-} group).

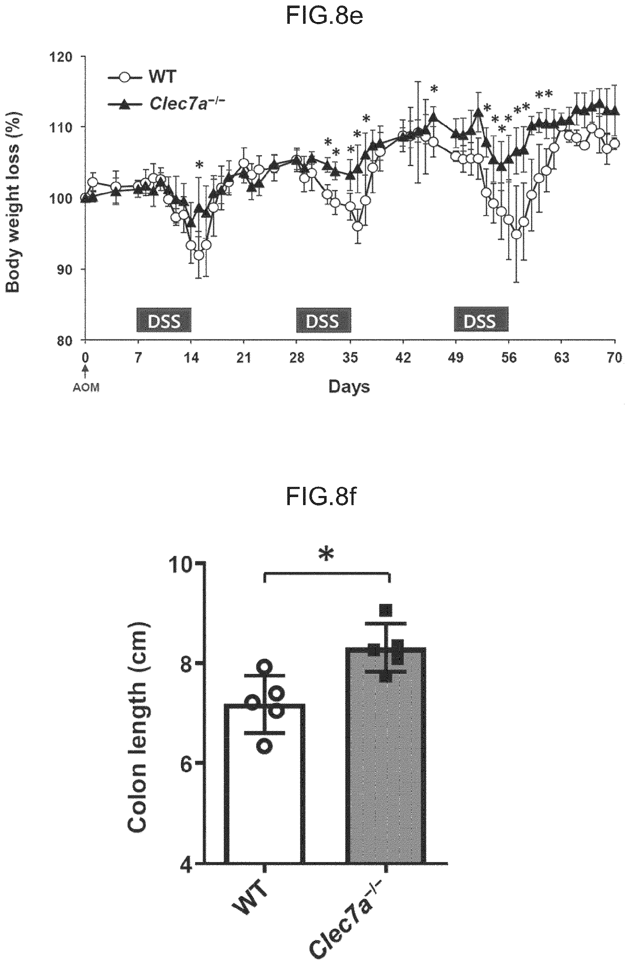

Fig. 8e: WT and the Clec7a^{-/-} mice that had been separately raised were intraperitoneally administered with AOM, and were administered 7 days later with 1% by mass of DSS for three cycles, as described in "Materials and Methods" to be described later. Body weight loss was measured over time during the induction of the colorectal tumors (n = 5/group).
Fig. 8f: WT and Clec7a^{-/-} mice that had been separately raised were intraperitoneally administered with AOM, and were administered 7 days later with 1% by mass of DSS for three cycles, as described in "Materials and Methods" to be described later. At Week 16 from the first AOM administration, the mice were sacrificed and the lengths of the colon were measured.

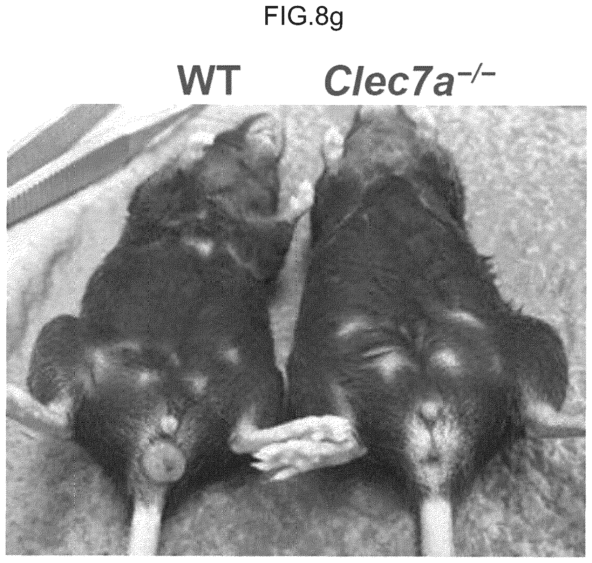

Fig. 8g: WT mice and Clec7a^{-/-} mice were administered with AOM, and were administered 7 days later with 1% by mass of DSS only for 7 days, and then were subjected to normal breeding for another 10 weeks before being sacrificed (WT n = 8, and Clec7a^{-/-} n = 9). Anal prolapse of the mice was observed.

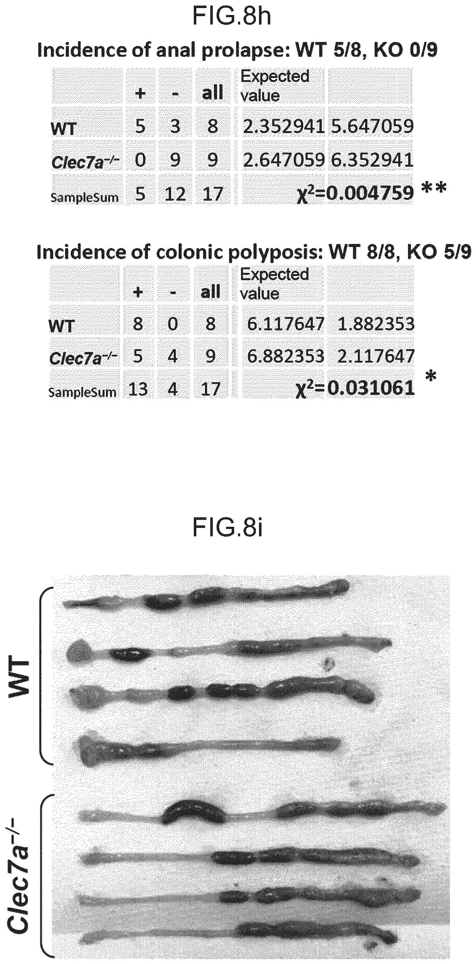

Fig. 8h: WT mice and Clec7a^{-/-} mice were administered with AOM, and were administered 7 days later with 1% by mass of DSS only for 7 days, and then were subjected to normal breeding for another 10 weeks before being sacrificed (WT n = 8, and Clec7a^{-/-} n = 9). The incidence rates of anal prolapse and colorectal tumors in the mice were statistically calculated.
Fig. 8i: WT mice and Clec7a^{-/-} mice were administered with AOM, and were administered 7 days later with 1% by mass of DSS only for 7 days, and then were subjected to normal breeding for another 10 weeks before being sacrificed (WT n = 8, and Clec7a^{-/-} n = 9). The lengths of the colon were measured.

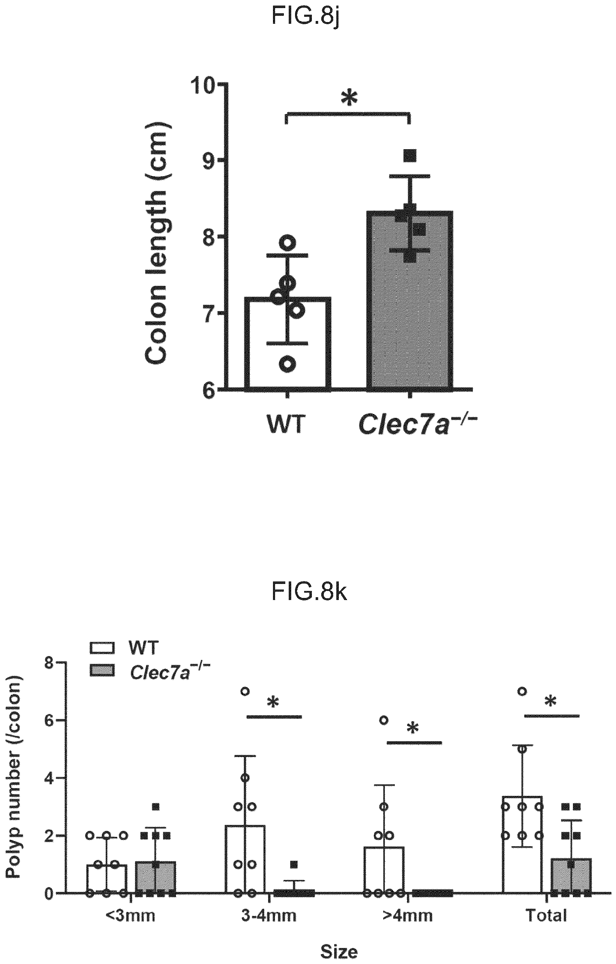

Fig. 8j: WT mice and Clec7a^{-/-} mice were administered with AOM, and were administered 7 days later with 1% by mass of DSS only for 7 days, and then were subjected to normal breeding for another 10 weeks before being sacrificed (WT n = 8, and Clec7a^{-/-} n = 9). The lengths of the colon were measured.
Fig. 8k: WT mice and Clec7a^{-/-} mice were administered with AOM, and were administered 7 days later with 1% by mass of DSS only for 7 days, and then were subjected to normal breeding for another 10 weeks before being sacrificed (WT n = 8, and Clec7a^{-/-} n = 9). The number of rectal polyps in the mice was measured.

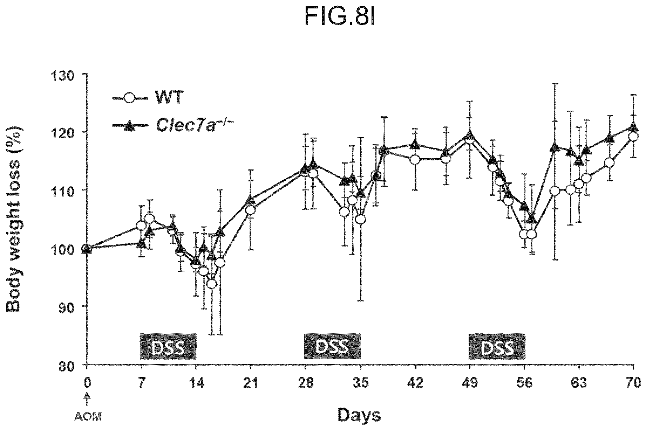

Fig. 8l: WT mice and Clec7a^{-/-} mice were co-housed after weaning at 4 weeks old, and were intraperitoneally administered 4 weeks later with AOM, followed by three cycles of 1% by mass of DSS (as described in "Materials and Methods"). Body weight loss was measured over time from the start of the first DSS administration (n = 5/group), as shown in the horizontal axis (n = 5/group).

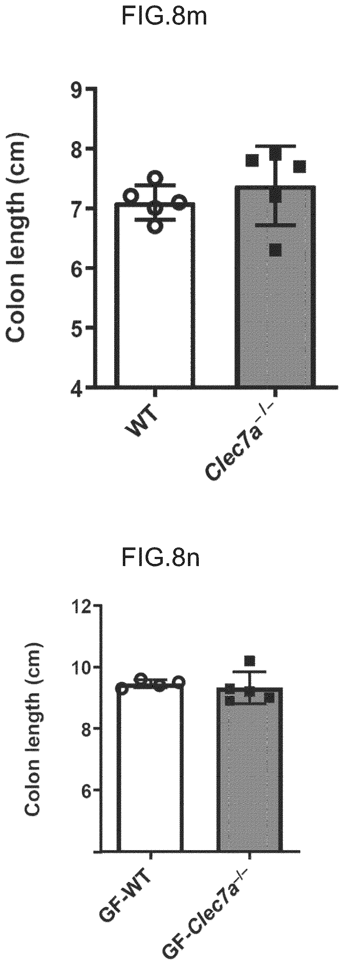

Fig. 8m: WT mice and Clec7a^{-/-} mice were co-housed after weaning at 4 weeks old, and were intraperitoneally administered 4 weeks later with AOM, followed by three cycles of 1% by mass of DSS (as described in "Materials and Methods"). At Week 16 from the first AOM administration, the mice were sacrificed and the lengths of the colon were measured (n = 5/group).
Fig. 8n: Sterile (GF) WT mice and GF Clec7a^{-/-} mice were administered with AOM, and then were administered with 1% by mass of DSS for three cycles. After 36 weeks from the AOM administration, the mice were sacrificed and the lengths of the colon were measured. The data in (Figs. 8a, 8b, 8e, 8f, 8l, and 8m) are representative of three experiments, and the data in (Fig. 8n) are representative of two independent experiments. The Data in (Figs. 8c and 8d) are pooled data from three independent experiments. The data in (Figs. 8a, 8b, 8d, 8e, 8f, and 8j to 8n) are expressed as mean ± SD. *p < 0.05, **p < 0.01, ***p < 0.001, and ****p < 0.0001.

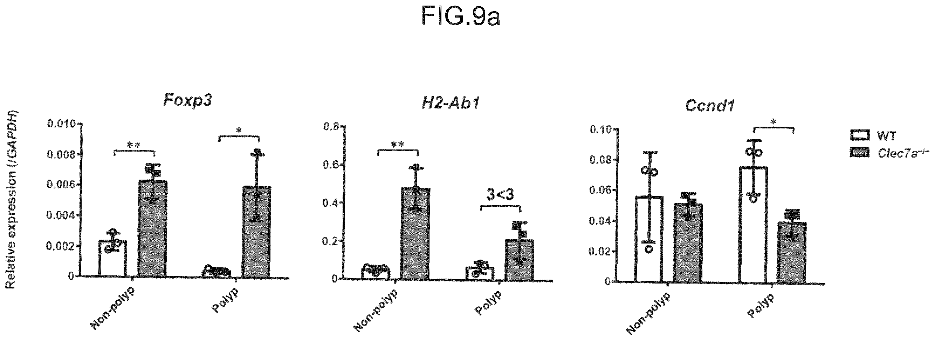

Fig. 9a: The majority of Dectin-1 expressing cells in colorectal tumors are MHC-II⁻myeloid cells. WT mice or Clec7a^{-/-} mice were administered with AOM, and were administered 7 days later with 1% by mass of DSS for 7 days for a total of three cycles. The mice were sacrificed at Week 16 after the first AOM administration, and mRNA expression of indicated genes in colon polyps or non-polyp tissues was determined by qPCR (n = 3).

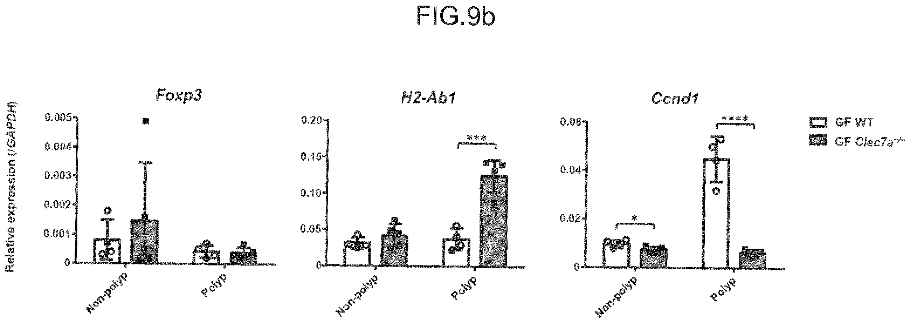

Fig. 9b: GF WT mice or GF Clec7a^{-/-} mice were administered with AOM, and were administered 7 days later with 1% by mass of DSS for 5 days for a total of three cycles. The mice were sacrificed at Week 36 after the first AOM administration, and mRNA expression of indicated genes in colon polyps or non-polyp tissues was determined by qPCR (WT n = 4, and Clec7a^{-/-} n = 5).

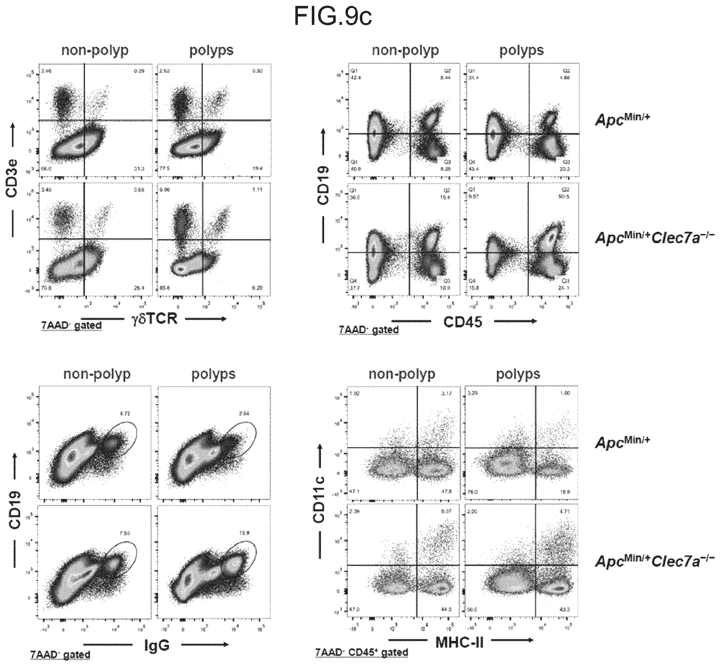

Fig. 9c: Dot plot panels showing the ratios of T cells, B cells, and DCs in tumor-infiltrating cells measured by flow cytometry, based on the experiment illustrated in Fig. 2a, are shown.

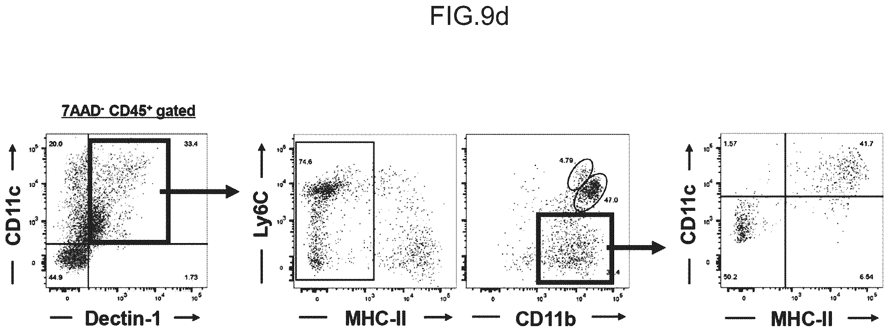

Fig. 9d: WT mice were administered with AOM, and were administered 7 days later with 1% by mass of DSS for 7 days for a total of three cycles. At Week 13 after the first AOM administration, the mice were sacrificed, and the types of Dectin-1 expressing cells infiltrating the colon polyps were measured by flow cytometry. The data in (Figs. 9a to 9d) are representative of two independent experiments.

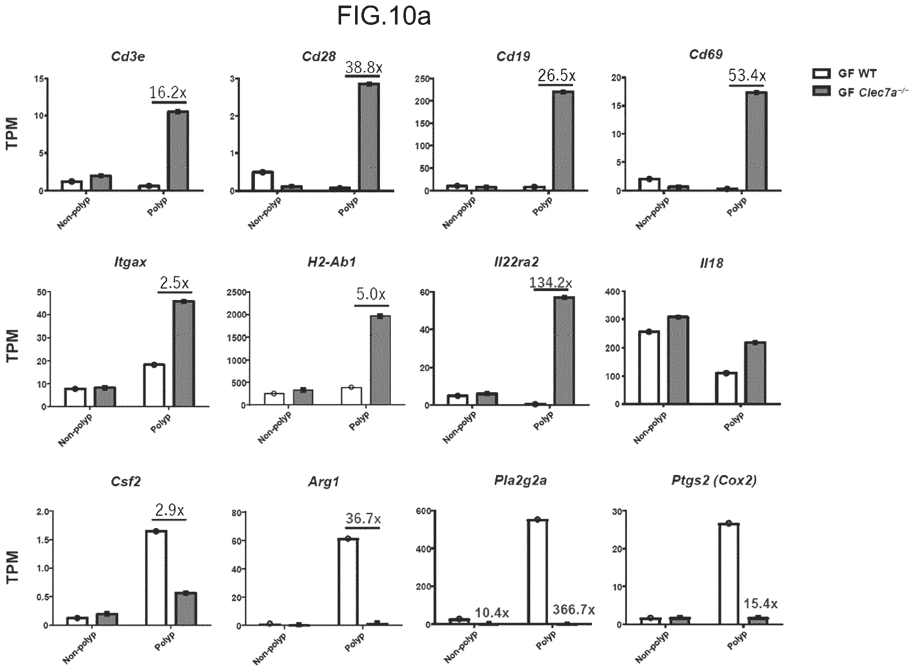

Fig. 10a: While expression of most of the genes representing the tumor-associated immune system is dependent on Dectin-1 signaling, they are not affected by commensal microbiota. Experiments illustrated in Figs. 3a to 3d were performed, and expression levels of selected genes encoding immune cell markers and tumor-associated factors in colon polyps or non-polyp tissues were determined by RNA-seq analysis.

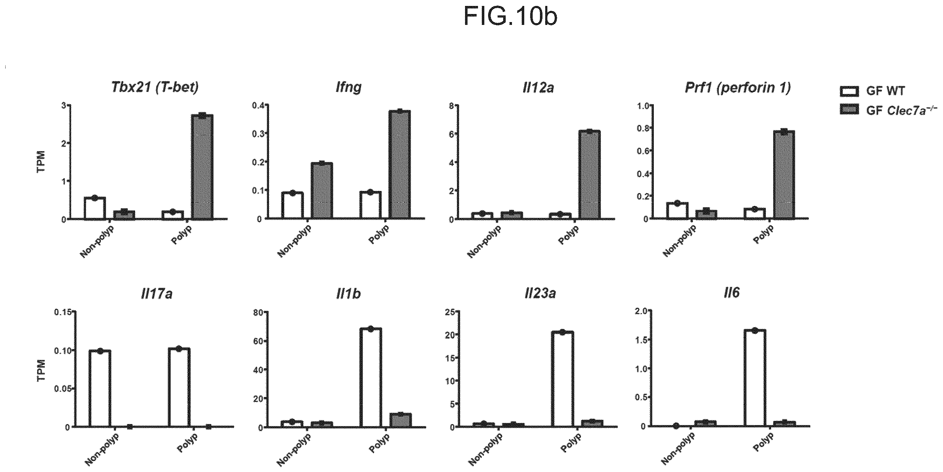

Fig. 10b: Experiments illustrated in Figs. 3a to 3d were performed, and the expression levels of genes associated with Th1 or Th17 in colon polyps or non-polyp tissues were determined by RNA-seq analysis.

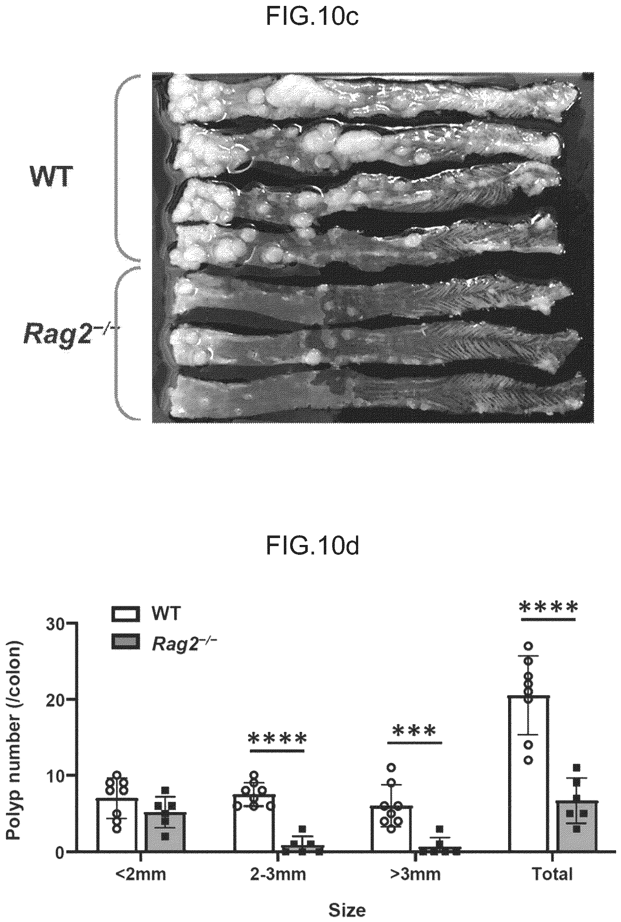

Fig. 10c: WT mice and Rag2^{-/-} mice were administered with AOM intraperitoneally, and were administered 7 days later with 1% by mass of DSS for three cycles, as described in "Materials and Methods" to be described later. After 12 weeks of the colorectal tumor induction, the mice were sacrificed, and gross observation of the colorectal tumors was performed (WT n = 8, and Rag2^{-/-} n = 6).
Fig. 10d: WT mice and Rag2^{-/-} mice were administered with AOM intraperitoneally, and were administered seven days later with 1% by mass of DSS for three cycles, as described in "Materials and Methods" to be described later. After 12 weeks of the colorectal tumor induction, the mice were sacrificed and the number of polyps were counted (WT n = 8, and Rag2^{-/-} n = 6). The data in (Figs. 10c and 10d) are representative of two independent experiments, and are expressed as mean ± SD. *p < **p < 0.01, ***p < 0.001, and ****p < 0.0001.

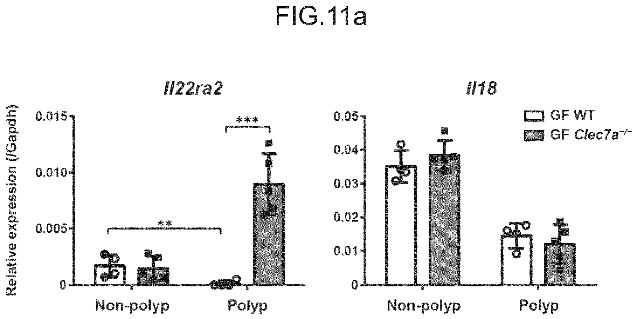

Fig. 11a: Synthesis of retinoic acid (RA) is not directly controlled by Dectin-1 signaling. RNA was prepared as illustrated in Figs. 2e to 2j, and the expression of IL-22bp (Il22ra2) and IL-18 (Il18) was measured by qPCR (WT n = 4, and Clec7a^{-/-} n = 5).

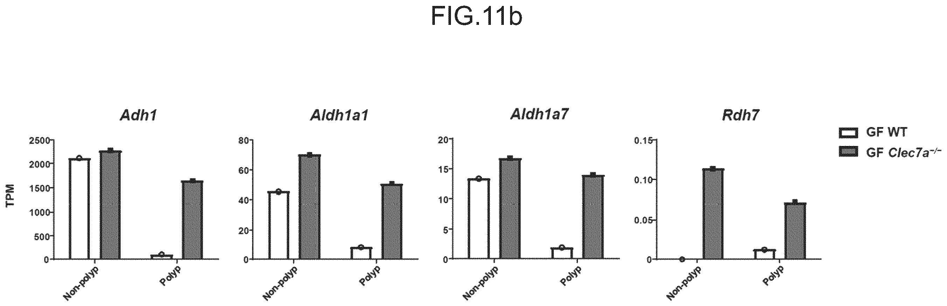

Fig. 11b: GF WT mice or GF Clec7a^{-/-} mice were administered with AOM, and were administered 7 days later with 1% by mass of DSS for five days for a total of three cycles. The mice were sacrificed at week 36 from the first AOM administration, and the expression of the genes encoding RA-associated synthases was measured by RNA-seq.

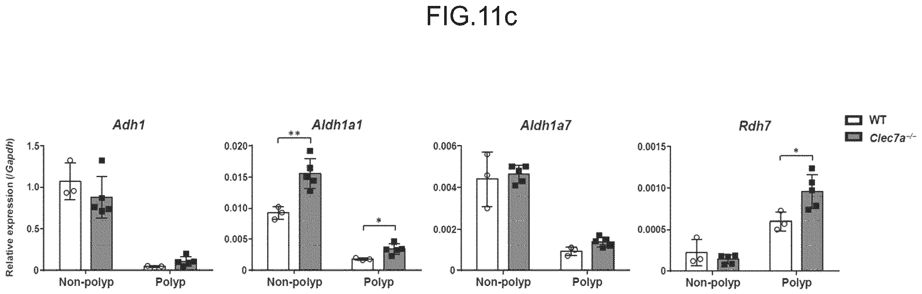

Fig. 11c: Total RNA of the polyps or non-polyp intestinal tissues from WT mice and Clec7a^{-/-} mice to which AOM was administered followed by administration of DSS for three cycles for a total of 16 weeks was collected, and the expression of indicated genes involved in the RA synthesis was examined by RTqPCR (WT n = 3, and Clec7a^{-/-} n = 5).

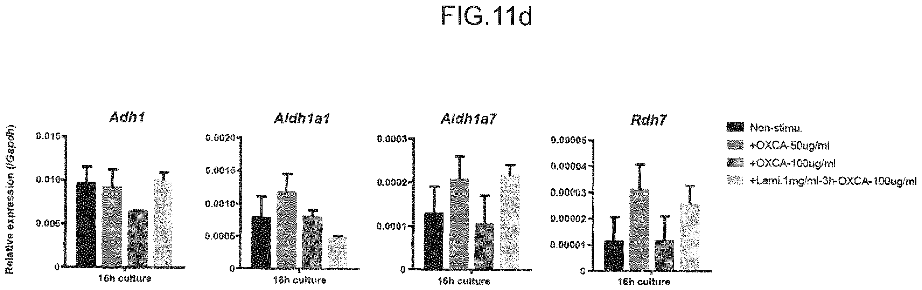

Fig. 11d: WT mice administered with AOM followed by DSS for three cycles for 12 weeks were sacrificed, and colon CD11b⁺ and CD11c⁺ myeloid-derived cells were purified by autoMACS. After 3 hours of preculture with laminarin, the cells were stimulated with OXCA for 16 hours, and mRNA expression of genes involved in RA synthesis was examined by RTqPCR. The data in (Figs. 11a, 11c, and 11d) are expressed as mean ± SD. *p < 0.05, **p < 0.01, and ***p < 0.001.

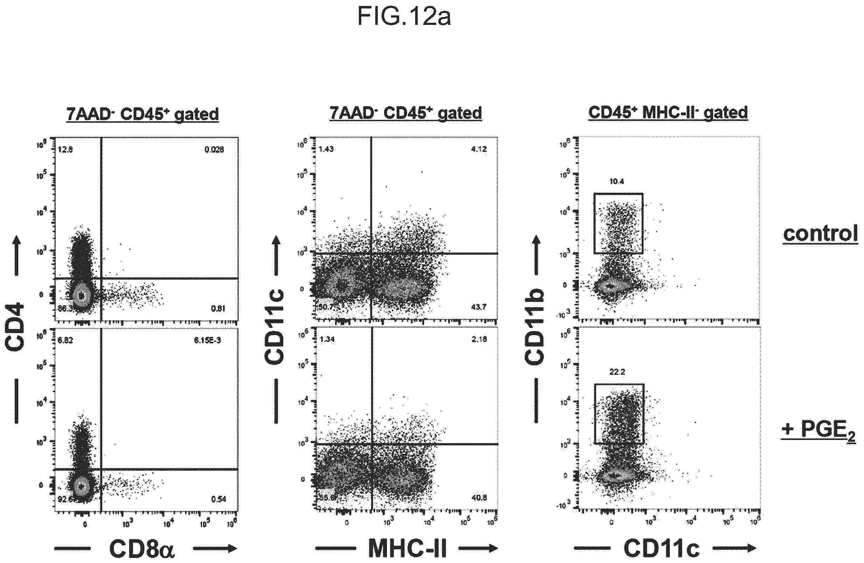

Fig. 12a: Levels of potential Dectin-1 endogenous ligands are unchanged in colorectal polyps. A dot panel of the flow cytometry analysis performed in Fig. 6i is shown.

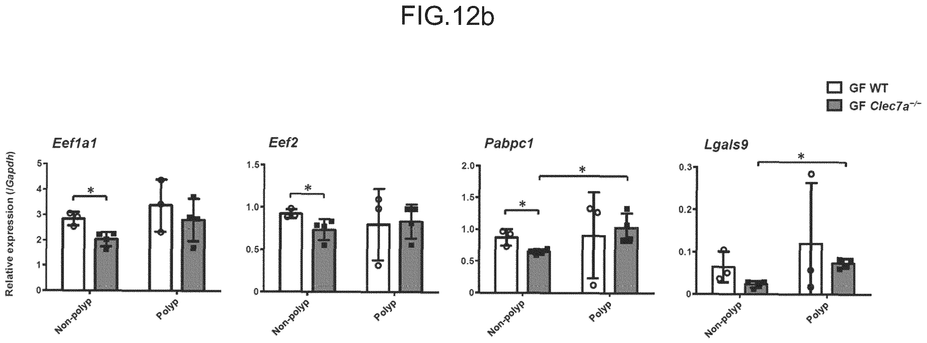

Fig. 12b: GF WT mice or GF Clec7a^{-/-} mice were administered with AOM, and were administered 7 days later with 1% by mass of DSS for 5 days for a total of three cycles. The mice were sacrificed at Week 36 from the first AOM administration, and the expression of indicated genes encoding potential Dectin-1 endogenous ligands was determined by RNA-seq.

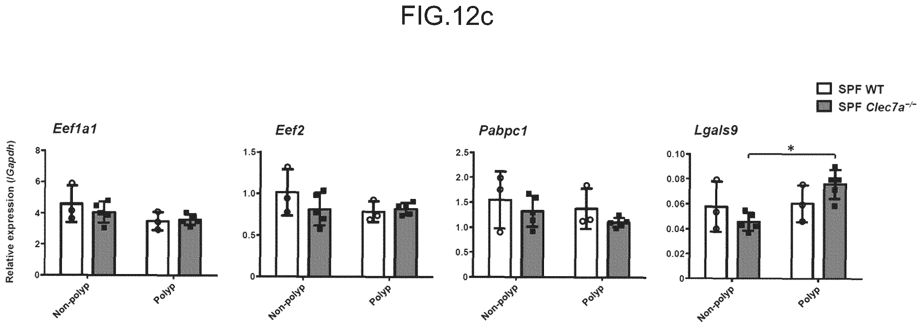

Fig. 12c: Total RNA of polyps or non-polyp intestinal tissues from WT mice and Clec7a^{-/-} mice administered with AOM followed by DSS administration for three cycles for a total of 16 weeks was collected, and the expression of indicated genes encoding potential Dectin-1 endogenous ligands was examined by qPCR (WT n = 3, and Clec7a^{-/-} n = 5). The data in (Figs. 12b and 12c) are expressed as mean ± SD. *p < 0.05.

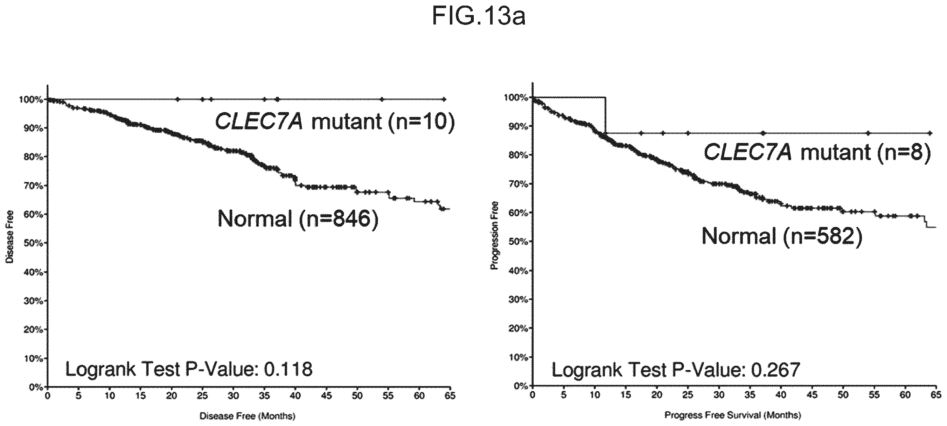

Fig. 13a: The expression of PGE₂ synthesizing enzymes is induced by Dectin-1 signaling of CRC patients. The disease free rate (left panel) and the progression-free survival rate (right panel) of the CRC patients with CLEC7A gene mutations and controls without the gene mutations were compared by analyzing shared data in the TCGA database.

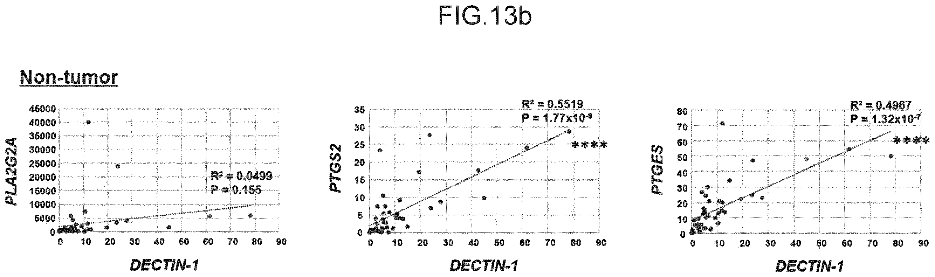

Fig. 13b: Non-tumor specimens from 44 CRC patients in total were collected, and a correlation of the expression between the PGE₂ synthase and Dectin-1 is shown.

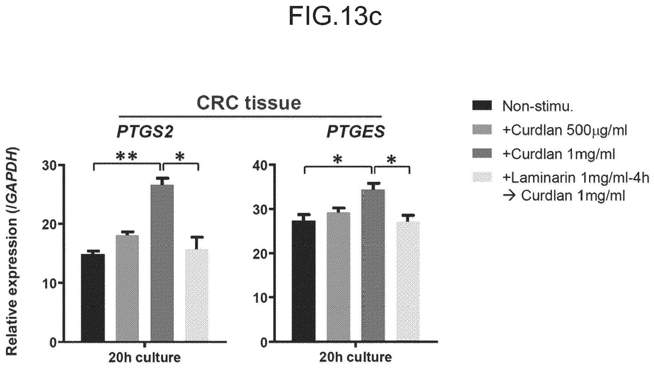

Fig. 13c: Fresh CRC specimens were collected after surgery, and small pieces of tumor tissues were stimulated with Curdlan for 20 hours. In the case of the laminarin treatment, the tissues were first treated with laminarin for 4 hours, and then Curdlan was added to the cultured tissues. The expression of the PGE₂ synthase was determined by qPCR. The data are expressed as mean ± SD of 3 wells. *p < 0.05, **p < 0.01, and ****p < 0.0001.

### DESCRIPTION OF EMBODIMENTS

Hereinafter, embodiments of the present disclosure will be described in detail. However, the present disclosure is not limited to the following embodiments. In the following disclosure, constituent elements (including element steps and the like) are not essential unless otherwise specified. The same also applies to numerical values and ranges thereof, and the present disclosure is not limited thereto.

In the present disclosure, the term "step" encompasses not only a step independent of other steps but also a step that cannot be clearly distinguished from other steps, as long as the purpose of the step is achieved.

In the present disclosure, a numerical range indicated using "to" includes numerical values described before and after "to" as a lower limit value and an upper limit value, respectively.

In the numerical ranges described in a stepwise manner in the present disclosure, an upper limit value or a lower limit value described in one numerical range may be replaced with an upper limit value or a lower limit value of another numerical range described in a stepwise manner. Further, in the numerical ranges described in the present text, an upper limit value or a lower limit value in a numerical range may be replaced with another value shown in the Examples.

In the present disclosure, in a case in which plural types of substances corresponding to a component are present in a composition, the content of the component in the composition refers to the total content of the plural substances present in the composition, unless otherwise specified.

### «Intestinal Tumor Suppressor, PGE₂ Production Inhibitor, and IL-22BP Production Promoter»

In one embodiment, the intestinal tumor suppressor according to the present disclosure contains a Dectin-1 inhibitor, and suppresses an intestinal tumor through Dectin-1 inhibition. In one embodiment, the intestinal tumor suppressor according to the present disclosure contains a β-glucan having a Dectin-1 inhibitory activity, and suppresses an intestinal tumor through the Dectin-1 inhibition. In one embodiment, the PGE₂ production inhibitor and the IL-22BP production promoter according to the present disclosure contain a β-glucan having a Dectin-1 inhibitory activity.

The inventors have found that Dectin-1 deficiency suppresses the development of colorectal tumors, and that this is mediated by the suppression of PGE₂ production and the promotion of IL-22BP production, independent of commensal microbiota. Based on these findings, it is believed that inhibition of Dectin-1, suppression of PGE₂ production, and/or promotion of IL-22BP production will be a useful strategy for suppressing intestinal tumors.

In the present disclosure, the term "Dectin-1 inhibitor" refers to a substance having an inhibitory activity against the functions of Dectin-1. The inhibitory activity against the functions of Dectin-1 may be, for example, an activity to inhibit the active site of Dectin-1, or an activity to inhibit a site other than the active site of Dectin-1 (that is, non-competitive inhibition). In one embodiment, the Dectin-1 inhibitor according to the present disclosure inhibits the functions of Dectin-1, suppresses PGE₂ production, and promotes IL-22BP production.

Whether or not a subject substance is a Dectin-1 inhibitor, that is, whether or not the functions of Dectin-1 are inhibited by the subject substance, is confirmed as follows. All cells of the spleen and regional lymph nodes are recovered from a C57BL/6 male mouse, and CD11b⁺ and CD11c⁺ cells are purified by MACS. Then, the cells are placed in a 96 well culture plate at 4 × 10⁵ cells/well, and the subject substance diluted in various concentrations is added and cultured for 3 hours. Thereafter, depleted-Zymosan (InvivoGen, Catalog #tlrl-zyd, USA) treated with polymyxin B for 1 hour is added to the culture wells at a concentration of 100 µg/ml, and the culture is further continued for 48 hours. The supernatant is recovered, and the amount of produced TNF is examined by ELISA. The amount of the TNF produced is compared between a case in which the subject substance is added and in a case in which an equal amount of solvent is added instead of the subject substance. In a case in which the TNF production is suppressed, it can be determined that the functions of Dectin-1 are inhibited.

Examples of the Dectin-1 inhibitor include a binding inhibitor that inhibits binding of a ligand to Dectin-1, a Dectin-1 activity inhibitor, and a signal transduction inhibitor that inhibits signal transduction from the Dectin-1.

In one embodiment, examples of the Dectin-1 inhibitor include a Dectin-1 antagonist. Whether or not a subject substance is a Dectin-1 antagonist can be confirmed by radiolabeling Curdlan, which is an agonistic ligand for Dectin-1, with [³H], and evaluating the inhibitory activity against the binding of the radioactive activity to Dectin-1. In a case in which the binding of the Curdlan to Dectin-1 is significantly inhibited as compared to the non-inhibited state (p = 0.05), it is determined that the β-glucan has a Dectin-1 inhibitory activity. TheDectin-1 inhibitory activity may be 10% or more, 20% or more, or 30% or more. Alternatively, the agonist activity can be evaluated by evaluating the degree of inhibition of the production of cytokines such as II,-1(3 or TNFα, the production of which is induced when Dectin-1 expressing cells are stimulated with a long-chain β-glucan such as Curdlan.

In one embodiment, the β-glucan having a Dectin-1 inhibitory activity can be useful as an intestinal tumor suppressor. In general, it is known that a β-glucans having a small molecular weight inhibit Dectin-1. Here, for example, even in a case of β-glucans having a large molecular weight, after being taken up by macrophages via Dectin-1, the β-glucan having a large molecular weight is decomposed into soluble β-glucan fragments having a smaller molecular weight. In other words, for example, when an insoluble β-glucan having a large molecular weight is orally ingested, a part of the β-glucan is decomposed into a soluble β-glucan having a small molecular weight. It is believed that soluble β-glucans suppress PGE₂ production by inhibiting intestinal Dectin-1 signaling, thereby promoting IL-22BP production, and consequently suppress tumor formation in the intestinal tract.

Note that the present disclosure is not limited by the foregoing presumed mechanisms.

β-glucan is a polysaccharide in which glucose is polymerized via β-1,3 linkages or the like. Raw materials for producing the β-glucan are not particularly limited as long as the raw materials contain a β-glucan. β-glucans are abundantly contained in cell walls of mushrooms, fungi, yeasts, seaweeds, and the like, and can be produced from these raw materials. The β-glucan can be produced by known methods. For example, the β-glucan can be extracted by immersing mushrooms, fungi, yeasts, seaweeds, or the like in hot water. Among the raw materials of the β-glucan used in the present disclosure, seaweeds are suitable as raw materials since seaweeds contain a large fraction of a β-glucan having a molecular weight of from 0.2 K to 100 K. The β-glucan contained in seaweeds is called laminarin. Further, the β-glucan extracted from mushrooms, fungi, yeasts, and the like has a large molecular weight, and therefore may be reduced in molecular weight by heat treatment, acid treatment, or the like. The β-glucan extracted from mushrooms, fungi, yeasts, and the like can be reduced in molecular weight according to the methods described in, for example, Ishimoto, Y, Ishibashi, K. I., Yamanaka, D., Adachi, Y, Kanzaki, K., Okita, K., Iwakura, Y, and Ohno, N. Modulation of an innate immune response by soluble yeast b-glucan prepared by a heat degradation method. Int. J. Biol. Macromol., 104, 367-376 (2017 Nov). doi: 10.1016/j.ijbiomac.2017.06.036. [Epub 2017 Jun 8], or Ishimoto, Y, Ishibashi, K. I., Yamanaka, D., Adachi, Y, Kanzaki, K., Iwakura, Y, and Ohno, N. Production of low-molecular weight soluble yeast b-glucans by an acid degradation method. Int. J. Biol. Macromol., 107, 2269-2278 (2018 Feb). doi: 10.1016/j.ijbiomac. 2017.10.094. [Epub 2017 Oct 16].

Laminarin (CAS registry number 9008-22-4) refers to a β-glucan derived from a seaweed such as *Eisenia Bicyclis,* and is also called laminaran. Laminarin is one of soluble β-glucans. Laminarin may be a β-glucan including β-1,3 linkages, or a β-glucan including a β-1,6-linked branched chain on the β-1,3-linked main chain. In other words, laminarin may have a β-1,6-linked side chain on a long-chain β-1,3-linked glycan. The structure of laminarin is represented by, for example, the following general formula (H and OH are present at the nonreducing end and the reducing end, respectively). In the following general formula, each of n1, n2, and m represents the number of each structural unit. The sum of n1 and n2 represents the length of the 1-3-linked main chain, and m represents a length of the 1-6-linked branched chain. Each of n1, n2, and m represents an integer of 1 or larger, and preferably an integer from 2 to 500. Note that, in the following general formula, n1 + n2 more preferably represents an integer from 2 to 25, from a viewpoint of a more preferable antagonist activity for Dectin-1. Further, m < (n1 + n2).

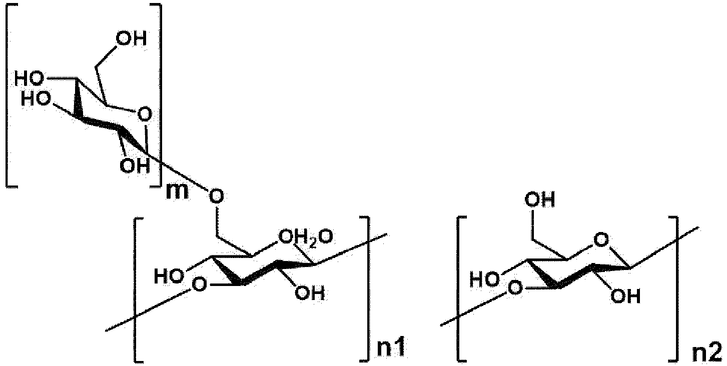

Among the seaweeds, laminarin is abundantly contained in cell walls of brown algae such as *Laminariales* and *Fucales,* and can be extracted from these brown algae. The extraction can be performed by various methods. For example, laminarin can be extracted by immersing the raw material in hot water, or by a method described in BULLETIN OF FISHERIES SCIENCES, HOKKAIDO UNIVERSITY, 56 (3): 75-86, December 2005 by Matsuda et al.

For details of the β-glucan, WO 2014/136982 A can also be referred to.

From the viewpoint of suppressing intestinal tumors, suppressing PGE₂ production, or promoting IL-22BP production, the β-glucan according to the present disclosure is preferably a soluble β-glucan, also preferably a β-glucan containing β-1,3 linkages or a β-glucan containing a β-1,6-linked branched chain on a β-1,3-linked main chain, and also preferably a β-glucan derived from a seaweed. The β-glucan according to the present disclosure is particularly preferably laminarin.

In the present disclosure, a soluble β-glucan is also referred to as a water-soluble β-glucan. In the present disclosure, the soluble β-glucan refers to a β-glucan capable of being dissolved in an amount of 20 g or more with respect to 1 L of water at 25°C.

The molecular weight of the β-glucan having a Dectin-1 inhibitory activity according to the present disclosure (hereinafter, also referred to as β-glucan according to the present disclosure) is preferably 100 K or less, and more preferably from 0.2 K to 100 K, from a viewpoint of an inhibitory action against the activation of Dectin-1. The molecular weight of the β-glucan according to the present disclosure may be from 0.2 K to 50 K, from 1 K to 10 K, 5 K or less, or from 0.2 K to 5 K. The molecular weight of the β-glucan according to the present disclosure is preferably 5 K or less from the viewpoint of a superior inhibitory activity against Dectin-1. Molecular weight distribution of the β-glucan according to the present disclosure may be a monomodal distribution, in which a β-glucan with one type of molecular weight is contained, or may be a bimodal distribution, in which β-glucans with two types of molecular weights are contained, a multimodal distribution such as a trimodal distribution or higher, or a smear molecular weight distribution.

In the present disclosure, "molecular weight" refers a weight-average molecular weight. The molecular weight of the β-glucan can be determined by a known method. The weight-average molecular weight can be measured by gel permeation chromatography (GPC). Note that the molecular weight of 1 K indicates that the molecular weight is 1000.

More specifically, analysis of the weight-average molecular weight of the β-glucan is performed using gel filtration chromatography (GPC) under the following conditions.
Column: G6000PWXI, and SB802
Column temperature: Pump: 40°C, Column: 60°C
Mobile phase: water
Flow rate: 0.5 ml/min
Detector: RI
Sample concentration: 0.1% (w/w)
Sample injection amount: 50 µl

As a method of obtaining the β-glucan, known methods can be adopted. For example, a β-glucan mixture containing β-glucans of various molecular weights is dissolved in distilled water (100 mg/ml), dialyzed against distilled water at 4°C for 2 days using a dialysis membrane having a freely-selected molecular weight cut-off value. After the dialysis, the dialyzed internal liquid is collected and lyophilized, whereby the β-glucan having a freely selected molecular weight can be obtained.

A β-glucan having a specific molecular weight can be obtained from a mixture containing β-glucans with various molecular weights by known methods such as ultrafiltration, dialysis or gel filtration. For example, in a case in which a β-glucan having a molecular weight of 100 K or less is prepared using a β-glucan having a molecular weight of more than 100 K as a raw material, the raw material can be subjected to chemical or enzymatical hydrolysis to be used. As the chemical hydrolysis, for example, an acid can be used. As the enzymatic hydrolysis, for example, an endo β-glucanase-type enzyme can be used. Alternatively, brown algae dry powder or the like containing a high proportion of an β-glucan having a specific molecular weight can also be used, as long as the effect according to the present disclosure can be obtained.

More specifically, examples of the method for obtaining a β-glucan with a lower molecular weight from a β-glucan with a high molecular weight include the following method. To a flask having a volume of 200 ml, 1,000 mg of a β-glucan with a high molecular weight (e.g., a β-glucan derived from mold) and 50 ml of 58% (w/w) concentrated sulfuric acid are added while being cooled, and mixed at 20°C for 3 hours while being stirred. Next, 350 ml of ice water is further added to the flask to dilute the mixture 8 times, and calcium hydroxide is further added to neutralize the mixture until the pH reaches 7.0. Then, the neutralized substance is filtered to remove calcium sulfate, and then the filtered substance is concentrated using an evaporator, centrifuged, and freeze-dried to obtain a β-glucan with a low molecular weight (e.g., a β-glucan having a molecular weight of 100 K or less).

In the present disclosure, the Dectin-1 inhibitory activity of the β-glucan refers to an activity capable of inhibiting activation of Dectin-1 by acting as a Dectin-1 antagonist. More specifically, the Dectin-1 inhibitory activity may be an activity of directly inhibiting (i.e., competitively inhibiting) the active site of Dectin-1, or may be an activity of inhibiting a site other than the active site of Dectin-1 (i.e., non-competitively inhibiting).

In the present disclosure, whether the β-glucan has a Dectin-1 inhibitory activity can be confirmed by radiolabeling Curdlan, which is an agonistic ligand for Dectin-1, with [³H], and evaluating the inhibitory activity against the binding of the radioactive activity to Dectin-1. In a case in which the binding of the Curdlan to Dectin-1 is significantly inhibited as compared to the non-inhibited state (p = 0.05), it is determined that the β-glucan has a Dectin-1 inhibitory activity. The Dectin-1 inhibitory activity may be 10% or more, 20% or more, or 30% or more. Alternatively, the Dectin-1 inhibitory activity can be evaluated by evaluating the degree of inhibition of the production of cytokines such as IL-1β or TNFα, the production of which is induced when Dectin-1 expressing cells are stimulated with a long-chain β-glucan such as Curdlan.

Whether the intestinal tumors are suppressed via Dectin-1 inhibition can be confirmed by measuring the expression level of: cytokines such as GM-CSF, IL-6, IL-1, and TNF; PGE₂; or IL-22 binding protein, which are controlled by Dectin-1 and are related to intestinal tumors, by ELISA.

The method for administering the Dectin-1 inhibitor, the β-glucan, or an agent containing a β-glucan to a subject is not particularly limited, and may be either oral administration or parenteral administration (e.g., subcutaneous administration, intraperitoneal administration, intramuscular administration, eye drops, ear drops, nasal administration, inhalation administration, transdermal administration, rectal administration, intrathecal administration, or intravenous administration), and oral administration or enteral administration is preferable. Examples of the dosage form suitable for the oral administration include tablets, capsules, powder, granules, solution, and elixir.

The subject to be administered with the Dectin-1 inhibitor, the β-glucan, or the agent containing a β-glucan is not particularly limited, and a mammal, such as a human, a monkey, a cow, a pig, a horse, a donkey, a sheep, a goat, a deer, a dog, a cat, a rabbit, a mouse, a rat, a guinea pig, a hamster, or a squirrel; and a bird, such as a chicken, a duck, a wild duck, a goose, a pheasant, a pigeon, a quail, a guinea fowl, a turkey, a parakeet, and a parrot, are preferable. A particularly preferred subject is a mammal.

The Dectin-1 inhibitor or the β-glucan used in the present disclosure is preferably administered to the subject by being mixed with a medicine, a food, or a feed. The intestinal tumor suppressor, the PGE₂ production inhibitor, and the IL-22BP production promoter according to the present disclosure may contain another component, such as a glycan other than the β-glucan, as long as the effect of the present disclosure can be obtained.

Examples of said another component include a vitamin, a component such as an amino acid, an excipient, a thickener, an isotonizing agent (e.g., solutes such as salt or glucose), or other additives for formulation. Examples of the additive for formulation include a liquid medium such as water, physiological saline, dextrose, or a similar sugar solution; a glycol such as ethylene glycol, propylene glycol, polyethylene glycol, or polypropylene glycol; an antioxidant such as sulfite; a pH adjuster and a buffer such as sodium citrate, sodium acetate, or sodium phosphate; a stabilizer such as sodium pyrosulfite, EDTA, thioglycolic acid, or thiolactic acid; an isotonizing agent such as sodium chloride and glucose; a local anesthetic such as procaine hydrochloride or lidocaine hydrochloride; and a surfactant such as dimethyl sulfoxide (DMSO). In addition to these, known additives can be included according to the dosage form.

The dose of the intestinal tumor suppressor, the PGE₂ production inhibitor, or the IL-22BP production promoter according to the present disclosure may be an amount effective for suppressing intestinal tumors, suppressing the PGE₂ production, or promoting the IL-22BP production. The dose is preferably increased or decreased according to the age, disease state, symptoms, family histories, and the like as appropriate. Further, the administration may be performed multiple times. In the case of the multiple administrations, it is preferable that the administration intervals are increased or decreased depending on the disease state, the symptoms, or the like as appropriate.

In the intestinal tumor suppressor according to the present disclosure, the intestinal tract may be either the large intestine (more specifically, a cecum, the colon, a rectum, etc.) or the small intestine (more specifically, a duodenum, a jejunum, an ileum, etc.), or may be the large intestine and the small intestine.

The intestinal tumor suppressor according to the present disclosure may be a therapeutic agent for reducing the number of intestinal tumors that is administered to a subject having intestinal tumors and that is used therapeutically to reduce the number of tumors already formed. The intestinal tumor suppressor according to the present disclosure may be a prophylactic agent for reducing the number of intestinal tumors that is administered to a subject without intestinal tumors and that is used prophylactically to reduce the number of tumors that can be formed in the future. Alternatively, the intestinal tumor suppressor according to the present disclosure may be a therapeutic agent for reducing an intestinal tumor that is administered to a subject having an intestinal tumor and that is therapeutically used to reduce the size of the already-formed tumor. The intestinal tumor suppressor according to the present disclosure may be a prophylactic agent for reducing the size of an intestinal tumor that is administered to a subject without an intestinal tumor and that is used prophylactically to reduce the size of a tumor that can be formed in the future.

By administering the intestinal tumor suppressor according to the present disclosure to a subject, the production of PGE₂ by the administered subject can be suppressed. In other words, the intestinal tumor suppressor according to the present disclosure may be used as a PGE₂ production inhibitor.

By administering the intestinal tumor suppressor according to the present disclosure to a subject, the production of IL-22BP by the administered subject can be promoted. In other words, the intestinal tumor suppressor according to the present disclosure may be used as an IL-22BP production promoter.

PGE₂ (prostaglandin E₂) is one of prostaglandins, which are physiologically active lipids. IL-22BP (IL-22 binding protein) is a potent regulator of tumor formation. It is believed that the intestinal tumor suppressor according to the present disclosure suppresses the PGE₂ synthesized (or produced) from the myeloid-derived suppressor cells (MDSCs) by inhibiting the Dectin-1 signal, thereby further inhibiting proliferation of the MDSCs, promoting the IL-22BP production, and finally suppressing the tumor formation in the intestinal tract.

In the present disclosure, as an indicator of the intestinal tumor suppression, visual inspection, endoscopic examination, histological examination (e.g., cytology and histopathological examination etc.), marker examination (e.g., a blood test, a urine test etc.), imaging diagnosis (e.g., X-ray inspection, CT scan, MRI scan, PET scan, and ultrasonic scan), or the like of the intestinal tract can be performed. Alternatively, as an indicator of the intestinal tumor suppression, the intestinal tumor suppression can be evaluated by a decrease in the protein expression level or the mRNA expression level of PGE₂, or in the mRNA expression level of an enzyme involved in the PGE₂ synthesis or a gene thereof (e.g., Pla2g2a or Pla2g2e (PhospholipaseA2 group IIA/IIE), Ptgs1 or Ptgs2 (Cox1/Cox2), Ptges2 or Ptges3 (PGE₂ synthase 2/3), etc.) in the intestinal tumors. Alternatively, as an indicator of the intestinal tumor suppression, the intestinal tumor suppression can be evaluated by an increase in the protein expression level of the IL-22BP or the mRNA expression level of the gene of the IL-22BP (Il-22bp) in the intestinal tumors.

In the present disclosure, the suppression of the PGE₂ production can be determined by a decrease in the protein expression level or the mRNA expression level of PGE₂ in the sample. The protein expression level of the PGE₂ can be measured by a known method such as ELISA. The mRNA expression level of the PGE₂ can be measured by a known method such as real-time PCR.

In the present disclosure, the promotion of the IL-22BP production can be determined by an increase in the protein expression level or the mRNA expression level of IL-22BP in the sample. The protein expression level of the IL-22BP can be measured by a known method such as ELISA. The mRNA expression level of the IL-22BP can be measured by a known method such as real-time PCR.

### «Other Embodiments»

The present disclosure also provides a method for preventing or treating an intestinal tumor, including administering an effective amount of a Dectin-1 inhibitor to a subject. Moreover, the present disclosure also provides a method for preventing or treating an intestinal tumor, including administering an effective amount of a β-glucan having a Dectin-1 inhibitory activity to a subject. The intestinal tumor may be an intestinal tumor with an increased expression of PGE₂ or an intestinal tumor with a decreased expression of IL-22BP.

Further, the present disclosure also provides a Dectin-1 inhibitor for preventing or treating an intestinal tumor. Moreover, the present disclosure also provides a β-glucan having a Dectin-1 inhibitory activity for preventing or treating an intestinal tumor. The intestinal tumor may be an intestinal tumor with an increased expression of PGE₂ or an intestinal tumor with a decreased expression of IL-22BP.

Further, the present disclosure also provides a use of a Dectin-1 inhibitor in the manufacture of a medicament for use in the prevention or treatment of an intestinal tumor. Moreover, the present disclosure also provides a use of a β-glucan having a Dectin-1 inhibitory activity in the manufacture of a medicament for use in the prevention or treatment of an intestinal tumor. The intestinal tumor may be an intestinal tumor with an increased expression of PGE₂ or an intestinal tumor with a decreased expression of IL-22BP.

In the foregoing embodiment, the "increased expression" and the "decreased expression" can be evaluated by measuring the amount of the target molecule in the tumor tissue and a non-tumor tissue by ELISA, real-time PCR, or the like. Further, evaluation can be made by measuring the amount of the target molecule in the tumor tissue and comparing the amount with a known standard amount.

Moreover, the present disclosure also provides a method for predicting severity of an intestinal tumor, including measuring at least one of the expression level of the Dectin-1 protein or the expression level of a CLEC7A gene in a tumor tissue. The measurement may be performed *in vivo* or *ex vivo.*

Although the mechanism of the method for predicting severity of an intestinal tumor is not clear, it is predicted as follows.

Intestinal tumors are facilitated by activation of Dectin-1 signaling. On the other hand, intestinal tumors are suppressed by inhibiting the Dectin-1 signaling. The expression level of the Dectin-1 protein or the expression level of the CLEC7A (C-Type Lectin Domain Containing 7A) gene encoding the Dectin-1 protein has a positive correlation with the severity of intestinal tumors. Accordingly, in measuring the expression level of the Dectin-1 protein or the expression level of the CLEC7A gene encoding the Dectin-1 protein, it is believed that the severity of intestinal tumors increases as the expression levels increase, and the severity of intestinal tumors decreases as the expression levels decrease.

Note that the present disclosure is not limited by the foregoing presumed mechanism.

The method of measuring the expression level of the Dectin-1 protein and the expression level of the CLEC7A gene in a tumor tissue is not particularly limited, and protein quantification methods (e.g., ELISA) or gene (e.g., mRNA) quantification methods (e.g., real-time PCR) that are generally performed may be used.

For the details of the features such as the Dectin-1 inhibitor, the β-glucan having a Dectin-1 inhibitory activity, the subject, the intestinal tumor, PGE₂, IL-22BP in the foregoing embodiments, the details previously described apply.

### Examples

Hereinafter, the present disclosure will be described in more detail with reference to the Examples. However, the present disclosure is not limited to the following Examples as long as it does not exceed the spirit of the present disclosure. Note that "%" is based on mass unless otherwise specified.

### 1. Dectin-1-deficiency suppresses colorectal tumor development in a commensal microbiota-independent manner

To investigate the potential role of Dectin-1 in intestinal tumorigenesis, we firstly examined the influence of Dectin-1 signaling on colorectal tumorigenesis in DSS-treated Apc^{Min} (Apc^{Min}-DSS) mice, which has a mutation in the β-catenin signaling pathway (Reference 18). Body weight loss in Apc^{Min/+}Dectin-1-deficient (Clec7a^{-/-}) mice after DSS-treatment was significantly milder than that in Apc^{Min/+} mice (Fig. 8a). At day 28 after DSS-treatment, shortening of colon length, one of the symptoms of colitis, was milder in Apc^{min/+}Clec7a^{-/-} mice (Figs. 1a and 8b), as in DSS only treated Clec7a^{-/-} mice (Reference 11), and the number of polyps in the colon was significantly reduced in Apc^{Min/+}Clec7a^{-/-} mice (Figs. 1a and 1b).

Further, the effect of Dectin-1-deficiency on the development of tumors in Apc^{Min} mice without DSS treatment was examined. After sacrificing mice at 20 to 23 weeks of age, it was found that the development of polyps in both colon and small intestine was significantly suppressed in Apc^{Min/+}Clec7a^{-/-} mice compared with that in Apc^{Min/+} mice (Figs. 1c, 1d, 8c, and 8d). The number of polyps of 3 mm or smaller was significantly decreased in the small intestines of Apc^{Min/+}Clec7a^{-/-} mice (Fig. 8d).

Next, using another chemical-induced colorectal tumor model, wild-type (WT) and Clec7a^{-/-} mice were administered with azoxymethane (AOM) and DSS for 3 cycles (AOM-3DSS). Clec7a^{-/-} mice exhibited milder body weight loss than WT mice during 3 cycles of DSS-treatment (Fig. 8e). Shortening of the colon length was significantly milder in Clec7a^{-/-}mice (Fig. 1e, 8f). The number of polys induced by AOM-3DSS in Clec7a^{-/-} mice was significantly less than those in WT mice (Fig. 1f). When mice were treated with AOM and only one cycle of DSS, anal prolapse (Figs. 8g and 8h) and shortening of colon length (Figs. 8i and 8j) were also significantly milder in Clec7a^{-/-} mice. Although all WT mice developed colorectal polyps, only half of the Clec7a^{-/-} mice developed polyps with smaller sizes (Figs. 8h and 8k). These results indicate that blockade of Dectin-1 signaling inhibits intestinal tumorigenesis.

We previously reported that colonization of a regulatory T cell (Treg)-inducing commensal bacterium *Lactobacillus murinus* is increased in the intestine of Clec7a^{-/-} mice to suppress intestinal inflammation (Reference 11). In fact, real-time RT-PCR showed that Foxp3 (Treg marker) expression in polyps and non-polyp tissue was significantly increased in Dectin-1-deficient mice (Fig. 9a). To examine the effects of intestinal microbiota in the regulation of tumorigenesis in Clec7a^{-/-} mice, Clec7a^{-/-} mice were continuously co-housed with WT mice after weaning, and these co-housed mice were administered with AOM-3DSS to induce colorectal tumors. However, the number of colorectal polyps in the Clec7a^{-/-} mice was significantly smaller than that in the WT mice, and in particular, large polyps with a diameter of 3 mm or larger were observed in the WT mice (Figs. 1g and 1h).

To further analyze the effects of commensal microbiota in the regulation of tumorigenesis by Dectin-1, germ-free (GF) WT and Clec7a^{-/-} mice were administered with AOM-3DSS. Thirty-six weeks after the AOM administration, the mice were sacrificed, and it was found that colorectal polyps were developed only in WT mice and not in Clec7a^{-/-} mice (Figs. 1i-1k), although the colon length was similar between these mice. In these GF mice, intestinal Foxp3 expression was similar between WT and Clec7a^{-/-} mice (Fig. 9b). These results indicate that the inhibition of tumor development in Clec7a^{-/-} mice is not dependent on commensal microbiota.

### 2. Infiltration of myeloid-derived suppressor cells (MDSCs) is decreased in polyps in Clec7a-/- mice

Next, Clec7a-/- mice in Apc^{Min/+} background were administered with 1% DSS to analyze the modification of tumor infiltrating cell populations in the colon. Flow cytometric analysis showed that T cells (including both αβ& γδ T cells), B cells (including IgG⁺ B cells), and DCs (MHC-II⁺ CD11c⁺ cells) were significantly increased (Figs. 2a and 9c), and MHC-II CD11c⁻CD11b⁺ myeloid-derived suppressor cells (MDSCs) were greatly decreased in polyps of Apc^{Min/+}Clec7a^{-/-} mice compared with Apc^{Min/+} mice (Fig. 3b).

Dectin-1 is widely known to be expressed on DCs, but in mouse colonic polyps, flow cytometry analysis showed that the expression of Dectin-1 was mainly on CD103⁻CD11c⁻CD11b⁺ cells (Fig. 2e, 92.4%). By analyzing Dectin-1-expressing populations, it was found that nearly 75% of Dectin-1⁺ were MHC-II⁻, in which nearly 50% were Ly6C^{int}Ly6G⁺CD11b⁺, 5% were Ly6C^{hi}CD11b⁺, and 19% were CD11c⁻MHC-II⁻Gr1⁻CD11b⁺ cells (Fig. 9d). These tumor-infiltrating myeloid cells are commonly known as granulocytic polymorphonuclear and monocytic MDSCs (Reference 22). IL-6 and IL-1β are reported to promote MDSC expansion (References 23 and 24), and when intestinal polyp-infiltrating CD11b⁺ and CD11c⁺ cells were stimulated with a β-glucan OXCA from *Candida albicans,* Il6 and Il1b expression was induced, and this induction was inhibited by a Dectin-1 antagonist laminarin (Fig. 2d), indicating that MDSC-promoters can be directly induced by Dectin-1 signaling.

### 3. Several tumor-regulating pathways are dependent on Dectin-1 signaling

To clarify the mechanism by which Dectin-1 signaling regulates tumorigenesis, gene expression in tumor and non-tumor tissues of GF mice after AOM-3DSS administration was examined by RNA-seq analysis. Since the whole gene background was lower under GF condition than SPF condition, Dectin-1-controled genes were easily found. The results showed that T cell- (e.g., Cd3e, Cd28, Cd69, Ccr6, Ccr9), Th1- (e.g., Cd4, Tbx21, Ifng, Il12a, Cxcr3), B cell- (e.g., Cd19, Ig-associated, Cxcr5), and DC- (e.g., Itgax, H2-Ab1, Ccr7) associated genes were greatly upregulated, and Th17-associated genes (e.g., Il17a, Il23a) were downregulated in the colorectal polyps of Clec7a^{-/-} mice (Figs. 3a, 10a, and 10b). Expression of tumor-regulating cytokines such as IL-22 (Il22) or IL-18 (Il18) was not changed but the expression of an IL-22 antagonistic receptor, IL-22 binding protein (Il-22bp or Il22ra2), was greatly upregulated in polyp tissues of Clec7a^{-/-} mice (Figs. 3a and 10a). By KEGG gene enrichment analysis, it was also found that immune-related genes such as antibody production, lymphocyte activation, NK cell-mediated cytotoxicity, and antigen presentation were upregulated in tumor tissues of Clec7a^{-/-} mice compared to that of WT mice (Fig. 2b). In contrast, pathways such as Wnt signaling, IL-17 signaling, Ras signaling or Arachidonic acid metabolism were downregulated in Clec7a^{-/-} mouse colon (Fig. 3b). The expression of MDSC-associated genes (Reference 25) was suppressed in Clec7a^{-/-} mouse polyps, consistent with the impairment of MDSC infiltration in colonic tumor in Clec7a^{-/-} mice (Fig. 2b). The expression of the Csf2, Il6, Il1b and Tnf, which promote MDSC differentiation (References 23 and 24), was greatly impaired in Clec7a^{-/-} mouse polyps (Figs. 3a, 10a, and 10b). Interestingly, it was also found that expression of genes encoding prostaglandin E2 (PGE₂) synthases, such as Pla2g2a (phospholipase A2), Ptgs1 (Cox1), Ptgs2 (Cox2), was downregulated in the polyps in Clec7a^{-/-} mice (Figs. 3d and 10a). The expression of Dectin-1 gene Clec7a was upregulated in colorectal polyps than that in non-polyp tissues (Fig. 3e), reflecting the expansion/infiltration of the major Dectin-1-expressing cells, MDSCs, in colonic tumors (Fig. 2b).

Both T cells and antibody-producing B cells are involved in anti-tumor immunity (References 19 to 21). However, when Rag2^{-/-} mice were administered with AOM-3DSS, it was found that the mice with no lymphocytes developed even less polyps compared with WT mice (Figs. 10c and 10d), indicating that these lymphocytes are not involved in the suppression of mouse colorectal cancer.

### 4. Dectin-1-deficiency suppresses colorectal tumor development by enhancing IL-22 binding protein production

IL-22bp expression was significantly upregulated in polyps of GF Clec7a^{-/-} AOM-3DSS mice (Figs. 3a and 10a). In the intestine of Apc^{Min/+}Clec7a^{-/-}-DSS mice, the expression of IL-22bp mRNA was also enhanced not only in polyps but also in non-polyp tissues (Figs. 4a and 11a). The mRNA expression of Il18 was normal in both GF Clec7a^{-/-} and Apc^{Min/+}Clec7a^{-/-} mice (Figs. 4a and 11a), although IL-18 is reported as a direct suppressor of IL-22bp (Reference 26).

IL-22bp^{-/-} mice was generated on the Apc^{Min/+}Clec7a^{-/-} background. The result showed that the prolonged life span of Apc^{Min/+}Clec7a^{-/-} mice compared with Apc^{Min/+} mice was totally canceled by IL-22bp-deficiency, and half Π-22bp-deficiency still caused shorter life of Clec7a^{-/-} mice (Fig. 4b). Although the number of polyps were reduced in Apc^{Min/+}Clec7a^{-/-}compared with Apc^{Min/+} mice (Figs. 8c and 8d), even more polyps were observed in Apc^{Min/+}Clec7a^{-/-}Il-22bp^{-/-} mice of younger age (Fig. 4c). These results indicate that the upregulation of IL-22bp is critical in the suppression of intestinal tumor development in Clec7a^{-/-} mice.

To identify IL-22bp-expressing cells in the intestine, EpCAM⁺ epithelial cells and CD45⁺ leucocytes were separated from the intestinal polyps. qPCR confirmed that IL-22bp mRNA was expressed only in the CD45⁺ population (Fig. 4d). Flow cytometry revealed that the IL-22bp protein was expressed in both CD11b⁻CD103⁺ and CD11b⁺CD103^{int}DCs, but not in CD11c⁻CD11b⁺MDSCs (Fig. 4e). Il-22bp was exclusively expressed in CD11b⁺ and CD11c⁺ myeloid cells, but not in Thy1.2⁺ T cells, CD19⁺ B cells or EpCAM+ epithelial cells in both colonic polyp and non-polyp tissues (Fig. 4f), and its expression was mainly detected in DCs but not in CD11c⁻ MDSCs (Fig. 4g). Il-22bp expression was not affected when the colonic CD11b⁺ and CD11c⁺ cells were treated with Dectin-1 ligands (Fig. 4h), suggesting that Dectin-1 is not directly involved in the regulation of Il22pb expression.

Retinoic acid (RA) is reported to modulate IL-22bp production (References 27 and 28) and the expression of enzymes catalyzing RA synthesis such as Adh1 and Aldh1a1 was enhanced in polyps in Clec7a^{-/-} mice (Figs. 11b and 11c). However, β-glucan did not influence the expression of these enzymes (Fig. 11d), suggesting the RA synthesis is not directly regulated by Dectin-1 signaling.

### 5. Dectin-1 signaling directly enhances PGE₂ production

The RNA-seq data obtained from AOM-3DSS-treated GF mice showed that the expression of genes involved in PGE2 synthesis, such as Pla2g2a/2e (for Phospholipase A2 group IIA/IIE), Ptgs1/2 (for Cox1/Cox2), and Ptges2/3 (for PGE2 synthase 2/3), was greatly upregulated in polyps of WT mice, but not in polyps in Clec7a^{-/-} mice (Figs. 3d and 10a). Decreased expression of Ptgs2 in colonic polyps and non-polyp tissues was also observed in Clec7a^{-/-} mice under SPF condition (Fig. 5a), and PGE₂ concentration in non-polyp tissue homogenate was significantly decreased in Clec7a^{-/-} mice (Fig. 5b). Enzymes involved in PGE₂-synthesis were preferentially expressed in CD11c⁻CD11b⁺ MDSCs, although CD11c⁺ DCs also expressed these genes with low levels (Fig. 5c).

Decreased Ptgs2 expression in intestinal polyps was also observed in Apc^{Min/+}Clec7a^{-/-}mice (Fig. 5d), and this decrease was more obvious in CD45⁺ intestinal leukocytes (Fig. 5e). When polyp-infiltrating leukocytes from Apc^{Min/+} mice were treated with Curdlan, a Dectin-1 agonistic β-glucan (that is, β-1,3-glucan, Curdlan from soil bacteria *Alcaligenes faecalis var. myxogenes,* weight-average molecular weight of more than 100K (FUJIFILM Wako Pure Chemical Corporation, Japan, Osaka)), the expression of genes encoding PGE synthase 3 (Ptges3), Cox1 (Ptgs1), and Cox2 (Ptgs2), were significantly induced and the induction was inhibited by Dectin-1 antagonistic laminarin (Fig. 5f). Stimulation and blockade of Dectin-1 signaling on purified polyp-infiltrating CD11b⁺ cells showed the similar modification of PGE₂ synthase expression (Fig. 5g), indicating that Dectin-1 signaling directly induces PGE₂ synthesis in intestinal tumor-infiltrating MDSCs.

### 6. Administration of Dectin-1 antagonist suppresses development of intestinal polyps by reducing PGE₂ in the intestine

To examine therapeutic effects of Dectin-1-blockade on intestinal tumorigenesis, WT mice were administered with AOM-3DS S, and were fed a diet supplemented with 5% of Dectin-1 antagonist laminarin (laminarin derived from *Eisenia* (Tokyo Chemical Industry Co., Ltd., Japan, Tokyo), weight-average molecular weight of 50 K or less), which is a soluble β-glucan, during 3 cycles of DSS-treatment. Consistent with the observations in Clec7a^{-/-} mice, it was revealed that mice fed with laminarin developed significantly lower burden of colorectal polyps than control mice (Figs. 6a and 6b). Blocking Dectin-1 signaling suppressed mouse intestinal PGE₂ production (Fig. 6c), accompanied with the reduction of CD11b (Itgam) expression (Fig. 6d). On the other hand, when DSS-treated Apc^{Min/+}Clec7a^{-/-} mice were intermittently administrated with PGE₂ for 4 weeks, polyp number was increased to the similar level of that in Apc^{Min/+} mice (Figs. 6e and 6f). Infiltration of CD11c⁺ DCs and T cells was suppressed, whereas infiltration of CD11b⁺ MDSCs was promoted in polyps after the PGE₂ administration (Figs. 6g and 12a). The expression of MDSC marker CD11b (Itgam) and Arginase-1 (Arg1), and of oncogenes Cyclin D1 (Ccnd1) and c-Myc (Myc), was enhanced, while Il-22bp expression was suppressed by PGE₂-administration (Fig. 6h), suggesting that MDSC infiltration, tumor growth, and IL-22bp production in colonic tumor are regulated by PGE₂. To confirm the regulatory function of PGE₂, intestinal polyp-infiltrating cells were stimulated with PGE₂, by which it was found that the expression of MDSC markers such as Arg1, CD11b and Nos2 were directly induced and Il-22bp was directly inhibited by the PGE₂ administration (Fig. 6i). Interestingly, it was also found that Cox2 mRNA level was negatively correlated with the Il-22bp expression in colonic polyps under both GF and SPF conditions (Fig. 6j). These results indicate that by blocking Dectin-1 signaling, PGE₂ synthesized from MDSCs is suppressed, which may further impair MDSC expansion and promote IL-22bp to finally suppress intestinal tumorigenesis.

### 7. Dectin-1 signaling enhances PGE₂ synthesis in colorectal cancer patients

To evaluate the role of Dectin-1 signaling on CRC development in humans, CRC specimens were collected first and were examined for CLEC7A expression. It was found that post-surgery survival rate of patients with high levels of CLEC7A expression (relative expression of 2 or higher, normalized with GAPDH) was significantly lower than those with low levels of CLEC7A expression (less than 2) (Fig. 7a). Next, it was found that CLEC7A expression levels were positively correlated with pathological tumor malignancy (Figs. 7b and 7c). Disease and progression rates were relatively decreased in CRC patients with CLEC7A mutation(s), although the tendency is not significant due to the small number of CRC patients with mutant CLEC7A in TCGA (The Cancer Genome Atlas) database (Fig. 13a). These observations suggest that Dectin -1 signaling in human also facilitates CRC development.

To elucidate whether the potential Dectin-1-PGE2-IL-22bp axis, discovered in mouse, also exists in human, the mRNA expression of IL-22BP and COX2 in tumors from CRC patients were examined first. IL-22BP was relatively decreased, and PTGS2 was significantly increased in tumor parts compared with non-tumor parts (Fig. 7d), and this tendency was more significant in comparison of samples from the same individuals (23 pairs of tumor and non-tumor tissues) (Fig. 7e). Further, strong positive correlation between intestinal expression levels of CLEC7A and PLA2G2A, PTGS2 or PTGES in CRC patients (Figs. 7f and 13b), and when CRC tissues or cancer-infiltrating leukocytes were treated with Curdlan, the induction of PGE2 synthase gene expression was ditected (Figs. 7g and 13c). Furthermore, Curdlan also upregulated expression of CD33 in both tumor or normal tissues (Fig. 7h), the common marker of human MDSCs (Reference 29), suggesting that DECTIN-1 signaling also promotes MDSCs in CRCs. Expression of CD33 and CD11B (ITGAM) in CRCs was directly induced by PGE₂ *in vitro,* and human IL-22BP was also suppressed by PGE₂ in a dose-dependent manner (Fig. 7i). These results suggest that Dectin-1-PGE2-IL-22BP axis may also exist and be involved in the regulation of human colorectal carcinogenesis.

### «Discussion»

It is now known that β-glucans have no chemical cytotoxic effects. Studies implicating the cytotoxic effects of β-glucans were mostly from studies using crude extracts of β-glucan containing herbs such as *Ganoderma lucidum,* in which other active components such as ganoderic acid from its mycelium (Reference 30) and triterpenes from its spore (Reference 31) are actually known to have direct anti-cancer effects. Previous studies showed that oral administration of (3-glucan enhances anti-tumor effects of antitumor agents (Reference 32), and that oral administration of yeast-derived β-glucan particles suppresses the growth of subcutaneously inoculated Lewis lung carcinoma (Reference 33). However, by labeling glucans with fluorescein, Cheung et al. tracked their processing *in vivo* after oral uptake, and found the large β-glucan molecules were taken up by macrophages via Dectin-1 and were then degraded into smaller soluble β-1,3-glucan fragments (Reference 34). Since the typical soluble type of β-glucan is laminarin, a Dectin-1 antagonistic ligand, it is believed that after oral uptake, some insoluble β-glucans with large molecules are degraded into laminarin to inhibit intestinal Dectin-1 signaling so as to exhibit anti-intestinal tumorigenesis consequently.

In the present study, it was shown that blocking Dectin-1 effectively suppressed development of colorectal tumors in mice of both AOM-DSS-induced CRC model and Apc^{Min} familial adenomatous polyposis coli model. Levels of PGE₂, which promotes MDSC expansion and tumor development, was reduced in Clec7a^{-/-} mice due to decreased expression of PGE₂ synthesizing enzymes including Cox2, Cox1 and Ptges3. In addition, expression of IL-22bp, inhibition of the activity of which can suppress the development of colorectal tumors, was significantly increased in Clec7a^{-/-} mice. Furthermore, it was found that CRC patients with high Dectin-1 expression had a shorter survival time as compared to CRC patients with low expression. A positive correlation was found between Dectin-1 expression levels and colorectal cancer stages. In CRC patients, IL-22BP expression was decreased and PTGS2 expression was increased in tumors compared with normal tissues. These results suggest that Dectin-1 plays important roles in the development of colorectal tumors in mice and humans, through modification of PGE₂ levels and IL-22bp expression.

PGE₂, a well-known principal mediator of inflammatory diseases, plays a critical role in the development of AOM-DSS-induced colon tumors by binding to the receptor EP2 to enhance the proliferation of colon cancer cells and expression of inflammation- and growth-related genes such as Tnf, Il6, Cxcl1, and Cox2, by activating the β-catenin axis (References 36, 11, and 37). In fact, intestinal polyposis in Apc-deficient mice is suppressed in Ptgs2 null mutant mice, and COX-2 inhibitors, represented by aspirin and other NSAIDs, can reduce the occurrence or progression of colorectal cancer and polyps (References 38 and 39). In this study, it was found that Dectin-1 deficiency impairs expression of a series of enzymes involved in PGE₂ synthesis, especially of phospholipase A2 and Cox2, in both AOM and Apc^{Min} induced intestinal tumors. It was also shown that Dectin-1 signaling directly induces expression of these PGE₂-synthases in tumor-infiltrating MDSCs.

Actually, intestinal levels of PGE₂ were decreased in Clec7a^{-/-} mice, and exogenous PGE₂ administration recovered colonic polyposis. These results indicate that Dectin-1 signaling promotes intestinal tumorigenesis by enhancing PGE₂ synthesis. Previous reports suggested involvement of Dectin-1 in PGE₂ production in house dust-induced allergic airway inflammation (Reference 40) and in human neutrophils cuased by a fungus *Paracoccidioides brasiliensis* (Reference 41).

IL-22BP is a soluble inhibitory receptor for IL-22 (References 42 and 43). IL-22BP plays an important role in the regulation of tumorigenesis and deficiency of this cytokine promotes colorectal tumor development (Reference 26). In the present study, it was shown that IL-22bp disruption completely abrogated the Dectin-1 deficiency-caused impairment of polyp formation, suggesting the major contribution of IL-22bp in the suppression of tumorigenicity in Clec7a^{-/-} mice. IL-22bp was exclusively produced by intestinal DCs but not by MDSCs, and it was also found that Il-22bp expression in tumor infiltrating cells was directly suppressed by PGE₂, synthases of which are mainly secreted by MDSCs.

Since Dectin-1 expression in colonic tumors was also mainly detected on MDSCs, it is believed that the impaired production of intestinal PGE₂ caused by Dectin-1 deficiency results in the upregulation of Il-22bp expression on DCs in Clec7a^{-/-} mice to suppress the tumorigenesis consequently. In support for this notion, it was shown that administrating Clec7a^{-/-} mice with PGE₂ suppressed colonic Il-22bp expression and abrogated the impaired tumor development arising from Dectin-1-deficiency.

MDSC accumulation in tumors is considered to enhance cancer progression by promoting invasion, angiogenesis and metastasis and inhibiting anti-tumor immunity (References 44 and 45). In the present study, it was found that the cytokines, such as GM-CSF, IL-6, IL-1 and TNF known to promote MDSC differentiation or increase suppressive activity (References 23 and 24), were the downstream of Dectin-1 signaling in mouse intestinal tumors, which explained why MDSC population and MDSC-associated gene expression were decreased in colonic polyps of Clec7a^{-/-} mice. Furthermore, it was shown that MDSCs are the major producers of PGE₂ synthases, and that PGE₂ enhances MDSC differentiation by inducing Itgam and Arg1 in mouse and ITGAM and CD33 in human intestinal tumor-infiltrating cells. These results raise the possibility that by an auto-amplification loop for inflammatory cytokine and PGE₂ production, Dectin-1 enhances MDSC expansion and differentiation to promote intestinal tumorigenesis. Since MDSCs suppresses anti-tumor responses, decreased MDSC population should facilitate the enhancement of lymphocyte-mediated anti-tumor immunity in Clec7a^{-/-} mice.

It has been suggested that intestinal environment such as commensal microbiota and inflammatory milieu affects development of colorectal tumors. In the present study, when the GF mice were treated with AOM-3DSS, it took 36 weeks to induce the obvious colorectal tumors compared with only 16 weeks under SPF condition. Chronic intestinal inflammation, such as inflammatory bowel disease (IBD), is considered as a possible risk factor for intestinal tumorigenesis (Reference 46), although some clinical research found only weak correlation between IBD and gastrointestinal malignancy (Reference 47). We previously showed that Dectin-1 signaling suppresses the growth of Treg-inducing bacteria such as *Lactobacillus* and *Clostridium* species by directly inducing anti-microbial calprotectin on neutrophils or by inducing anti-microbial peptides through IL-17F (References 11 to 13). Thus, blocking Dectin-1 signaling increases colonic Treg cells to suppress mouse colitis (References 11 to 13). In the present study, however, Clec7a^{-/-} mice co-housed with WT mice or Clec7a^{-/-} mice raised under GF conditions still developed less numbers of polyps than WT mice, indicating that commensal bacteria are not involved in the reduced polyp formation in Clec7a^{-/-} mice. Since Tregs were not increased in GF Clec7a^{-/-} mice and Rag2^{-/-} mice with no lymphocytes still exhibited enough capability against intestinal tumorigenesis, it is believed that not Tregs, but decreased PGE₂ and increased IL-22BP suppress polyp formation in Clec7a^{-/-} mouse intestines.

We showed that oral administration of a Dectin-1 antagonistic ligand laminarin to mice suppresses AOM-DSS-induced colorectal polyposis. Regarding the source of Dectin-1 ligands, mouse chow usually contains 10% or more of yeast extract, which contains abundant glucans. Our daily diets also contain lots of β-glucan-containing foods, such as mushrooms, yeasts and seaweeds. A recent report identified an endogenous ligand, galectin-9, in pancreatic cancer tissues (Reference 17). In the present study, the expression levels of galectin-9 was analyzed, and it was found that its expression was upregulated in polyps compared with non-polyp tissues in Clec7a^{-/-} but the expression was not upregulated in WT mice (Fig 12b, 12c). Overall, the present study revealed a critical role of Dectin-1 in facilitating intestinal tumorigenesis by promoting MDSC-PGE₂-mediated IL-22bp suppression, which suggests Dectin-1 as a promising target for the prevention and treatment of CRCs.

### «Materials and Methods»

### 1. Mice

Clec7a^{-/-} mice were used after backcrossing for 9 generations to C57BL/6J. Wild-type (WT) C57BL/6J mice purchased from Sankyo Lab Service were used as the control. Apc^{Min/+} mice kindly provided by Dr. Ryo Abe, Tokyo University of Science, were crossed with Clec7a^{-/-} mice to generate Apc^{Min/+}Clec7a^{-/-} mice. Apc^{Min/+}Clec7a^{-/-}Il-22bp^{-/-} mice were generated by the CRISPR-Cpf1 method by using Apc^{Min/+}Clec7a^{-/-} zygotes. Age- and sex-matched WT mice were separated or co-housed with Clec7a^{-/-} mice after weaning at 4 weeks old. All mice were kept under specific pathogen-free conditions with g-ray sterilized normal diet, acidified (0.002 N HCl, pH 2.5) tap water, and autoclaved wooden chip bed in environmentally controlled clean rooms at the experimental animal facility in Research Institute for Biomedical Sciences, Tokyo University of Science, and in Zhongshan School of Medicine, Sun Yat-sen Univerisity. All animal experiments were carried out following the institutional ethical regulations and guidelines and with protocols approved by the Institutional Animal Care and Use Committee of the Tokyo University of Science, and by the Experimental Animal Manage and Use Committee of Sun Yat-sen University in China.

### 2. Human CRC Sample Collection

Before the collection of human samples from CRC patients, written informed consent was obtained from each patient before the colorectal tumorectomy, and all experiments were carried out following the institutional ethical regulations and guidelines under the protocols approved by Committee for Clinical Investigation of the First Affiliated Hospital, Sun Yat-sen University in China. For the tissue-infiltrating cell purification or tissue or cell *in vitro* culture, after each surgery, tumor specimens or/and normal tissue around tumor were cut and were transferred into ice-cold PBS and placed on ice until use. For the mRNA level and relative survival rate analysis, 47 tumor specimens and 43 non-tumor specimens were collected from 70 CRC patients, which contained 23 pairs of tumor and non-tumor from the same individuals. Details of patient clinical information including gender, age, and disease severity are shown in Tables 1 to 3.

**[Table 1]**

| **disease** | **sample #** | **age** | **sex** | **operation time** | **death time** | **last follow-up visit** | **T** | **N** | **M** | **TNM stage** | **pathological grading** | **tumor size (cm)** |
|---|---|---|---|---|---|---|---|---|---|---|---|---|
| CRC | 001359 | 82 | female | 2010/01/25 | / | 2018/07/26 | T2 | N0 | M0 | I | moderately differentiated | 5.5 |
| CRC | 001361 | 58 | female | 2010/01/27 | / | 2018/06/28 | T4 | N0 | M0 | IIB | moderately^{~}poorly | 2.5 |
| CRC | 001364 | 65 | female | 2010/02/02 | / | 2015/06/23 | T4 | N0 | M0 | IIB | moderately^{~}poorly | 7 |
| CRC | 001365 | 50 | female | 2010/02/03 | 2014/08/15 | 2015/05/29 | T4a | N1 | M0 | IIIB | poorly differentiated | 3.5 |
| CRC | 001367 | 75 | male | 2010/02/05 | / | 2020/02/28 | T4 | N0 | M0 | IIB | moderately^{~}poorly | 4 |
| CRC | 001368 | 70 | male | 2010/02/05 | / | 2017/08/16 | T4 | N1 | M0 | IIIB | moderately differentiated | 4 |
| CRC | 001369 | 68 | male | 2010/02/05 | / | 2020/05/27 | T3 | N1 | M0 | IIIB | moderately differentiated | 5 |
| CRC | 001371 | 61 | female | 2010/02/10 | 2011/02/23 | 2011/03/23 | T3 | N1 | M1 | IV | moderately differentiated | 5 |
| CRC | 001372 | 75 | male | 2010/02/11 | / | 2020/05/27 | T4 | N0 | M0 | IIB | moderately differentiated | 7 |
| CRC | 001374 | 55 | male | 2010/02/21 | / | 2020/05/27 | T4 | NO | M0 | IIB | moderately differentiated | 4 |
| CRC | 001375 | 63 | male | 2010/02/21 | / | 2019/08/05 | T3 | N1a | M0 | IIIB | poorly differentiated | 6 |
| CRC | 001377 | 45 | male | 2010/02/25 | / | 2019/11/20 | T4 | N1 | M1 | IV | moderately differentiated | 2.5 |
| CRC | 001379 | 72 | male | 2010/02/24 | 2010/12/31 | 2011/04/02 | T4 | N1 | M1 | IV | moderately differentiated | 6.5 |
| CRC | 001380 | 57 | male | 2010/02/25 | 2011/04/15 | 2011/10/08 | T4 | N2 | M1 | IV | moderately differentiated | 3 |
| CRC | 001381 | 74 | female | 2010/02/25 | 2010/03/30 | 2010/03/26 | T4 | N2 | M1 | IV | poorly differentiated | 8 |
| CRC | 001382 | 75 | male | 2010/02/26 | / | 2020/05/28 | T4 | N0 | MO | IIB | moderately differentiated | 3.5 |
| CRC | 001383 | 78 | female | 2010/03/01 | / | 2020/08/24 | T3 | N0 | M0 | II(A) | moderately differentiated | 4 |
| CRC | 001384 | 56 | male | 2010/03/09 | / | 2020/05/27 | T3 | N1 | M0 | IIIB | moderately differentiated | 2.5 |
| CRC | 001385 | 37 | female | 2010/03/09 | 2012/12/15 | 2013/06/19 | T4a | N1b | M0 | IIIB | moderately differentiated | 3 |
| CRC | 001386 | 71 | female | 2010/03/10 | 2014/09/16 | 2014/09/16 | T4b | N0 | M1 | IVa | moderately differentiated | 18 |
| CRC | 001387 | 69 | female | 2010/03/11 | / | 2019/09/02 | T4b | Nla | M0 | IIIC | moderately differentiated | 5 |
| CRC | 001388 | 71 | male | 2010/03/12 | / | 2018/08/01 | T3 | N2 | M0 | IIIB/C? | moderately-poorly | 4 |
| CRC | 001389 | 62 | female | 2010/03/15 | 2014/05/15 | 2014/06/04 | T3 | N1 | M0 | IIIB | moderately differentiated | 1.5 |

**[Table 2]**

| **disease** | **sample #** | **age** | **sex** | **operation time** | **death time** | **last follow-up visit** | **T** | **N** | **M** | **TNM stage** | **pathological grading** | **tumor size (cm)** |
|---|---|---|---|---|---|---|---|---|---|---|---|---|
| CRC | 001395 | 84 | male | 2010/03/25 | / | 2019/09/03 | T3 | N0 | M0 | IIA | moderately differentiated | 10 |
| CRC | 001397 | 66 | male | 2010/03/29 | 2019/01/15 | 2020/05/27 | T3 | N0 | M0 | IIA | moderately differentiated | 7 |
| CRC | 001399 | 65 | female | 2010/03/30 | / | 2020/05/27 | T3 | N0 | M0 | IIA | moderately-poorly | 7.5 |
| CRC | 001406 | 77 | male | 2010/04/09 | 2013/02/28 | 2013/11/13 | T4 | N2 | M1 | IV | moderately differentiated | 5 |
| CRC | 001407 | 98 | male | 2010/04/12 | / | 2011/07/21 | T3 | N1 | M0 | IIIB | moderately differentiated | 3.5 |
| CRC | 001408 | 83 | female | 2010/04/14 | / | 2019/09/02 | T2 | N0 | M0 | I | well differentiated | 5 |
| CRC | 001409 | 41 | male | 2010/04/19 | 2011/07/15 | 2012/04/09 | T4 | N2 | M1 | IV | moderately-poorly | 12 |
| CRC | 001410 | 63 | female | 2010/04/20 | / | 2019/09/02 | T2 | N1 | M0 | IIIA | moderately differentiated | 2 |
| CRC | 001411 | 58 | female | 2010/04/21 | / | 2020/05/27 | T4 | N2 | M0 | IIIC | moderately differentiated | 4 |
| CRC | 001412 | 50 | male | 2010/04/23 | / | 2020/09/30 | T4 | N0 | M0 | IIB | well-moderately | 3.5 |
| CRC | 001413 | 54 | female | 2010/04/26 | / | 2018/07/26 | T2 | N0 | M0 | I | moderately differentiated | 4.5 |
| CRC | 001414 | 66 | female | 2010/04/27 | 2013/01/07 | 2013/04/02 | T3 | N2 | M1 | IV | moderately differentiated | 4 |
| CRC | 001416 | 86 | male | 2010/04/29 | / | 2020/05/27 | T4 | N1 | M0 | IIIB/C? | moderately differentiated | 7 |
| CRC | 001417 | 95 | female | 2010/04/29 | / | 2020/05/27 | T4 | N1 | M0 | IIIB/C? | moderately-poorly | 7 |
| CRC | 001418 | 70 | male | 2010/04/29 | / | 2018/09/29 | T3 | N0 | M0 | II(A) | moderately differentiated | 6 |
| CRC | 001419 | 53 | female | 2010/04/29 | 2010/09/01 | 2015/03/31 | T3 | N3 | M1 | IV | moderately-poorly | 5 |
| CRC | 001421 | 36 | male | 2010/05/05 | 2012/01/15 | 2012/08/10 | T4 | N1 | M0 | IIIB/C? | moderately differentiated | 4 |
| CRC | 001422 | 79 | male | 2010/05/06 | / | 2019/09/02 | T3 | N1 | M0 | IIIB | moderately differentiated | 6 |
| CRC | 001423 | 72 | female | 2010/05/06 | 2015/11/15 | 2016/01/14 | T3 | N1 | M0 | IIIC(B) | moderately differentiated | 2.5 |
| CRC | 001424 | 75 | female | 2010/05/10 | / | 2020/05/27 | T4 | N2 | M0 | IIIC | poorly differentiated | 10 |
| CRC | 001425 | 91 | female | 2010/05/11 | / | 2020/05/27 | T4 | N2 | M0 | IIIC | well-moderately | 4 |
| CRC | 001427 | 72 | male | 2010/05/13 | 2012/05/15 | 2019/09/02 | T3 | N0 | M0 | IIA | moderately differentiated | 2.5 |
| CRC | 001428 | 67 | female | 2010/05/14 | / | 2018/09/29 | T4 | N0 | M0 | IIB | moderately differentiated | 4 |

**[Table 3]**

| **disease** | **sample #** | **age** | **sex** | **operation time** | **death time** | **last follow-up visit** | **T** | **N** | **M** | **TNM stage** | **pathological grading** | **tumor size (cm)** |
|---|---|---|---|---|---|---|---|---|---|---|---|---|
| CRC | 001429 | 75 | female | 2010/05/17 | / | 2020/05/27 | T4 | N0 | M0 | IIB | mucinous adenocarcinoma | 2.5 |
| CRC | 001431 | 69 | female | 2010/05/18 | / | 2011/10/10 | 72 | N0 | M0 | I | moderately differentiated | 8 |
| CRC | 001432 | 72 | male | 2010/05/18 | 2013/05/15 | 2015/07/02 | T4 | N1 | M1 | IV | moderately differentiated | 3 |
| CRC | 001433 | 36 | male | 2010/05/19 | 2012/04/15 | 2013/11/18 | T3 | N2 | M0 | IIIC | poorly differentiated | 7 |
| CRC | 001435 | 72 | female | 2010/05/25 | / | 2019/01/18 | T3 | N2 | M0 | IIIC | mucinous adenocarcinoma | 4 |
| CRC | 001439 | 82 | female | 2010/05/31 | / | 2020/07/30 | T3 | N0 | M1 | IV | moderately differentiated | 4 |
| CRC | 001441 | 45 | female | 2010/06/02 | / | 2014/06/30 | T4 | N0 | M0 | IIB | moderately differentiated | 3 |
| CRC | 001443 | 54 | female | 2010/06/04 | / | 2018/07/04 | T3 | N0 | M0 | IIA | moderately-poorly | 3.5 |
| CRC | 001444 | 69 | male | 2010/06/04 | 2014/10/15 | 2015/08/06 | T3 | N2 | M1 | IV | moderately differentiated | 4.5 |
| CRC | 001445 | 78 | male | 2010/06/04 | / | 2020/05/28 | T4 | N0 | M0 | IIB | moderately differentiated | 4 |
| CRC | 001450 | 77 | male | 2010/06/08 | / | 2018/01/23 | T4 | N0 | M0 | IIB | moderately differentiated | 9 |
| CRC | 001451 | 64 | male | 2010/06/08 | / | 2020/05/28 | T4 | N1 | M0 | IIIB/C? | well-moderately | 4 |
| CRC | 001452 | 72 | female | 2010/06/09 | / | 2020/05/28 | T4 | N0 | M0 | IIB | moderately differentiated | 7 |
| CRC | 001453 | 57 | male | 2010/06/11 | / | 2020/05/28 | T4 | N0 | M0 | IIB | moderately differentiated | 9 |
| CRC | 001456 | 81 | female | 2010/06/12 | / | 2020/05/28 | T4 | N0 | M0 | IIB | mucinous adenocarcinoma | 6 |
| CRC | 001460 | 75 | female | 2010/06/17 | / | 2019/01/18 | T4 | N1 | M0 | IIIB | moderately differentiated | 6 |
| CRC | 001462 | 66 | male | 2010/06/18 | / | 2020/09/10 | T3 | N0 | M0 | IIB(A) | moderately-poorly | 7 |
| CRC | 001463 | 60 | male | 2010/06/21 | 2012/04/15 | 2012/11/22 | T4 | N2 | M1 | IV | poorly differentiated | 7 |
| CRC | 001466 | 43 | male | 2010/06/23 | / | 2011/04/21 | T4 | N0 | M0 | IIB | moderately differentiated | 5 |
| CRC | 001474 | 78 | male | 2010/06/28 | / | 2018/08/27 | T2 | N2b | M0 | IIIB | moderately differentiated | 3.5 |
| CRC | 001475 | 75 | male | 2010/06/29 | 2011/07/15 | 2011/10/27 | T4 | N2 | M1 | IV | poorly differentiated | 4.5 |
| CRC | 001476 | 84 | male | 2010/06/30 | / | 2020/05/28 | T4a | N1 | M0 | IIIB | moderately differentiated | 4 |

### 3. Cell and tissue in vitro culture

Infiltrating cells in mouse/human colorectal tumor/non-tumor tissues (2 × 10⁵) or mouse/human colorectal tumor/non-tumor tissue pieces (3 mm × 3 mm cut) were cultured with Dectin-1 ligands Curdlan, OXCA, or laminarin, or with PGE₂ stimulation for 20 h at 37 °C under 5% CO₂ in 96-well flat-bottomed plates (Falcon, Becton Dickinson) in a volume of 0.2 ml RPMI containing 10% FBS and 1% streptomycin plus penicillin. After the culture, cells or tissues were collected and mRNA expression of tumor- or anti-tumor-associated genes was measured by qPCR described in Quantitative reverse transcription PCR section described later.

### 4. Chemical induction of colorectal tumor

Six to seven weeks old mice were administrated intraperitoneally with AOM (Fujifilm Wako Pure Chemical Corporation, Osaka) of 10 µg per gram of body weight. One week after the administration, these mice were treated with 1% by mass DSS (36 to 50 kDa; MP Biomedicals, Illkirch, France) in their drinking water for 7 days followed by 14 days of normal water treatment for 1 cycle. After 3 cycles of DSS/normal water-treatment, these mice were further raised for another 2 to 6 weeks before sacrifice.

### 5. Induction of spontaneous small intestinal polyp and colorectal tumor

Apc^{Min/+} or Apc^{Min/+}Clec7a^{-/-} mice were normally raised in SPF condition for 19 to 21 weeks after being born, and polyps in small intestines emerged spontaneously. For the induction of colorectal tumor, same mice with 6 to 7 weeks old were treated with 1% by mass DSS for 1 week, and then were normally raised for another 4 to 5 weeks before sacrifice.

### 6. Cell preparation

Colonic tumor/non-tumor tissue-infiltrating cells were isolated using the method described below. Tissue was sliced into 1 mm thick, and was shaken for 40 min in Hanks' Balanced Salt Solution (HBSS) containing 3 mM EDTA at 37°C. Intra-epithelial lymphocytes and colonic epithelial cells floating in the culture supernatant were discarded, and the gut slices were washed twice with HBSS and then were shaken at 37°C for 120 min in RPMI containing 10% FBS and 1% streptomycin + penicillin, 200 U/ml collagenase (C2139; Sigma-Aldrich), and 5 U/ml DNase 1 (Sigma-Algrich). After the culturing, the samples were vortexed for 10 seconds, and single cell suspension was harvested after sterile gauze-filtration. In some experiments, tissue-infiltrating cells were further purified to lymphocytes on a 45%/66.6% discontinuous Percoll (Pharmacia, Uppsala, Sweden) gradient for 20 min.

### 7. Flow Cytometry

Antibodies against mouse CD4 (GK1.5), CD8α (53-6.7), TCRγδ (GL3), CD45 (30-F11), CD19 (6D5), CD11b (M1/70), CD11c (N418), I-A/I-E (M5/114.15.2), CD103 (2E7), IgG1 (RMG1-1), IFN-y (XMG1.2), and IL-17 (TC11-18H10.1) were purchased from Biolegend (San Diego, U.S.A.). Antibody against mouse Dectin-1 (2A11) was purchased from Abeam (Cambridge, UK). Anti-mouse IL-22bp polyclonal antibody was purchased from R&D systems (Biotechne, Minnesota, U.S.A). 7-AAD (7-amino-actinomycin D) viability staining solution was obtained from eBioscience (San Diego, U.S.A.). The 2.4G2 (anti-FCγRII/III-specific mAb) was obtained from American Type Culture Collection (Manassas, VA). All antibodies were used at a 1:250 dilution. Cells isolated from mouse intestines were washed twice with FACS Hanks buffer (HBSS containing 2% FCS and 0.1% sodium azide) and then treated with 2.4G2 to block FcR binding. Cells were then surface-stained with mAbs for 25 to 30 minutes. For cytokine FACS, cells were firstly stimulated or not stimulated with 50 ng/ml PMA (Sigma Aldrich) + 500 ng/ml ionomycin (Sigma Aldrich), or without any stimulation, and 1 mM monensin (Sigma Aldrich) for 4 hr. After the stimulation, cells were washed twice with FACS Hanks buffer (HBSS containing 2% FCS and 0.1% sodium azide) and then treated with 2.4G2 to block FcR binding. Cells were then surface-stained with mAbs for 30 min and were fixed and permeabilized with Cytofix/Cytoperm solution (BD Biosciences) for 20 min. After washing with 1× washing buffer, cells were incubated with anti-mouse IFN-y mAb and IL-17 mAb for another 30 min. A Canto II or FACSCalibur flow cytometer (BD Biosciences) and FlowJo 10.0 FACS software were used for the analysis.

### 8. Purification of epithelial cells, myeloid-derived cell and other immune cells from large intestine

Whole single cells from mouse whole intestines or from polyps were labeled with biotin-conjugated anti-mouse CD326 (G8.8) (Biolegend) followed by labeling with anti-Biotin microbeads (Miltenyi Biotec, Bergisch Gladbach, Germany). CD326⁺ epithelial cells were positively purified by using autoMACS (Miltenyi Biotec). The negative fraction was then stained with anti-mouse CD45 microbeads for the next leukocyte purification, or was stained with anti-mouse CD11b and CD11c microbeads for the myeloid-derived cell, Thy1.2 for the T cells or CD19 for B cell purification by autoMACS selection.

### 9. Quantitative reverse transcription PCR

Total RNA was extracted with Mammalian Total RNA Miniprep kit (Sigma Aldrich). RNA was denatured in the presence of an oligo dT primer and then reverse transcribed with the High Capacity cDNAReverse Transcription Kit (Applied Biosystems, San Francisco, U.S.A.). qPCR was performed with a SYBR Green qPCR kit (Carlsbad, USA) and C1000 Thermal Cycler system (Bio-Rad, Hercules, USA) with the sets of primers described in Table 4, and relative levels of each mRNA expression were calculated with the comparative cycle threshold (Ct value) method and were normalized with Gapdh mRNA expression level.

**[Table 4]**

| **mouse** | | |
|---|---|---|
| **Gene name** | ***Forward*** | ***Reverse*** |
| **Clec7a** | SEQ ID NO: 1 GACTTCAGCACTCAAGACATCC | SEQ ID NO: 2 TTGTGTCGCCAAAATGCTAGG |
| **II1b** | SEQ ID NO: 3 CAACCAACAAGTGATATTCTCCATG | SEQ ID NO: 4 GATCCACACTCTCCAGCTGCA |
| **II6** | SEQ ID NO: 5 GAGGATACCACTCCCAACAGACC | SEQ ID NO: 6 AAGTGCATCATCGTTGTTCATACA |
| **II18** | SEQ ID NO: 7 ACTGTACAACCGCAGTAATACGC | SEQ ID NO: 8 AGTGAACATTACAGATTTATCCC |
| **II18bp** | SEQ ID NO: 9 CCTACTTCAGCATCCTCTACTGG | SEQ ID NO: 10 AGGGTTTCTTGAGAAGGGGAC |
| **II22ra2** | SEQ ID NO: 11 CATTGCCTTCTAGGTCTCCTCA | SEQ ID NO: 12 CCTGCTTGCCAGTGCAAAAT |
| **Ptgs1** | SEQ ID NO: 13 ATGAGTCGAAGGAGTCTCTCG | SEQ ID NO: 14 GCACGGATAGTAACAACAGGGA |
| **Ptgs2** | SEQ ID NO: 15 TTCAACACACTCTATCACTGGC | SEQ ID NO: 16 AGAAGCGTTTGCGGTACTCAT |
| **Ptges2** | SEQ ID NO: 17 CCTCGACTTCCACTCCCTG | SEQ ID NO: 18 TGAGGGCACTAATGATGACAGAG |
| **Ptges3** | SEQ ID NO: 19 TGTTTGCGAAAAGGAGAATCCG | SEQ ID NO: 20 CCATGTGATCCATCATCTCAGAG |
| **Adh1** | SEQ ID NO: 21 GCAAAGCTGCGGTGCTATG | SEQ ID NO: 22 TCACACAAGTCACCCCTTCTC |
| **Aldh1a1** | SEQ ID NO: 23 ATACTTGTCGGATTTAGGAGGCT | SEQ ID NO: 24 GGGCCTATCTTCCAAATGAACA |
| **Aldh1a7** | SEQ ID NO: 25 ACTTGGAAGTTAGGCCCTGC | SEQ ID NO: 26 TGTGAAGGACACTTTGTCGATG |
| **Rdh7** | SEQ ID NO: 27 TGGGTCGAGTGTCTTTGTGTG | SEQ ID NO: 28 AACCCGCCAGGCTCTATGATA |
| **Eef1a1** | SEQ ID NO: 29 CAACATCGTCGTAATCGGACA | SEQ ID NO: 30 GTCTAAGACCCAGGCGTACTT |
| **Eef2** | SEQ ID NO: 31 TGTCAGTCATCGCCCATGTG | SEQ ID NO: 32 CATCCTTGCGAGTGTCAGTGA |
| **Pabpc1** | SEQ ID NO: 33 CAAGCCAGTACGCATCATGTG | SEQ ID NO: 34 TGCTTCCTGTGTTTCAAAGTGT |
| **Lgals9** | SEQ ID NO: 35 ATGCCCTTTGAGCTTTGCTTC | SEQ ID NO: 36 AACTGGACTGGCTGAGAGAAC |
| **H2-Ab1** | SEQ ID NO: 37 AGCCCCATCACTGTGGAGT | SEQ ID NO: 38 GATGCCGCTCAACATCTTGC |
| **Ki67** | SEQ ID NO: 39 TATCATTGACCGCTCCTTTAGGT | SEQ ID NO: 40 GCTCGCCTTGATGGTTCCT |
| **Vegfa** | SEQ ID NO: 41 GCACATAGAGAGAATGAGCTTCC | SEQ ID NO: 42 CTCCGCTCTGAACAAGGCT |
| **Ccnd1** | SEQ ID NO: 43 GCGTACCCTGACACCAATCTC | SEQ ID NO: 44 CTCCTCTTCGCACTTCTGCTC |
| **mye** | SEQ ID NO: 45 ATGCCCCTCAACGTGAACTTC | SEQ ID NO: 46 CGCAACATAGGATGGAGAGCA |
| **Arg1** | SEQ ID NO: 47 CTCCAAGCCAAAGTCCTTAGAG | SEQ ID NO: 48 AGGAGCTGTCATTAGGGACATC |
| **Nos2** | SEQ ID NO: 49 GTTCTCAGCCCAACAATACAAGA | SEQ ID NO: 50 GTTCTCAGCCCAACAATACAAGA |
| **Itgam** | SEQ ID NO: 51 ATGGACGCTGATGGCAATACC | SEQ ID NO: 52 TCCCCATTCACGTCTCCCA |
| **Foxp3** | SEQ ID NO: 53 CCCATCCCCAGGAGTCTTG | SEQ ID NO: 54 ACCATGACTAGGGGCACTGTA |
| **Gapdh** | SEQ ID NO: 55 TTCACCACCATGGAGAAGGC | SEQ ID NO: 56 GGCATGGACTGTGGTCATGA |

| **human** | | |
|---|---|---|
| **Gene name** | ***Forward*** | ***Reverse*** |
| **CD33** | SEQ ID NO: 57 GGTGTGACTACGGAGAGAACC | SEQ ID NO: 58 GGTAGGGTGGGTGTCATTCC |
| **HLA-DRA** | SEQ ID NO: 59 ATACTCCGATCACCAATGTACCT | SEQ ID NO: 60 GACTGTCTCTGACACTCCTGT |
| **ITGAM** | SEQ ID NO: 61 GCCTTGACCTTATGTCATGGG | SEQ ID NO: 62 CCTGTGCTGTAGTCGCACT |
| **CLEC7A** | SEQ ID NO: 63 GGAAGCAACACATTGGAGAATGG | SEQ ID NO: 64 CTTTGGTAGGAGTCACACTGTC |
| **IL22RA2** | SEQ ID NO: 65 TGTTGGGGTACTCAAGAACTCT | SEQ ID NO: 66 CCCTCCCGTAATAAGGTTCCTG |
| **PLA2G2A** | SEQ ID NO: 67 GAAAGGAAGCCGCACTCAGTT | SEQ ID NO: 68 CAGACGTTTGTAGCAACAGTCA |
| **PTGS2** | SEQ ID NO: 69 CTGGCGCTCAGCCATACAG | SEQ ID NO: 70 CGCACTTATACTGGTCAAATCCC |
| **PTGES** | SEQ ID NO: 71 TCCTAACGCTTTTGTCGCCTG | SEQ ID NO: 72 CGCTTCCCAGAGGATCTGC |
| **GAPDH** | SEQ ID NO: 73 TGTGGGCATCAATGGATTTGG | SEQ ID NO: 74 ACACCATGTATTCCGGGTCAAT |

### 10. PGE₂ concentration measurement

Polyp and non-polyp tissues from mouse colon were harvested and were put into 2 ml tubes containing 500 µl lysis buffer for protein [RIPA buffer: 50 mM Tris (pH 8.0) + 100 mM NaCl + 0.1% Triton X-100] and protease inhibitor cocktail (Takara Bio, Shika, Japan). Tissues were homogenized with beads and Micro Smasher (MS-100R, TOMY). After being centrifuged, supernatant of the tissue homogenate was collected and PGE₂ concentration was measured by ELISA Development Kit for PGE₂ (R&D systems, Minneapolis, USA). The final concentration of PGE₂ was normalized with total protein concentration of each individual.

### 11. RNA sequencing and analysis

RNA was isolated from colorectal tumor or non-tumor tissues using Sigma Aldrich total RNA-prep kit. RNA was prepared using Contech SmartSeq2 library prep kits and was sequenced at NovaSeq6000. Data were normalized and differential expression of genes was identified through usegalaxy.org. Pathway and process enrichment analysis by using the relative gene expression data was carried out through metascape.org.

### 12. Statistical analysis

Differences in parametric data were evaluated by the unpaired two-tailed Student's t test. For experiments containing more than two relative groups, one-way ANOVA followed by Dunnett's or by Tukey's multiple comparisons test was performed. Statistical significance of survival rate between groups was assessed by log-rank test. Statistics were computed with PRISM8 software (GraphPad Software). For the CRC patient study, categorical variables were compared by Fisher tests, and correlation was analyzed by Spearman's correlation test. Statistical analysis was performed using R software (version 4.0.3, R Foundation). Difference of P value <0.05 were considered statistically significant.

References
1. Bray, F., et al. Global cancer statistics 2018: GLOBOCAN estimates of incidence and mortality worldwide for 36 cancers in 185 countries. CA Cancer J Clin 68, 394-424 (2018).
2. Bosman, F. & Yan, P. Molecular pathology of colorectal cancer. Pol J Pathol 65, 257-266 (2014).
3. Taylor, P.R., et al. The beta-glucan receptor, Dectin-1, is predominantly expressed on the surface of cells of the monocyte/macrophage and neutrophil lineages. J Immunol 169, 3876-3882 (2002).
4. Taylor, P.R., et al. Dectin-1 is required for beta-glucan recognition and control of fungal infection. Nat Immunol 8, 31-38 (2007).
5. Saijo, S., et al. Dectin-1 is required for host defense against Pneumocystis carinii but not against Candida albicans. Nat Immunol 8, 39-46 (2007).
6. Tang, C., Makusheva, Y, Sun, H., Han, W. & Iwakura, Y Myeloid C-type lectin receptors in skin/mucoepithelial diseases and tumors. J Leukoc Biol 106, 903-917 (2019).
7. Brown, G.D., Willment, J.A. & Whitehead, L. C-type lectins in immunity and homeostasis. Nat Rev Immunol 18, 374-389 (2018).
8. Ruutu, M., et al. beta-glucan triggers spondylarthritis and Crohn's disease-like ileitis in SKG mice. Arthritis Rheum 64, 2211-2222 (2012).
9. Yoshitomi, H., et al. A role for fungal {beta}-glucans and their receptor Dectin-1 in the induction of autoimmune arthritis in genetically susceptible mice. J Exp Med 201, 949-960 (2005).
10. Lilly, L.M., et al. The beta-glucan receptor Dectin-1 promotes lung immunopathology during fungal allergy via IL-22. J Immunol 189, 3653-3660 (2012).
11. Tang, C., et al. Inhibition of Dectin-1 Signaling Ameliorates Colitis by Inducing Lactobacillus-Mediated Regulatory T Cell Expansion in the Intestine. Cell Host Microbe 18, 183-197 (2015).
12. Kamiya, T., et al. beta-Glucans in food modify colonic microflora by inducing antimicrobial protein, calprotectin, in a Dectin-1-induced-IL-17F-dependent manner. Mucosal Immunol 11, 763-773 (2018).
13. Tang, C., et al. Suppression of IL-17F, but not of IL-17A, provides protection against colitis by inducing Treg cells through modification of the intestinal microbiota. Nat Immunol 19, 755-765 (2018).
14. Chiba, S., et al. Recognition of tumor cells by Dectin-1 orchestrates innate immune cells for anti-tumor responses. Elife 3, e04177 (2014).
15. Zhao, Y, et al. Dectin-1-activated dendritic cells trigger potent antitumour immunity through the induction of Th9 cells. Nat Commun 7, 12368 (2016).
16. Seifert, L., et al. Dectin-1 Regulates Hepatic Fibrosis and Hepatocarcinogenesis by Suppressing TLR4 Signaling Pathways. Cell Rep 13, 1909-1921 (2015).
17. Daley, D., et al. Dectin 1 activation on macrophages by galectin 9 promotes pancreatic carcinoma and peritumoral immune tolerance. Nat Med 23, 556-567 (2017).
18. Taketo, M.M. & Edelmann, W. Mouse models of colon cancer. Gastroenterology 136, 780-798 (2009).
19. Borst, J., Ahrends, T., Babala, N., Melief, C.J.M. & Kastenmuller, W. CD4(+) T cell help in cancer immunology and immunotherapy. Nat Rev Immunol 18, 635-647 (2018).
20. Tsou, P., Katayama, H., Ostrin, E.J. & Hanash, S.M. The Emerging Role of B Cells in Tumor Immunity. Cancer Res 76, 5597-5601 (2016).
21. Moutai, T., Yamana, H., Nojima, T. & Kitamura, D. A novel and effective cancer immunotherapy mouse model using antigen-specific B cells selected in vitro. PLoS One 9, e92732 (2014).
22. Ostrand-Rosenberg, S. & Fenselau, C. Myeloid-Derived Suppressor Cells: Immune-Suppressive Cells That Impair Antitumor Immunity and Are Sculpted by Their Environment. J Immunol 200, 422-431 (2018).
23. Lechner, M.G., Liebertz, D.J. & Epstein, A.L. Characterization of cytokine-induced myeloid-derived suppressor cells from normal human peripheral blood mononuclear cells. J Immunol 185, 2273-2284 (2010).
24. Xiang, X., et al. Induction of myeloid-derived suppressor cells by tumor exosomes. Int J Cancer 124, 2621-2633 (2009).
25. Alshetaiwi, H., et al. Defining the emergence of myeloid-derived suppressor cells in breast cancer using single-cell transcriptomics. Sci Immunol 5(2020).
26. Huber, S., et al. IL-22BP is regulated by the inflammasome and modulates tumorigenesis in the intestine. Nature 491, 259-263 (2012).
27. Martin, J.C., et al. Interleukin-22 binding protein (IL-22BP) is constitutively expressed by a subset of conventional dendritic cells and is strongly induced by retinoic acid. Mucosal Immunol 7, 101-113 (2014).
28. Lim, C., Hong, M. & Savan, R. Human IL-22 binding protein isoforms act as a rheostat for IL-22 signaling. Sci Signal 9, ra95 (2016).
29. Greten, T.F., Manns, M.P. & Korangy, F. Myeloid derived suppressor cells in human diseases. Int Immunopharmacol 11, 802-807 (2011).
30. Tang, W., Liu, J.W., Zhao, W.M., Wei, D.Z. & Zhong, J.J. Ganoderic acid T from Ganoderma lucidum mycelia induces mitochondria mediated apoptosis in lung cancer cells. Life Sci 80, 205-211 (2006).
31. Lin, S.B., Li, C.H., Lee, S.S. & Kan, L.S. Triterpene-enriched extracts from Ganoderma lucidum inhibit growth of hepatoma cells via suppressing protein kinase C, activating mitogen-activated protein kinases and G2-phase cell cycle arrest. Life Sci 72, 2381-2390 (2003).
32. Cheung, N.K., Modak, S., Vickers, A. & Knuckles, B. Orally administered beta-glucans enhance anti-tumor effects of monoclonal antibodies. Cancer Immunol Immunother 51, 557-564 (2002).
33. Albeituni, S.H., et al. Yeast-Derived Particulate beta-Glucan Treatment Subverts the Suppression of Myeloid-Derived Suppressor Cells (MDSC) by Inducing Polymorphonuclear MDSC Apoptosis and Monocytic MDSC Differentiation to APC in Cancer. J Immunol 196, 2167-2180 (2016).
34. Hong, F., et al. Mechanism by which orally administered beta-1,3-glucans enhance the tumoricidal activity of antitumor monoclonal antibodies in murine tumor models. J Immunol 173, 797-806 (2004).
35. Park, J.Y, Pillinger, M.H. & Abramson, S.B. Prostaglandin E2 synthesis and secretion: the role of PGE2 synthases. Clin Immunol 119, 229-240 (2006).
36. Mizuno, R., Kawada, K. & Sakai, Y Prostaglandin E2/EP Signaling in the Tumor Microenvironment of Colorectal Cancer. Int J Mol Sci 20(2019).
37. Castellone, M.D., Teramoto, H., Williams, B.O., Druey, K.M. & Gutkind, J.S. Prostaglandin E2 promotes colon cancer cell growth through a Gs-axin-beta-catenin signaling axis. Science 310, 1504-1510 (2005).
38. Marnett, L.J. Aspirin and the potential role of prostaglandins in colon cancer. Cancer Res 52, 5575-5589 (1992).
39. Oshima, M., et al. Suppression of intestinal polyposis in Apc delta716 knockout mice by inhibition of cyclooxygenase 2 (COX-2). Cell 87, 803-809 (1996).
40. Ito, T., Hirose, K., Norimoto, A., Saku, A. & Nakajima, H. Dectin-1 plays a critical role in HDM-induced PGE2 production in macrophages. Allergol Int 66S, S44-S46 (2017).
41. Balderramas, H.A., et al. Human neutrophils produce IL-12, IL-10, PGE2 and LTB4 in response to Paracoccidioides brasiliensis. Involvement of TLR2, mannose receptor and Dectin-1. Cytokine 67, 36-43 (2014).
42. Kotenko, S.V, et al. Identification, cloning, and characterization of a novel soluble receptor that binds IL-22 and neutralizes its activity. J Immunol 166, 7096-7103 (2001).
43. Dumoutier, L., Lejeune, D., Colau, D. & Renauld, J.C. Cloning and characterization of IL-22 binding protein, a natural antagonist of IL-10-related T cell-derived inducible factor/IL-22. J Immunol 166, 7090-7095 (2001).
44. De Cicco, P., Ercolano, G. & Ianaro, A. The New Era of Cancer Immunotherapy: Targeting Myeloid-Derived Suppressor Cells to Overcome Immune Evasion. Front Immunol 11, 1680 (2020).
45. Sieminska, I. & Baran, J. Myeloid-Derived Suppressor Cells in Colorectal Cancer. Front Immunol 11, 1526 (2020).
46. Grivennikov, S.I., Greten, F.R. & Karin, M. Immunity, inflammation, and cancer. Cell 140, 883-899 (2010).
47. Kappelman, M.D., et al. Risk of cancer in patients with inflammatory bowel diseases: a nationwide population-based cohort study with 30 years of follow-up evaluation. Clin Gastroenterol Hepatol 12, 265-273 e261 (2014).

The disclosure of Japanese Patent Application No. 2021-204572 is incorporated herein by reference in its entirety. All literatures, patent applications, and technical standards described in the present disclosure are incorporated herein by reference to the same extent as a case in which the literatures, the patent applications, and the technical standards were specifically and individually indicated to be incorporated by reference.

## Claims

1. An intestinal tumor suppressor comprising a β-glucan having a Dectin-1 inhibitory activity, wherein the intestinal tumor suppressor suppresses an intestinal tumor through Dectin-1 inhibition.

2. The intestinal tumor suppressor according to claim 1, wherein the β-glucan is a β-glucan including β-1,3 linkages, or a β-glucan including a β-1,6-linked branched chain on a β-1,3-linked main chain.

3. The intestinal tumor suppressor according to claim 1 or 2, wherein a molecular weight of the β-glucan is from 0.2 K to 100 K.

4. The intestinal tumor suppressor according to any one of claims 1 to 3, wherein the β-glucan is laminarin.

5. A PGE₂ production inhibitor comprising a β-glucan having a Dectin-1 inhibitory activity.

6. The PGE₂ production inhibitor according to claim 5, wherein the β-glucan is a β-glucan including β-1,3 linkages, or a β-glucan including a β-1,6-linked branched chain on a β-1,3-linked main chain.

7. The PGE₂ production inhibitor according to claim 5 or 6, wherein a molecular weight of the β-glucan is from 0.2 K to 100 K.

8. The PGE₂ production inhibitor according to any one of claims 5 to 7, wherein the β-glucan is laminarin.

9. An IL-22BP production promoter comprising a β-glucan having a Dectin-1 inhibitory activity.

10. The IL-22BP production promoter according to claim 9, wherein the β-glucan is a β-glucan including β-1,3 linkages, or a β-glucan including a β-1,6-linked branched chain on a β-1,3-linked main chain.

11. The IL-22BP production promoter according to claim 9 or 10, wherein a molecular weight of the β-glucan is from 0.2 K to 100 K.

12. The IL-22BP production promoter according to any one of claims 9 to 11, wherein the β-glucan is laminarin.

13. An intestinal tumor suppressor comprising a Dectin-1 inhibitor, wherein the intestinal tumor suppressor suppresses an intestinal tumor through Dectin-1 inhibition.

14. The intestinal tumor suppressor according to claim 13, wherein the Dectin-1 inhibitor is a Dectin-1 antagonist.

15. The intestinal tumor suppressor according to claim 13, wherein the Dectin-1 inhibitor is an anti-Dectin-1 inhibitory antibody or a Dectin-1 inhibitory small molecule compound.

16. The intestinal tumor suppressor according to any one of claims 1 to 4, 13, and 14, wherein the intestinal tumor is an intestinal tumor accompanied by an increase in an expression level of PGE₂ or an intestinal tumor accompanied by a decrease in an expression level of IL-22BP.
